# FASCICULE DE BREVET EUROPEEN

(11) **EP 1 228 760 B1**
(45) Date de publication et mention de la délivrance du brevet: **31.08.2011**
(21) Numéro de dépôt: 02076763.8
(22) Date de dépôt: 10.10.2000
(51) Int. Cl.: A61K 31/426, A61K 31/421, A61K 31/42, A61K 31/417, C07D 233/54, A61K 31/4164, A61K 31/4178, A61P 25/08, A61P 25/28, A61P 29/02, A61P 29/00, A61P 1/00

(54) **Dérivés d'hétérocycles à 5 chaînons, leur préparation et leur application à titre de médicaments**
Fünfgliedrige-Heteroringderivate , ihre Herstellung und ihre Verwendung als Arzneimittel
5-membered heterocycle derivatives, production thereof and use thereof as medicaments

(30) Priorité: 11.10.1999 FR 9912643; 01.08.2000 FR 0010151; 01.09.2000 FR 0011169
(43) Date de publication de la demande: 07.08.2002
(62) Demande divisionnaire de: 00967988.7
(73) Titulaire: IPSEN PHARMA, 92100 Boulogne-Billancourt (FR)
(72) Inventeur: Chabrier de Lassauniere, Pierre-Etienne, 75016 Paris (FR); Harnett, Jeremiah, 91190 Gif-sur-Yvette (FR); Bigg, Dennis, 91190 Gif-sur-Yvette (FR); Pommier, Jacques, 75015 Paris (FR); Lannoy, Jacques, 91470 Bievres (FR); Liberatore, Anne-Marie, 78610 Auffargis (FR); Thurieau, Christophe, 75116 Paris (FR)
(74) Mandataire: Audonnet, Nathalie

(56) Documents cités:
- EP-A- 0 432 740
- EP-A- 0 908 180
- WO-A-00/39130
- WO-A-96/00730
- WO-A-96/16040
- WO-A-98/15274
- WO-A-98/27108
- WO-A-98/58934
- WO-A-99/64401
- WO-A-99/64420
- FR-A- 2 132 632
- US-A- 4 372 964
- US-A- 5 620 999
- DATABASE CA [en ligne] CHEMICAL ABSTRACTS SERVICE, COLUMBUS, OHIO, US; APOYAN, N. A. ET AL: "Antiinflammatory properties of some alkoxyphenylimidazole and alkoxybenzyloxazoline derivatives" retrieved from STN Database accession no. 88:44938 HCA XP002263543 & BIOLOGICHESKII ZHURNAL ARMENII (1977), 30(8), 96-7 ,

## Description

La présente invention concerne l'utilisation de composés de formule générale (I) pour préparer un médicament destiné à inhiber les monoamine oxydases (MAO) et/ou peroxydation lipidique et/ou à agir en tant que modulateurs des canaux sodiques. Elle a également pour objet, en tant que médicaments, des composés de formule générale (I) définie ci-après. Elle concerne de plus de nouveaux composés de formule générale (I).

Les composés évoqués ci-dessus présentent souvent 2 ou 3 des activités citées ci-dessus, lesquelles leur confèrent des propriétés pharmacologiques avantageuses.

En effet, compte tenu du rôle potentiel des MAO et des ROS (« *reactive oxygen species* » ou espèces réactives de l'oxygène, à l'origine de la peroxydation lipidique) en physiopathologie, les nouveaux dérivés décrits répondant à la formule générale **(I)** peuvent produire des effets bénéfiques ou favorables dans le traitement de pathologies où ces enzymes et/ou ces espèces radicalaires sont impliquées. Notamment :
- les troubles du système nerveux central ou périphérique comme par exemple les maladies neurologiques où l'on peut notamment citer la maladie de Parkinson, les traumatismes cérébraux ou de la moelle épinière, l'infarctus cérébral, l'hémorragie sub arachnoïde, l'épilepsie, le vieillissement, les démences séniles, la maladie d'Alzheimer, la chorée de Huntington, la sclérose latérale amyotrophique, les neuropathies périphériques, la douleur ;
- la schizophrénie, les dépressions, les psychoses ;
- les troubles de la mémoire et de l'humeur ;
- les pathologies comme par exemple la migraine ;
- les troubles du comportement, la boulimie et l'anorexie ;
- les maladies auto-immunes et virales comme par exemple le lupus, le sida, les infections parasitaires et virales, le diabète et ses complications, la sclérose en plaques.
- l'addiction aux substances toxiques ;
- les pathologies inflammatoires et prolifératives ;
- et plus généralement toutes les pathologies caractérisées par une production excessive des ROS et/ou une participation des MAO.

Dans l'ensemble de ces pathologies, il existe des évidences expérimentales démontrant l'implication des ROS (Free Radic. Biol. Med. (1996) 20, 675-705 ; Antioxid. Health. Dis. (1997) 4 (Handbook of Synthetic Antioxidants), 1-52) ainsi que l'implication des MAO (Goodman & Gilman's : The pharmacological basis of therapeutics , 9th ed., 1995,431-519).

L'intérêt d'une combinaison des activités inhibitrice de MAO et inhibitrice de la peroxydation lipidique est par exemple bien illustré dans la maladie de Parkinson. Cette pathologie est caractérisée par une perte des neurones dopaminergiques de la voie nigrostriatal dont la cause serait en partie liée à un stress oxydatif dû aux ROS. De la dopamine exogène à partir de L Dopa est utilisé en thérapeutique afin de maintenir des taux suffisants de dopamine. Les inhibiteurs de MAO sont aussi utilisés avec la L Dopa pour éviter sa dégradation métabolique mais n'agissent pas sur les ROS. Des composés agissant à la fois sur les MAO et les ROS auront donc un avantage certain.

Par ailleurs, le caractère de modulateur des canaux sodiques est très utile pour des indications thérapeutiques comme :
- le traitement ou la prévention de la douleur, et notamment :
   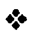 des douleurs post-opératoires,
   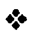 de la migraine,
   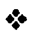 des douleurs neuropathiques telles que la névralgie du trijumeau, la douleur post-herpétique, les neuropathies diabétiques, les névralgies glosso-pharyngiennes, les radiculopathies et neuropathies secondaires à des infiltrations métastatiques, l'adiposis dolorosa et les douleurs liées aux brûlures,
   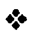 des douleurs centrales consécutives aux accidents cérébraux vasculaires, lésions thalamiques et sclérose en plaques, et
   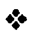 des douleurs chroniques inflammatoires ou liées à un cancer ;

   - le traitement de l'épilepsie ;
   - le traitement de troubles liés à la neurodégénération, et en particulier :
      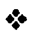 des accidents cérébraux vasculaires,
      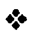 du traumatisme cérébral, et
      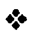 de maladies neurodégénératives telles que la maladie d'Alzheimer, la maladie de Parkinson et la sclérose latérale amyotrophique ;
   - le traitement des troubles bipolaires et du syndrome du colon irritable.

Les avantages concrets de la présence dans un composé d'au moins une de ces activités ressortent donc clairement de ce qui précède.

La demande de brevet européen EP 432 740 décrit des dérivés d'hydroxyphénylthiazoles, lesquels peuvent être utilisés dans le traitement des maladies inflammatoires, en particulier les maladies rhumatismales. Ces dérivés d'hydroxyphénylthiazoles montrent des propriétés de piégeurs de radicaux libres et d'inhibiteurs du métabolisme de l'acide arachidonique (ils inhibent la lipoxygénase et la cyclooxygénase).

D'autres dérivés d'hydroxyphénylthiazoles ou d'hydroxyphényloxazoles sont décrits dans la demande de brevet PCT WO 99/09829. Ceux-ci possèdent des propriétés analgésiques.

Un certain nombre de dérivés d'imidazoles de structures proches ou identiques à celles de composés répondant à la formule générale **(I)** selon l'invention ont par ailleurs été décrits par la demanderesse dans la demande de brevet PCT WO 99/64401 comme agonistes ou antagonistes de la somatostatine. Lesdits dérivés d'imidazoles possèdent cependant des propriétés thérapeutiques dans des domaines différents de ceux indiqués ci-dessus (la suppression de l'hormone de croissance et le traitement de l'acromégalie, le traitement de la resténose, l'inhibition de la sécrétion d'acide gastrique et la prévention des saignements gastro-intestinaux notamment).

Par ailleurs, les composés de formule générale **(A1)** 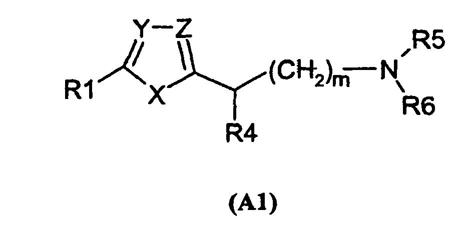 dans laquelle
R1 représente l'un des radicaux aryle, hétéroaryle, aralkyle ou cycloalkyle optionnellement substitués par un à trois substituants choisis indépendamment parmi un atome halogène, le radical CF₃, CN, OH, alkyle ou alkoxy, SO₂R9 avec R9 représentant NH₂ ou NHCH₃ ;
X représente NR2, R2 représentant H ou alkyle ;
Y représente N ou CR3 ;
Z représente CR3 ou N ;
à la condition toutefois que Y et Z ne sont pas tous deux CR3 ou N en même temps ;
R3 représente H, alkyle, halogène, hydroxyalkyle ou phényle éventuellement substitué par 1 à 3 subtituants choisis parmi H, CF₃, CN, SO₂NH₂, OH, alkyle ou alkoxy ;
m représente 0, 1 ou 2 ;
R4 représente H ou alkyle ;
lorsque Z représente CR3, alors R3 et R4 peuvent aussi représenter ensemble -(CH₂)ₙ₁-avec n1 entier de 2 à 4 ou R2 et R4 peuvent aussi représenter ensemble -(CH₂)ₙ₂- avec n2 entier de 2 à 4 ;
R5 et R6 représentent indépendamment H, alkyle, alkoxy, aryle ou aralkyle ;
NR5R6 pouvant aussi représenter ensemble (notamment) :
- le radical 2-(1,2,3,4-tétrahydroquinolyl) éventuellement substitué,
- un radical 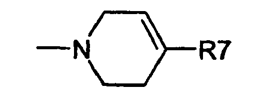 dans lequel R7 représente l'un des radicaux phényle, benzyle ou phénéthyle dans lequels le cycle phényle peut être substitué ;
- un radical 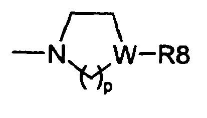 dans lequel p est un entier de 1 à 3,
   W est N et R8 représente H, CF₃, l'un des radicaux phényle, pyridyle ou pyrimidinyle éventuellement substitués de 1 à 2 fois par des radicaux choisis parmi halogène, OH, alkyle ou alkoxy, ou
   W est CH et R8 représente phényle éventuellement substitué ou aralkyle éventuellement substitué sur le groupe aryle ;
   ont été décrits dans la demande de brevet PCT WO 96/16040 en tant qu'agonistes partiels ou antagonistes des sous-récepteurs de la dopamine du cerveau ou en tant que formes prodrogues de tels agonistes partiels ou antagonistes. Ces composés présenteraient de ce fait des propriétés intéressantes dans le diagnostic et le traitement de désordres affectifs tels que la schizophrénie et la dépression ainsi que certains désordres du mouvement tels que la maladie de Parkinson.

Il a également été décrit dans la demande de brevet PCT WO 98/27108 que certains amides de formule générale **(A2)** 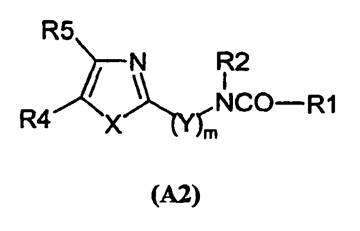 dans laquelle :
R1 représente notamment un radical alkyle, phényle éventuellement substitué ou aryle hétérocyclique éventuellement substitué ;
R2 représente H ou phénylalkyle ;
R4 représente H, quinolyle, 3-4-méthylènedioxyphényle ou l'un des radicaux phényle ou pyridyle éventuellement substitués, par un ou des radicaux choisis notamment parmi alkyle, alkoxy, alkylthio, hydroxy éventuellement protégé, amino, alkylamino, dialkylamino ;
R5 représente H ou un radical imidazolyle, phényle, nitrophényle, phénylalkyle, ou encore un radical -CO-N(R7)(R8), dans lequel R7 et R8 représentent indépendamment H, phényle, phénylalkyle, alkyle ou alkoxy ;
ou R4 et R5 en combinaison forment un groupe de formule -CH=CH-CH=CH- ;
Y est un radical phénylène substitué par un radical phényle, phénoxy ou phénylalkoxy, ou un groupe de formule -CH(R3)-, dans laquelle R3 représente H ou un radical de formule -(CH₂)ₙ-R6, dans laquelle R6 représente un radical hydroxy éventuellement protégé, acyle, carboxy, acylamino, alkoxy, phénylalkoxy, alkylthio, phényle éventuellement substitué, pyridyle éventuellement substitué, pyrazinyle, pyrimidinyle, furyle, imidazolyle, naphtyle, N-alkylindolyle ou 3,4-méthylènedioxyphényle et n est un entier de 0 à 3 ;
R2 et R3 pris ensemble avec les atomes de carbone qui les portent pouvant former un groupe phényle ;
X représente S ou NR9 ;
R9 représentant H, un radical alkyle ou cycloalkyle, ou encore un radical benzyle éventuellement substitué une fois sur sa partie phényle par H, alkyle ou alkoxy ;
sont des inhibiteurs des NO synthases et peuvent être utilisés pour traiter des maladies qui comprennent notamment l'ischémie cardiovasculaire ou cérébrale, l'hémorragie cérébrale, les troubles du système nerveux central, la maladie d'Alzheimer, la sclérose en plaques, le diabète, l'hépatite, la migraine, l'arthrite rhumatoïde et l'ostéoporose.

Dans un domaine différent, la demanderesse a elle-même précédemment décrit dans la demande de brevet PCT WO 98/58934 des dérivés d'amidines ayant la faculté d'inhiber les NO Synthases et/ou la peroxydation lipidique.

D'autres dérivés d'imidazole à activité thérapeutique sont également décrits dans la littérature comme par exemple dans FR 2132632, US5620999, US4372964 et Biologicheskii Zhumal Armenii (1977), 30(8), 96-7.

La demanderesse a maintenant découvert de façon surprenante que certains intermédiaires des premières étapes de synthèse des amidines décrites dans la demande de brevet PCT WO 98/58934, et plus généralement certains dérivés d'hétérocycles à cinq chaînons, à savoir les produits de formule générale **(I)** définie ci-après, possèdent au moins l'une des trois propriétés choisies parmi les propriétés suivantes (et souvent même deux de ces trois propriétés voire parfois les trois à la fois) :
- des propriétés d'inhibition des MAO ;
- des propriétés d'inhibition de la peroxydation lipidique ; et
- des propriétés de modulation des canaux sodiques.

Ces propriétés avantageuses offrent l'intérêt d'ouvrir à de tels composés de nombreuses applications, en particulier dans le traitement des maladies neurodégénératives, et notamment celles indiquées précédemment, de la douleur ou de l'épilepsie.

La présente invention concerne des composés de formule générale **(I)** : 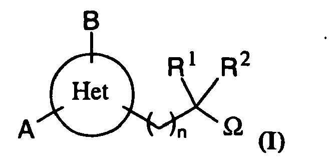 sous forme racémique, d'énantiomère ou toute combinaison de ces formes, dans laquelle Het est un hétérocycle à 5 chaînons comportant 2 hétéroatomes et tel que la formule générale (I) corresponde exclusivement à l'une des sous-formules suivantes : 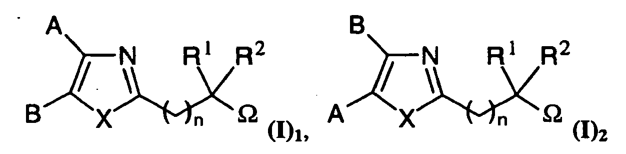 dans lesquelles
A représente un radical 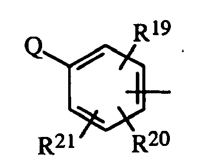 dans lequel Q représente un radical phényle éventuellement substitué par un ou des substituants choisis indépendamment parmi un atome halogène, un radical OH, cyano, nitro, alkyle, alkoxy ou -NR¹⁰R¹¹ et un groupe de deux substituants représentant ensemble un radical méthylène dioxy ou éthylènedioxy,
R¹⁰ et R¹¹ représentant, indépendamment, un atome d'hydrogène ou un radical alkyle,
R¹⁹, R²⁰ et R²¹ représentent, indépendamment, un hydrogène, un halogène, le groupe OH ou SR²⁶, ou un radical alkyle, cycloalkyle, alkényle, alkoxy, cyano, nitro,
R²⁶ représentant un atome d'hydrogène ou un radical alkyle,
X représente S ou NR³⁸,
R³⁸ représentant un atome d'hydrogène,
R¹ représente un atome d'hydrogène, un radical alkyle, aminoalkyle, alkoxyalkyle, cycloalkyle, cycloalkylalkyle, trifluorométhylalkyle, alkényle, allènyle, allènylalkyle, alkynyle, cyanoalkyle, -(CH₂),-Z¹R³⁹, -(CH₂)_{g}-COR⁴⁰, -(CH₂)_{g}-NHCOR⁷⁰, aryle, aralkyle, arylcarbonyle, hétéroarylalkyle ou aralkylcarbonyle, le groupement aryle des radicaux aryle, aralkyle, arylcarbonyle, hétéroarylalkyle ou aralkylcarbonyle étant lui-même éventuellement substitué par un ou plusieurs substituants choisis parmi le groupe constitué des radicaux alkyle, halogène, alkoxy, nitro, cyano, cyanoalkyle, amino, alkylamino, dialkylamino, -(CH₂)ₖ-Z²R³⁹ ou -(CH₂)ₖ-COR⁴⁰,
Z¹ et Z² représentant une liaison, -O-, -NR⁴¹- ou -S-,
R³⁹ et R⁴¹ représentant, indépendamment à chaque fois qu'ils interviennent, un atome d'hydrogène ou un radical alkyle, alkényle, alkynyle ou cyanoalkyle,
R⁴⁰ représentant, indépendamment à chaque fois qu'il intervient, un atome d'hydrogène ou un radical alkyle, allènyle, allènylalkyle, alkényle, alkynyle, cyanoalkyle, alkoxy ou NR⁴²R⁴³,
R⁴² et R⁴³ représentant, indépendamment à chaque fois qu'ils interviennent, un atome d'hydrogène ou un radical alkyle, allènyle, allènylalkyle, alkényle, alkynyle ou cyanoalkyle,
et R² représente un atome d'hydrogène, un radical alkyle, aminoalkyle, alkoxyalkyle, cycloalkyle, cycloalkylalkyle, trifluorométhylalkyle ou -(CH₂)_{g}-NHCOR⁷¹, ou encore l'un des radicaux aralkyle ou hétéroarylalkyle éventuellement substitués sur le groupe aryle ou hétéroaryle par un ou des groupes choisis indépendamment parmi le groupe composé d'un atome halogène et d'un radical alkyle, alkoxy, hydroxy, cyano, nitro, amino, alkylamino ou dialkylamino,
R⁷⁰ et R⁷¹ représentant indépendamment un radical alkyle ou alkoxy ;
ou bien R¹ et R², pris ensemble avec l'atome de carbone qui les porte, forment un carbocycle de 3 à 7 chaînons ;
B représente un atome d'hydrogène ou un radical alkyle;
Ω représente l'un des radicaux NR⁴⁶R⁴⁷ ou OR⁴⁸, dans lesquels :
R⁴⁶ et R⁴⁷ représentent, indépendamment, un atome d'hydrogène ou un radical alkyle, cycloalkyle, cycloalkylalkyle, alkényle, alkynyle, allènyle, allènylalkyle, cyanoalkyle, -(CH₂)_{g}-Z⁴R⁵⁰, -(CH₂)ₖ-COR⁵¹, -(CH₂)ₖ-COOR⁵¹, -(CH₂)ₖ-CONHR⁵¹ ou -SO₂R⁵¹, ou encore un radical choisi parmi les radicaux aryle, aralkyle, aryloxyalkyle, arylcarbonyle, arylimino, aralkylcarbonyle, hétéroaryle et en particulier pyridinyle, pyridinylalkyle ou pyridinylcarbonyle, le groupement aryle ou hétéroaryle desdits radicaux aryle, aralkyle, aryloxyalkyle, arylcarbonyle, arylimino, aralkylcarbonyle, hétéroaryle, pyridinylalkyle ou pyridinylcarbonyle étant éventuellement substitué par un ou des substituants choisis indépendamment parmi halogène, alkyle, alkoxy, hydroxy, nitro, cyano, cyanoalkyle, amino, alkylamino, dialkylamino, -(CH₂)ₖ-Z⁵R⁵⁰, -(CH₂)ₖ-COR⁵¹ et -(CH₂)ₖ-COOR⁵¹,
Z⁴ et Z⁵ représentant une liaison, -O-, -NR⁵²- ou -S-,
R⁵⁰ et R⁵², représentant, indépendamment à chaque fois qu'ils interviennent, un atome d'hydrogène ou un radical alkyle, alkényle, alkynyle, alkoxy, allènyle, allènylalkyle ou cyanoalkyle,
R⁵¹ représentant, indépendamment à chaque fois qu'ils intervient, un atome d'hydrogène, l'un des radicaux cycloalkyle ou cycloalkylalkyle dans lesquels le radical cycloalkyle compte de 3 à 7 atomes de carbone, un radical alkyle linéaire ou ramifié comptant de 1 à 8 atomes de carbone, un radical alkényle, alkynyle, allènyle, allènylalkyle, cyanoalkyle, alkoxyalkyle ou NR⁵⁸R⁵⁹, ou encore un radical aryle ou aralkyle, ledit radical aryle ou aralkyle pouvant être substitué par un ou des substituants choisis indépendamment parmi un atome halogène et un radical alkyle ou alkoxy,
R⁵⁸ et R⁵⁹ représentant, indépendamment, un atome d'hydrogène ou un radical alkyle, alkényle, alkynyle, allènyle, allènylalkyle ou cyanoalkyle,
g et p, chaque fois qu'ils interviennent, étant indépendamment des entiers de 1 à 6, et k et n, chaque fois qu'ils interviennent, étant indépendamment des entiers de 0 à 6;
et R⁴⁸ représente un atome d'hydrogène ou un radical alkyle, alkynyle ou cyanoalkyle ;
ou des sels pharmaceutiquement acceptable de ces composés.

La présente invention concerne préférentiellement des composés tels que définis ci-dessus ou un de leur sels pharmaceutiquement acceptable, caractérisés en qu'il s'agit de composés choisi parmi :
2-(4-[1,1'-biphényl]-4-yl-1H-imidazol-2-yl)éthylcarbamate de butyle ;
N-[2-(4-[1,1'-biphényl]-4-yl-1H-imidazol-2-yl)éthyl]-1-butanesulfonamide ;
4-[2-(2-{[butylamino)carbonyl]amino}éthyl)-1H-imidazol-4-yl]-1,1'-biphényle;
*N*-benzyl-*N*-[(4-[1,1'-biphényl]-4-yl-1*H*-imidazol-2-yl)méthyl]-1-hexanamine ;
*N*-benzyl(4-[1,1'-biphényl]-4-yl-1H-imidazol-2-yl)-*N*-méthylméthanamine ;
(R,S)-4-(2-{1-[(*tert-*butoxycarbonyl)amino]pentyl}-1*H*-imidazol-4-yl)-1,1'-biphényle;
(R,S)-*N*-benzyl-1-(4-[1,1'-biphényll-4-yl-1*H*-imidazol-2-yl)-1-pentanamine ;
(4-[1,1'-biphényl]-4-yl-1*H*-imidazol-2-yl)méthylcarbamate de butyle ;
4-(2-{[(*tert-*butoxycarbonyl)amino]méthyl)-1*H*-imidazol-4-yl)-1,1'-biphényle;
(4-[1,1'-biphényl]-4-yl-1*H*-imidazol-2-yl)méthanamine;
*N,N*-dibenzyl(4-[1,1'-biphényl]-4-yl-1*H*-imidazol-2-yl)méthanamine ;
4-(2-{[(*tert-*butoxycarbonyl)(méthyl)amino]méthyl}-1*H*-imidazol-4-yl)-1,1'-biphényle ;
4-(2-{(1*R*)-1-[(*tert-*butoxycarbonyl)amino]-2-cydohexyléthyl)-1*H*-imidazol-4-yl)-1,1'-biphényle;
4-(2-{2-[(*tert-*butoxycarbonyl)amino]éthyl}-1*H*-imidazol-4-yl)-1,1'-biphényle;
(1*R*)-1-(4-[1,1'-biphényl]-4-yl-1*H*-imidazol-2-yl)-2-cydohexyléthanamine ;
(4-[1,1'-biphényl]-4-yl-1*H*-imidazol-2-yl)-*N*-méthylméthanamine ;
2-(4-[1,1'-biphényl]-4-yl-1*H*-imidazol-2-yl)éthanamine ;
*N*-benzyl-2-(4-[1,1'-biphényl]-4-yl-1*H*-imidazol-2-yl)éthanamine ;
4-(2-{[benzyl(*tert-*butoxycarbonyl)amino]méthyl}-1*H*-imidazol-4-yl)-1,1'-biphényle ;
(1*R*)-1-(4-[1,1'-biphényl]-4-yl-1*H*-imidazol-2-yl)-3-phényl-1-pronanamine
4-(2-{(1*R*)-1-[(*tert*-butoxycarbonyl)amino]-3-phénylpropyl)-1*H*-imidazol-4-yl)-1,1'-biphényle ;
*N*-benzyl(4-[1,1'-biphényl]-4-yl-1*H*-imidazol-2-yl)méthanamine;
(1*R*)-*N*-benzyl-1-(4-[1,1'-biphényl]-4-yl-1*H*-imidazol-2-yl)-2-cyclohexyléthanamine ;
(1*R*)-*N*-benzyl-1-(4-[1,1'-biphényl]-4-yl-1*H*-imidazol-2-yl)-3-phényl-1-propanamine ;
4-(2-{3-[(*tert-*butoxycarbonyl)amino]propyl}-1*H*-imidazol-4-yl)-1,1'-biphényle ;
(R,S)-1-(4-[1,1'-biphényl]-4-yl-1*H*-imidazol-2-yl)-1-pentanamine ;
*N*-[2-(4-[1,1'-biphényl]-4-yl-1*H*-imidazol-2-yl)éthyl]-1-hexanamine ;
4-[2-(2-{[(*tert*-butylamino)carbonyl]amino}éthyl)-1*H*-imidazol-4-yl]-1,1'-biphényle ;
*N*-benzyl-3-(4-[1,1'-biphényl]-4-yl-1*H*-imidazol-2-yl)-1-propanamine ;
3-(4-[1,1'-biphényl]-4-yl-1*H*-imidazol-2-yl)-1-propanamine ;
(R,S)-1-(4-[1,1'-biphényl]-4-yl-1*H*-imidazol-2-yl)-1-heptanamine;
(R,S)-4-(2-{1-[(*tert*-butoxycarbonyl)amino]heptyl}-1*H*-imidazol-4-yl)-1,1'-biphényle;
4-(2-{(1*S*)-1-[(*tert*-butoxycarbonyl)amino]propyl}-1*H*-imidazol-4-yl)-1,1'-biphényle;
(R,S)-*N*-benzyl-1-(4-[1,1'-biphényl]-4-yl-1*H*-imidazol-2-yl)-1-heptanamine;
(1*S*)-1-(4-[1,1'-biphényl]-4-yl-1*H*-imidazol-2-yl)-1-propanamine;
(1*S*)-*N*-benzyl-1-(4-[1,1'-biphényl]-4-yl-1*H*-imidazol-2-yl)-1-propanamine;
4-[2-(2-([(néopentyloxy)carbonyl]amino}éthyl)-1*H*-imidazol-4-yl]-1,1'-biphényle;
4-(2-{(1*R*)-1-[(*tert-*butoxycarbonyl)amino]butyl}-1*H*-imidazol-4-yl)-1,1'-biphényle;
(1*R*)-1-(4-[1,1'-biphényl]-4-yl-1*H*-imidazol-2-yl)-1-butanamine ;
(1*R*)-*N*-benzyl-1-(4-[1,1'-biphényl]-4-yl-1*H*-imidazol-2-yl)-1-butanamine ;
*N*-[(1*S*)-1-(4-[1,1'-biphényl]-4-yl-1*H*-imidazol-2-yl)propyl]-1-butanamine ;
4-[2-(2-{[(benzyloxy)carbonyl]amino}éthyl)-1*H*-imidazol-4-yl]-1,1'-biphényle ;
4-(2-{1-[(butoxycarbonyl)amino]-1-méthyléthyl}-1*H*-imidazol-4-yl)-1,1'-biphényle ;
4-(2-{2-[(isobutoxycarbonyl)amino]éthyl}-1*H*-imidazol-4-yl)-1,1'-biphényle ;
4-(2-{(1*S*)-1-[(butoxycarbonyl)amino]éthyl}-1*H*-imidazol-4-yl)-1,1'-biphényle ;
4-(2-{(1*R*)-1-[(butoxycarbonyl)amino]éthyl}-1*H*-imidazol-4-yl)-1,1'-biphényte ;
4-(2-{2-[(méthoxycarbonyl)amino]éthyl}-1*H*-imidazol-4-yl)-1,1'-biphényle ;
4-(2-{2-[(propoxycarbonyl)amino]éthyl}-1*H*-imidazol-4-yl)-1,1'-biphényle ;
4-(2-{2-[(éthoxycarbonyl)amino]éthyl}-1*H*-imidazol-4-yl)-1,1'-biphényle ;
4-[2-(1-{[(benzyloxy)carbonyl]amino}-1-méthyléthyl)-1*H*-imidazol-4-yl]-1,1'-biphényle;
*N*-[2-(4-[1,1'-biphényl]-4-yl-1*H*-imidazol-2-yl)éthyl]-cyclohexanamine ;
(R,S)-*N*-[1-(4-[1,1'-biphényl]-4-yl-1*H*-imidazol-2-yl)heptyl]-cyclohexanamine ;
2-(4-[1,1'-biphényl]-4-yl-1*H*-imidazol-2-yl)éthylcarbamate d'hexyle ;
4-[2-(2-{[(cyclohexyloxy)carbonyl]amino}éthyl)-1*H*-imidazol-4-yl]-1,1'-biphényle ;
4-[2-(2-{[(cyclopentyloxy)carbonyl]amino}éthyl)-1*H*-imidazol-4-yl]-1,1'-biphényle ;
2-(4-[1,1'-biphényl]-4-yl-1*H*-imidazol-2-yl)éthylcarbamate de cyclohexylméthyle ;
4-bromo-4'-(2-{2-[(butoxycarbonyl)amino]éthyl}-1*H*-imidazol-4-yl)-1,1'-biphényle ;
2-(4-[1,1'-biphényl]-4-yl-1*H*-imidazol-2-yl)éthylcarbamate de cyclobutylméthyle ;
4-[2-(2-{[(2-méthoxyéthoxy)carbonyl]amino}éthyl)-1*H*-imidazol-4-yl]-1,1'-biphényle ;
2-[4-(4'-bromo-1,1'-biphényl-4-yl)-1*H*-imidazol-2-yl]éthylcarbamate de cyclohexylméthyle ;
2-[4-(4'-bromo-1,1'-biphényl-4-yl)-1H-imidazol-2-yl]éthylcarbamate de cyclobutylméthyle ;
2-[4-(4'-bromo-1,1'-biphényl-4-yl)-1H-imidazol-2-yl]éthylcarbamate d'isobutyle ;
2-[4-(4'-bromo-1,1'-biphényl-4-yl)-1*H*-imidazol-2-yl]éthylcarbamate de cyclohexyle ;
*N*-[2-(4-[1,1'-biphényl]-4-yl-1*H*-imidazol-2-yl)éthyl]-3,3-diméthyl-butanamide. et de manière très préférentielle de formule
2-(4-[1,1'-biphényl]-4-yl-1H-imidazol-2-yl)éthylcarbamate de butyle.

La présente invention a également pour objet l'utilisation des composés tels que définis ci-dessus ou un de leurs sels pharmaceutiquement acceptable, pour préparer un médicament pour le traitement ou la prévention de la douleur, le traitement de l'épilepsie, le traitement de troubles liés à la neurodégénération et en particulier des accidents cérébraux vasculaires, du traumatisme cérébral, ou le traitement des troubles bipolaires ou du syndrome du colon irritable, et de préférence pour le traitement des accidents cérébraux vasculaires ou le traitement du traumatisme cérébral.

La présente invention a également pour à titre de médicament un composé tels que définis ci-dessus.

Dans certains cas, les composés selon la présente invention (i.e. les composés de formule générale (**I**) peuvent comporter des atomes de carbone asymétriques. Par conséquent, les composés selon la présente invention ont deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

La composition pharmaceutique peut être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

Conformément à l'invention, on peut préparer les composés de formule générale **(I)** par les procédés décrits ci-dessous.

### PREPARATION DES COMPOSES DE L'INVENTION :

### Généralités

Les préparations des composés de l'invention qui répondent aux formules générales (I), **(II)** ou **(III)** dans lesquels Ω représente OH sont effectuées de façon analogue à celles décrites dans la demande de brevet PCT WO 99/09829 et la demande de brevet européen EP 432 740.

En ce qui concerne les composés de l'invention qui répondent aux formules générales **(I), (II)** et **(III)** et dans lesquels Het est un cycle imidazole, l'homme du métier pourra aussi utilement consulter la demande de brevet PCT WO 99/64401.

Les préparations des autres composés de l'invention qui répondent aux formules générales **(I), (II)** et **(III)** sont effectuées de façon analogue à celles décrites dans la demande de brevet PCT WO 98/58934 (*cf. en particulier en pages 39 à 45 de ce document les synthèses des intermédiaires de formules générales (XXV) et (XXVIII)*) ou selon les procédures décrites ci-après.

### Préparation des composés de formule générale (I)

Les composés de formule générale **(I)** peuvent être préparés par les 8 voies synthétiques illustrées ci-dessous (schéma 1) à partir des intermédiaires de formule générale **(IV), (V), (VI), (VII), (VIII), (IX), (X)** et **(I)***bis* dans lesquels A, B, Ω, R¹, R², Het et n sont tels que définis ci-dessus, L est un groupe partant comme par exemple un halogène, Alk est un radical alkyle, Gp est un groupe protecteur pour une fonction amine, par exemple un groupement 2-(triméthylsilyl)éthoxyméthyle (SEM), et Gp' un groupe protecteur pour une fonction alcool, par exemple un groupe de type benzyle, acétate ou encore silyle comme le *tert*-butyldiméthylsilyle, et enfin A représente une liaison ou un radical -(CH₂)ₓ-, -CO-(CH₂)ₓ-, -(CH₂)_{y}-O- ou -C(=NH)-. Bien entendu, l'homme du métier pourra choisir d'utiliser d'autres groupes protecteurs Gp et Gp' parmi ceux qu'il connaît, et notamment ceux cités dans : Protective groups in organic synthesis, 2nd ed., (John Wiley & Sons Inc., 1991).

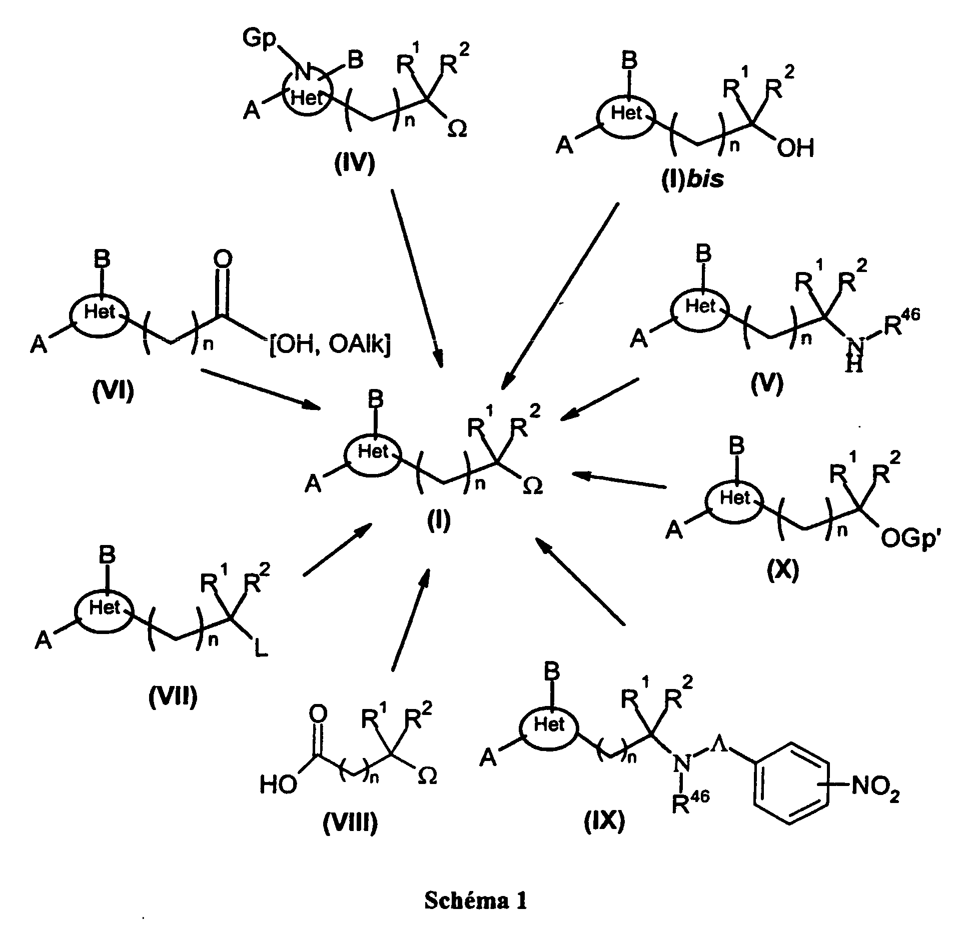

### Voie 1 : Het est imidazole et Ω est NR⁴⁶R⁴⁷ mais pas un radical de type carbamate

Les amines et les carboxamides de formule générale **(I)**, schéma 2, dans laquelle A, B, R¹, R², R⁴⁶, R⁴⁷, Het et n sont tels que définis ci-dessus, sont préparés par déprotection par exemple, dans le cas où Gp représente SEM, avec du fluorure de *tert-*butylammonium (TBAF) dans du THF, de l'amine de formule générale **(IV)** pour libérer l'amine de l'hétérocycle du composé de formule générale **(I).** Les amines protégées de formule générale **(IV)** sont accessibles par une voie générale de synthèse décrite dans Biorg. and Med Chem. Lett., 1993, 3, 915 et Tetrahedron Lett., 1993. 34, 1901 et plus particulièrement dans la demande de brevet PCT WO 98/58934.

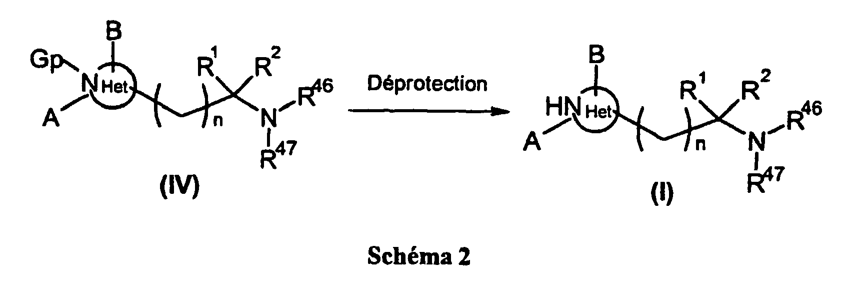

### Voie 2: Het est imidazole, oxazole ou thiazole et Ω est NR⁴⁶R⁴⁷

Les amines et les carboxamides de formule générale **(I),** schéma 3, dans lesquelles A, B, R¹, R², R⁴⁶, Het, g, k et n sont tels que définis ci-dessus, Δ représente un radical alkyle, cycloalkylalkyle, arylalkyle, aryle, allényle, allénylalkyle, alkényle, alkynyle, cyanoalkyle ou hydroxyalkyle et Δ' représente un radical alkyle, cycloalkylalkyle, arylalkyle ou aryle lorsque g ou k ne représente pas 0, ou Δ' représente un radical alkyle, cycloalkylalkyle, arylalkyle ou un radical aryle de préférence désactivé (c'est-à-dire un radical aryle substitué par un groupe attracteur d'électrons comme par exemple un groupe nitro ou cyano) lorsque g ou k représente 0, sont préparés par condensation des amines de formule générale **(V)** avec les acides carboxyliques (ou les chlorures d'acide correspondants) de formule générale **(XIII)** dans les conditions classiques de la synthèse peptidique, avec les aldéhydes de formule générale **(XII)** en présence d'un agent réducteur comme le triacétoxyborohydrure de sodium ou le borohydrure de sodium, dans un alcool aliphatique inférieur comme le méthanol et éventuellement en présence de tamis moléculaires, ou avec les dérivés halogénés (Hal = atome halogène) de formule générale **(XI).** En particulier, lorsque Δ représente un radical allényle, allénylalkyle, alkényle, alkynyle, cyanoalkyle ou hydroxyalkyle, les composés de formule générale **(V)** sont convertis en les composés de formule générale **(I)** correspondants par réaction avec les dérivés halogénés de formule générale **(XI)** dans un solvant comme l'acétonitrile, le dichlorométhane ou l'acétone et en présence d'une base comme par exemple la triéthylamine ou le carbonate de potassium à une température comprise entre la température ambiante et la température de reflux du solvant.

Les dérivés de formule générale **(V)** sont notamment accessibles par une voie générale de synthèse décrite dans Biorg. and Med. Chem. Lett., 1993, 3, 915 et Tetrahedron Lett., 1993. 34, 1901, et plus particulièrement dans la demande de brevet WO 98/58934. Lorsque R⁴⁶ = H, les composés de formule générale **(V)** peuvent être préparés, par exemple, selon un protocole décrit dans la demande de brevet WO 98/58934 (en utilisant l'acide aminé adéquat à la place du N-Boc-sarcosinamide).

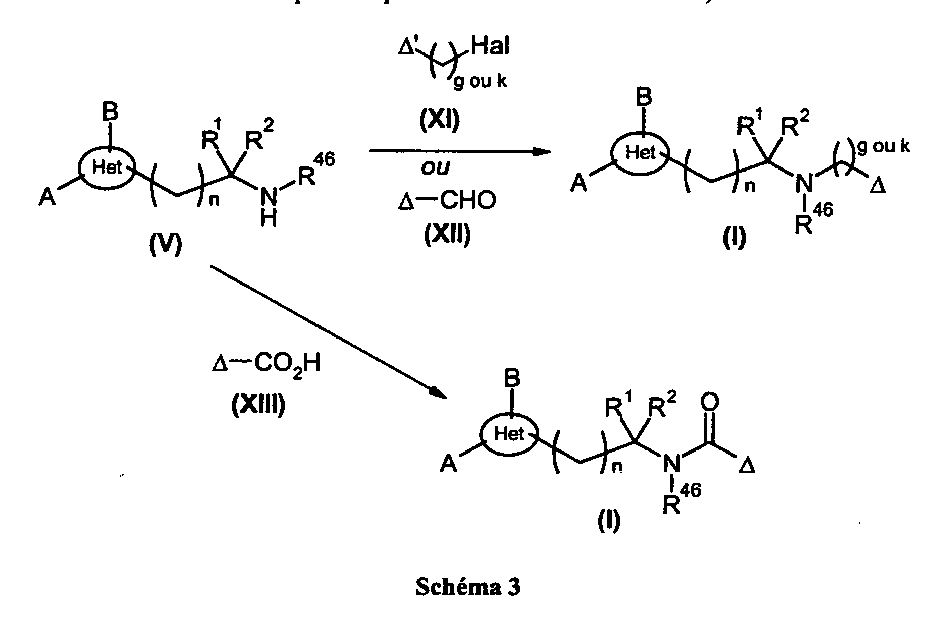

Dans le cas particulier où R⁴⁷ représente un radical cycloalkyle, les amines de formule générale **(I),** schéma *3bis,* dans laquelle A, B, R¹, R², R⁴⁶, Het et n sont tels que définis ci-dessus et i représente un entier de 0 à 4 sont préparés par condensation des amines de formule générale **(V)** avec les cycloalkylcétones de formule générale **(XIV)** en présence d'un agent réducteur comme le triacétoxyborohydrure de sodium ou le borohydrure de sodium dans un alcool aliphatique inférieur comme le méthanol et éventuellement en présence de tamis moléculaires à température ambiante.

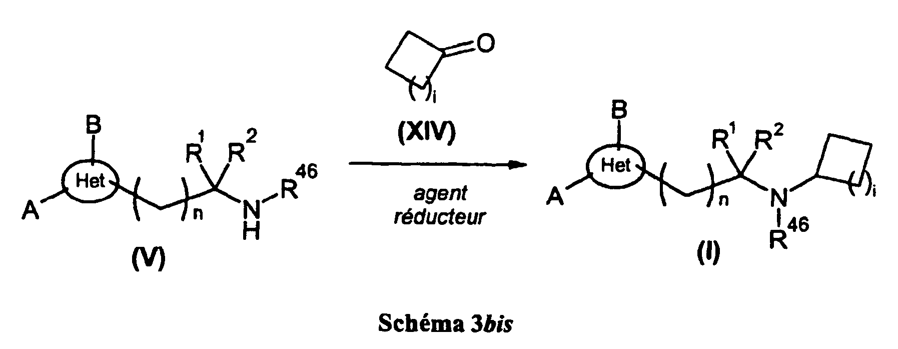

Les sulfonamides de formule générale **(I),** schéma 3*ter,* dans laquelle A, B, R¹, R², R⁴⁶, Het et n sont tels que définis ci-dessus, R⁴⁷ représente un radical -SO₂-Δ et Δ représente un radical alkyle, cycloalkyle, cycloalkylalkyle ou arylalkyle, sont préparés par condensation des amines de formule générale **(V)** avec les sulfochlorures de formule générale **(XV)** dans des conditions classiques, par exemple dans un solvant comme le diméthylformamide à température ambiante.

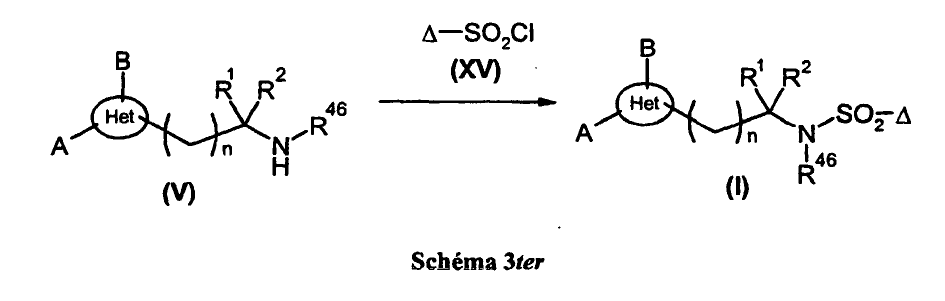

Les urées de formule générale **(I),** schéma 3*quater,* dans laquelle A, B, R¹, R², R⁴⁶, Het et n sont tels que définis ci-dessus, R⁴⁷ représente un radical -CO-NH-Δ et Δ représente un radical alkyle, cycloalkyle, cycloalkylalkyle ou arylalkyle, sont préparés par réaction des amines de formule générale **(V)** avec les isocyanates de formule générale **(XVI)** dans un solvant inerte comme le dichlorométhane ou le 1,2-dichloroéthane.

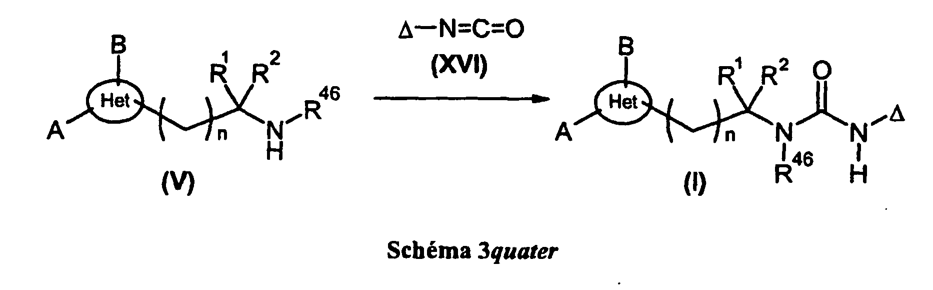

### Voie 3 : Het est oxazole ou thiazole, R¹ et R² sont tous deux H et Ω est OH.

Les dérivés alcooliques de formule générale **(I),** schéma 4, dans lesquels A, B, Het et n sont tels que définis ci-dessus et R¹ et R² sont des atomes d'hydrogène sont obtenus par réduction des acides ou esters de formule générale **(VI)** (accessibles par une voie générale de synthèse décrite dans J.Med Chem., 1996, 39, 237-245 et la demande de brevet PCT WO 99/09829). Cette réduction peut, par exemple, être effectuée par action d'hydrure de bore ou de lithium aluminium ou encore d'hydrure de diisobutylaluminium dans un solvant polaire aprotique comme le tétrahydrofuranne.

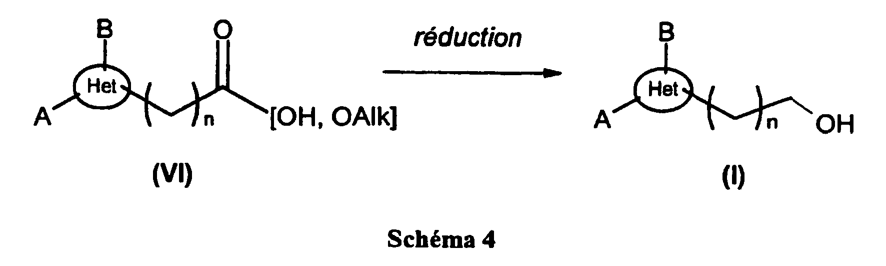

### Voie 4 : Het est oxazole ou thiazole et Ω est NR⁴⁶R⁴⁷

Les amines de formule générale **(I),** schéma 5, dans laquelles A, B, R¹, R², R⁴⁶, R⁴⁷ Het, et n sont tels que définis ci-dessus, sont préparées par condensation des amines primaires ou secondaires de formule générale R⁴⁶-NHR⁴⁷ avec les composés de formule générale **(VII)** (dans lesquels L représente de préférence un atome halogène Hal, mais peut aussi représenter un groupe mésylate ou tosylate) selon une voie générale de synthèse décrite dans J. Med. Chem., 1996, 39, 237-245 et la demande de brevet PCT WO 99/09829 ou le brevet US 4,123,529. Cette voie de synthèse peut en particulier être employée lorsque R⁴⁶ et R⁴⁷ pris ensemble forment avec l'atome d'azote qui les porte un hétérocycle non aromatique de 4 à 8 chaînons. La réaction a typiquement lieu dans un solvant anhydre (par exemple le diméthylformamide, le dichlorométhane, le tétrahydrofuranne ou l'acétone) en présence d'une base (par exemple Na₂CO₃ ou K₂CO₃ en présence de triéthylamine), et de préférence en chauffant.

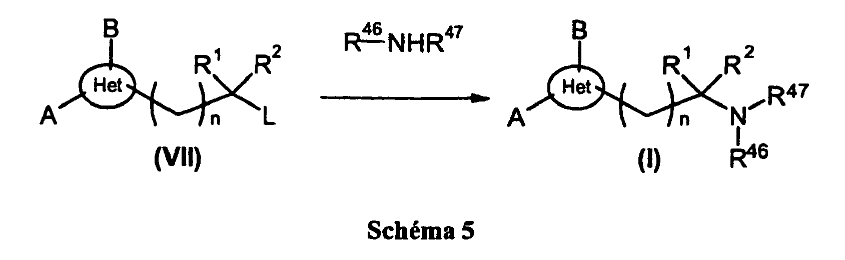

### Voie 5 : Het est imidazole et Ω est un radical de type carbamate

Lorsque Ω est un radical de type carbamate, les acides de formule générale **(VIII)** peuvent être cyclisés sous forme de dérivés d'imidazoles de formule générale **(I)**, schéma 6, par ajout de carbonate de césium suivi d'une condensation avec une α-halogénocétone de formule A-CO-CH(B)-[Br, CI] suivie de l'addition d'un large excès d'acétate d'ammonium (par exemple 15 ou 20 équivalents par équivalent d'acide de formule générale **(VIII))**. Cette réaction s'effectue de préférence dans un mélange de xylènes et en chauffant (on peut aussi, le cas échéant, éliminer simultanément l'eau formée au cours de la réaction).

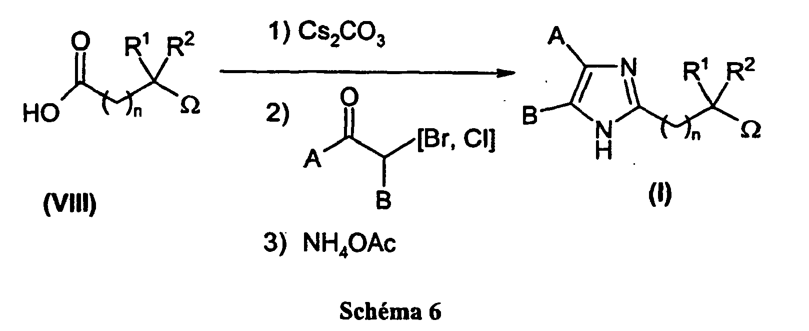

### Voie 6 : Het est imidazole, oxazole ou thiazole et Ω est NR⁴⁶R⁴⁷

Lorsque Ω est un radical NR⁴⁶R⁴⁷ dans lequel R⁴⁷ est un radical comprenant une terminaison du type aminophénylène, alkylaminophénylène ou dialkylaminophénylène, les composés de formule générale **(I),** dans laquelle A, B, Het, n, R¹, R² et R⁴⁶ sont tels que définis ci-dessus et A représente une liaison ou un radical -(CH₂)ₓ-, -CO-(CH₂)ₓ-, -(CH₂)_{y}-O- ou -C(=NH)-, x et y étant des entiers de 0 à 6, peuvent être obtenus, schéma 7, par réduction du composé de formule générale **(IX)**, par exemple par action d'hydrogène en présence d'un catalyseur du type palladium sur charbon dans un solvant comme par exemple le méthanol, l'éthanol, le dichlorométhane ou le tétrahydrofuranne. La réduction de la fonction nitro peut aussi être effectuée, par exemple, en chauffant le produit dans un solvant approprié tel que l'acétate d'éthyle avec un peu d'éthanol en présence de SnCl₂ (J. Heterocyclic Chem. (1987), 24, 927-930 ; Tetrahedron Letters (1984), 25 (8), 839-842) ou en présence de SnCl₂ / Zn (Synthesis (1996),9,1076-1078), à l'aide de NaBH₄-BiCl₃ (Synth. Com. (1995) 25 (23), 3799-3803) dans un solvant tel que l'éthanol, ou alors en utilisant du Ni Raney additionné d'hydrate d'hydrazine (Monatshefte für Chemie, (1995), 126, 725-732), ou encore à l'aide d'indium dans un mélange d'éthanol et de chlorure d'ammonium à reflux (Synlett (1998) 9, 1028).

Lorsque R⁴⁷ est un radical du type aminophénylène, alkylaminophénylène ou dialkylaminophénylène (Alk et Alk' sont des radicaux alkyle identiques ou différents),
le composé de formule générale **(IX)** est réduit pour conduire au dérivé aniline de formule générale **(I)** et éventuellement mono- ou di-alkylé selon des réactions classiques connues de l'homme du métier. La mono-alkylation est réalisée par amination réductrice avec un aldéhyde ou par une substitution nucléophile par réaction avec un équivalent d'halogénoalkyle Alk-Hal. Une deuxième alkylation peut ensuite être réalisée le cas échéant au moyen d'un halogénoalkyle Alk'-Hal.

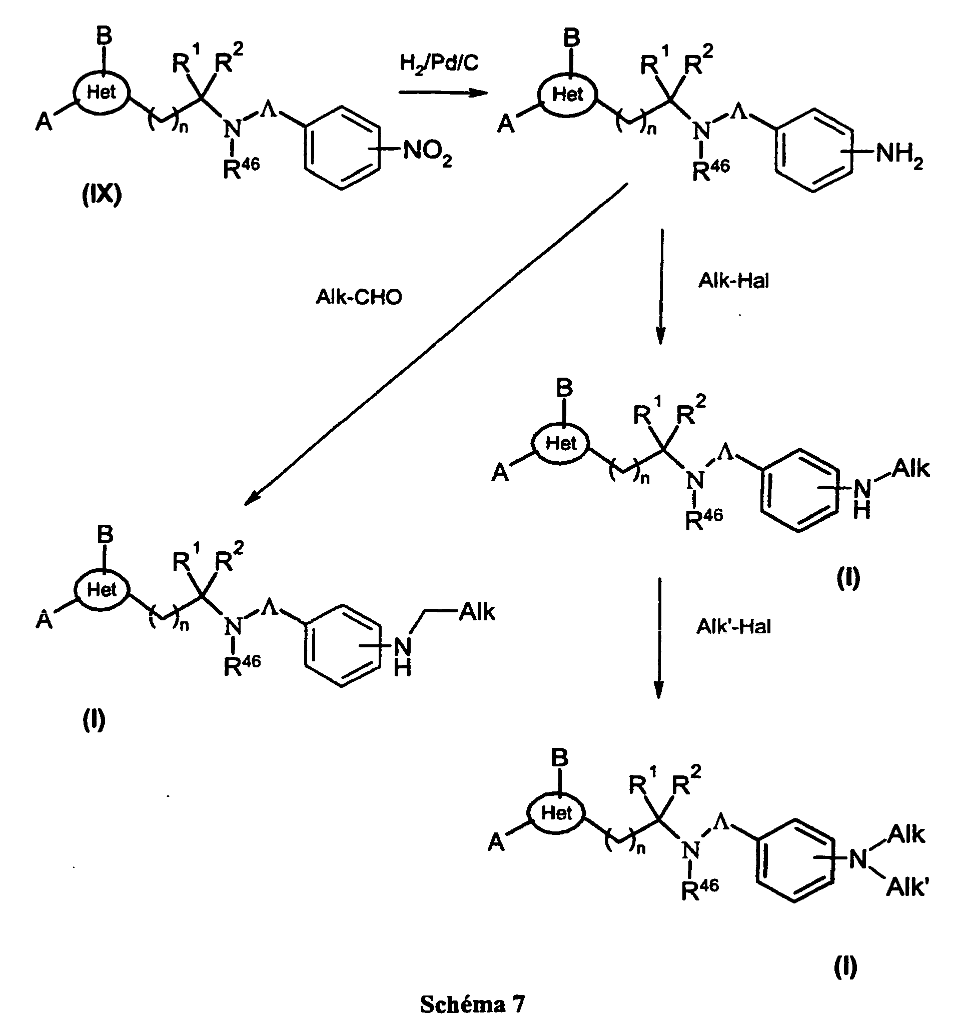

Dans le cas particulier où Alk = Alk' = -CH₃ et où A ne représente pas -CH₂-, le dérivé nitro de formule générale **(IX)** sera traité par des quantités adéquates de paraformaldéhyde sous un flux d'hydrogène dans un solvant comme l'éthanol et en présence d'un catalyseur du type palladium sur charbon (schéma 7*bis*).

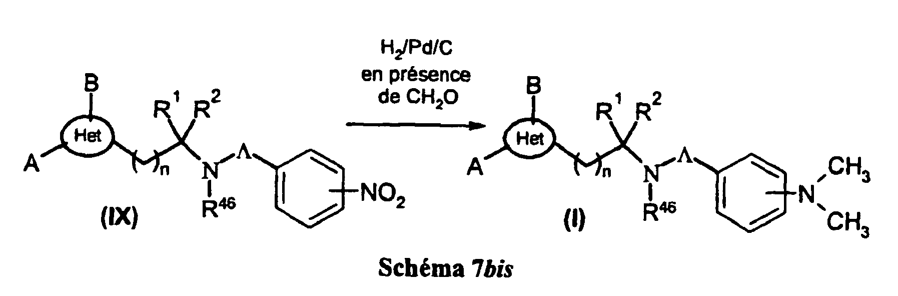

### Voie 7 : Het est imidazole, oxazole ou thiazole et Ω est OH

Cette voie peut être utilisée lorsque Ω est OH. Contrairement à la voie 3, R¹ et R² peuvent ne pas être des atomes d'hydrogène. Dans ce cas, les composés de formule générale **(I)** peuvent être obtenus, schéma 8, par déprotection de l'alcool protégé de formule générale **(X).**

Dans le cas où Gp' est un groupe protecteur de type silyle, la déprotection pourra être faite, par exemple, par addition de fluorure de tétra-*tert-*butylammonium dans un solvant comme le tétrahydrofuranne. Dans le cas où Gp' est un groupe protecteur de type benzyle, la déprotection sera faite par hydrogénation dans un solvant comme par exemple le méthanol, l'éthanol, le dichlorométhane ou le tétrahydrofuranne. Dans le cas où Gp' est un groupe protecteur de type acétate, la déprotection pourra être effectuée, par exemple, à l'aide de carbonate de sodium ou de potassium dans un solvant alcoolique comme le méthanol. Pour les autres cas, l'homme du métier consultera utilement le document suivant : Protective groupes in organic synthesis, 2nd ed., (John Wiley & Sons Inc., 1991).

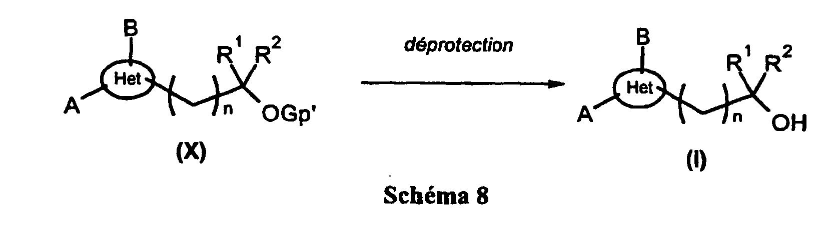

### Voie 8 : Het est imidazole, oxazole ou thiazole et Ω est OR⁴⁸ avec R⁴⁸ H

Les composés de formule générale **(I)** dans lesquels Ω est un radical OR⁴⁸ avec R⁴⁸ H sont obtenus, par exemple, schéma 9, à partir des alcools de formule générale **(I)*bis*** (qui sont des composés de formule générale **(I)** telle que définie précédemment dans laquelle Ω représente OH) par réaction de ces derniers avec un halogénure de formule générale R⁴⁸-Hal (Hal = Br, Cl ou I) dans un solvant comme le dichlorométhane, l'acétonitrile, le tétrahydrofuranne anhydre ou l'éther anhydre et en présence d'une base comme le carbonate de potassium ou de sodium, l'hydrure de sodium ou la triéthylamine.

Dans le cas où les radicaux A, B, R¹ et R² comportent des fonctions alcool, phénol, amine ou aniline, il peut être nécessaire d'ajouter des étapes de protection / déprotection de ces fonctions selon des méthodes classiques connues de l'homme du métier (étapes non représentées dans le schéma 9).

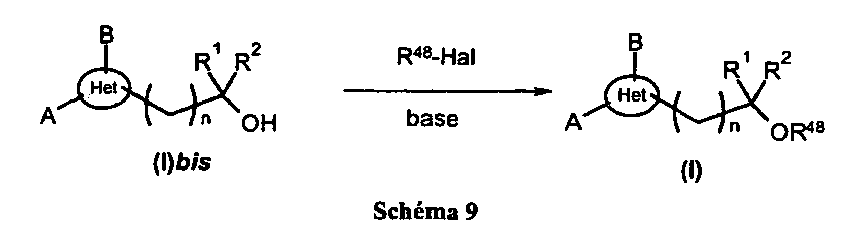

### Préparation des intermédiaires de synthèse

### Préparation des imidazoles et thiazoles de formule générale (V)

### Schéma général

Le dérivé cétonique non commercial de formule générale **(V.i)** ou **(V.i)₂** dans laquelle A et B sont tels que définis dans la formule générale **(I)** est converti, schéma 3.1, en l'α-bromo-cétone correspondante de formule générale **(V.ii)** ou **(V.ii)₂** par réaction avec un agent de bromation tel que CuBr₂ (J. Org. Chem. (1964), 29, 3459), du brome (J. Het. Chem. (1988), 25, 337), du N-bromosuccinimide (J. Amer. Chem. Soc. (1980), 102, 2838) en présence d'acide acétique dans un solvant comme l'acétate d'éthyle ou le dichlorométhane, HBr ou Br₂ dans de l'éther, de l'éthanol ou de l'acide acétique (Biorg. Med Chem. Lett. (1996), 6(3), 253-258 ; J. Med. Chem. (1988), 31(10), 1910-1918 ; J. Am. Chem. Soc. (1999), 121, 24) ou encore à l'aide d'une résine de bromation *(*J. Macromol. Sci. Chem. (1977), A11, (3) 507-514). Dans le cas particulier où A est un radical p-diméthylaminophényle, il est possible d'utiliser le mode opératoire figurant dans la publication Tetrahedron Lett., 1998, 39 (28), 4987. L'amine de formule générale **(V)** est ensuite obtenue selon les procédures représentées dans les schémas 3.2 (imidazoles) et 3.3 (thiazoles) ci-après.

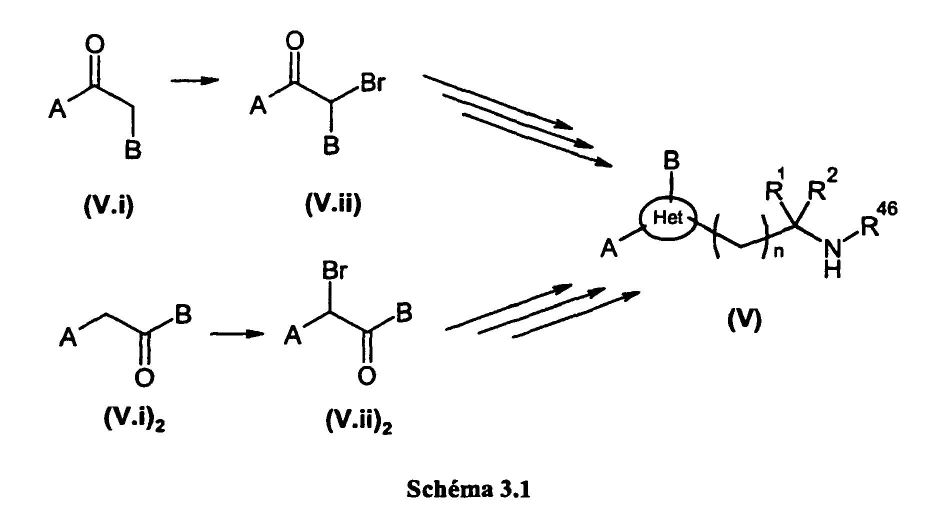

Alternativement à la synthèse présentée dans le schéma 3.1, l'homme du métier pourra, le cas échéant, utiliser une α-chloro-cétone au lieu d'une α-bromo-cétone.

### Obtention des imidazoles de formule générale (V)

L'acide de formule générale **(V.iii),** dans laquelle Gp représente un groupe protecteur pour une fonction amine, par exemple un groupe protecteur de type carbamate, est traité, schéma 3.2, avec Cs₂CO) dans un solvant tel que le méthanol ou l'éthanol. Au sel de césium récupéré est ajoutée l'α-halogéno-cétone de formule générale **(V.ii)** dans un solvant inerte tel que le diméthylformamide. Le cétoester intermédiaire cyclise par chauffage à reflux dans du xylène (mélange d'isomères) en présence d'un large excès d'acétate d'ammonium (15 ou 20 équivalents par exemple) pour donner le dérivé d'imidazole de formule générale **(V.iv)** (l'eau formée étant éventuellement éliminée en cours de réaction).

Dans le cas où R³⁸ n'est pas H, la fonction amine du cycle imidazole du composé de formule générale **(V.iv)** est substituée par réaction avec le dérivé halogéné R³⁸-Hal (Hal = atome halogène) ; la fonction amine protégée est ensuite déprotégée dans des conditions classiques (par exemple : acide trifluoroacétique ou HCl dans un solvant organique lorsqu'il s'agit d'un groupe protecteur de type carbamate, ou encore hydrogénation en présence de palladium sur charbon lorsque le groupe protecteur est un carbamate de benzyle).

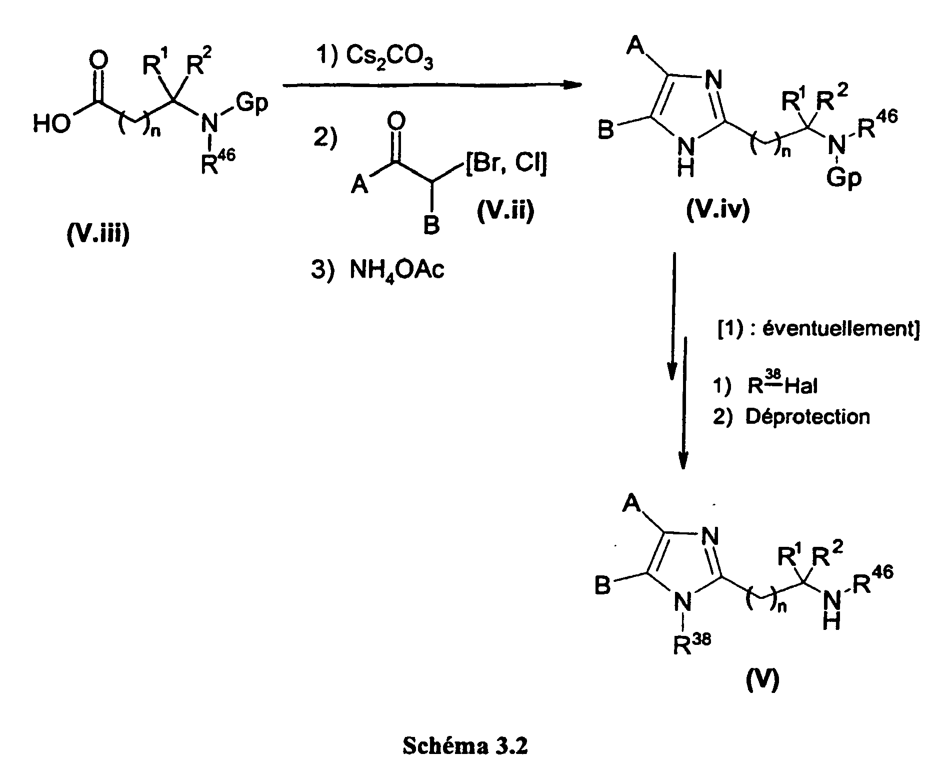

### Obtention des thiazoles de formule générale (V) destinés à la préparation de composés de formule générale (I)₁ ou (I)₂:

Le thiocarboxamide de formule générale **(V.v)**, dans laquelle Gp représente un groupe protecteur pour une fonction amine, par exemple un groupe protecteur de type carbamate, obtenu par exemple par réaction du carboxamide correspondant avec le réactif de Lawesson ou avec (P₂S₅)₂, est mis à réagir, schéma 3.3, avec l'α-bromo-cétone de formule générale **(V.ii)** ou **(V.ii)₂** selon un protocole expérimental décrit dans la littérature (J. Org. Chem., (1995), 60, 5638-5642). La fonction amine protégée est ensuite déprotégée dans des conditions classiques en milieu acide fort (par exemple : acide trifluoroacétique ou HCl dans un solvant organique lorsqu'il s'agit d'un groupe protecteur de type carbamate), libérant l'amine de formule générale **(V).**

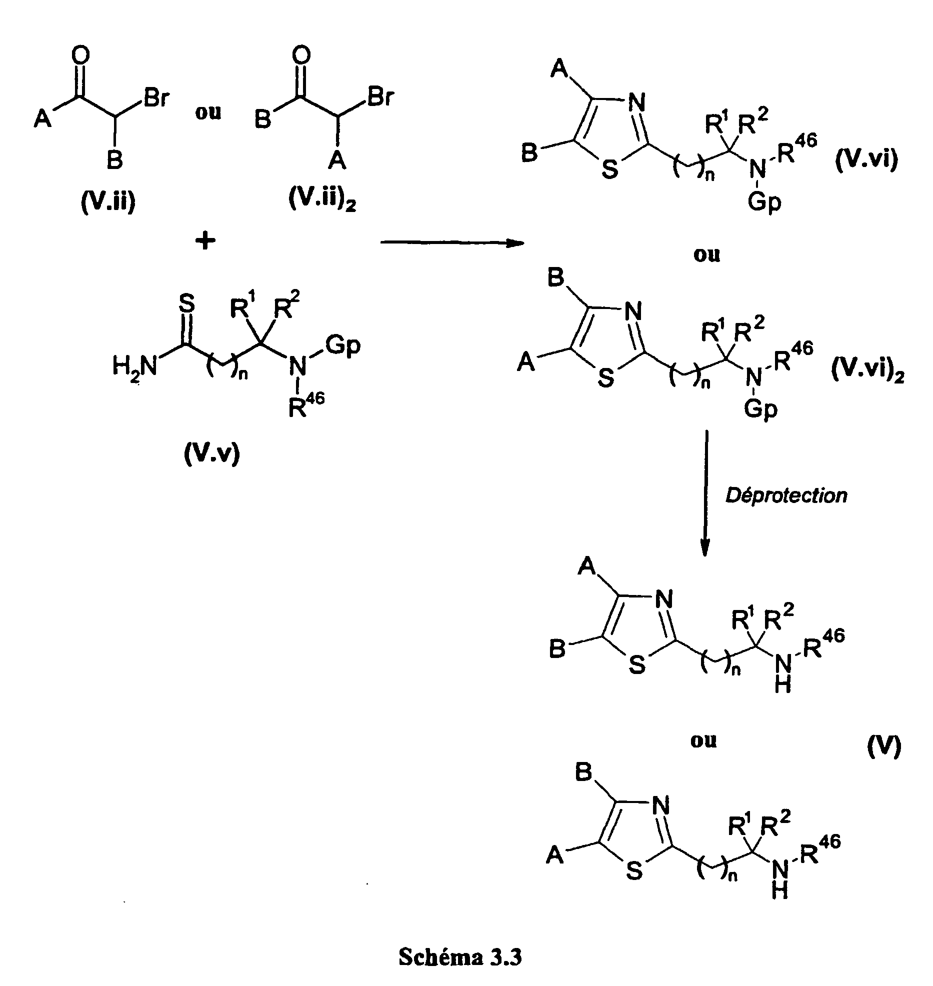

### Obtention des thiazoles de formule générale (V) destinés à la préparation de composés de formule générale (I)₃:

Ces composés sont obtenus selon une méthode résumée dans le schéma 3.4 ci-dessous. Le carboxamide de formule générale **(VII.ii)** est d'abord traité, par exemple, par le réactif de Lawesson ou avec (P₂S₅)₂ puis le thiocarboxamide de formule générale **(VII.iii)** obtenu est mis à réagir avec le dérivé halogéné de formule générale **(V.vii)** (cf. Biorg. Med. Chem. Lett. (1996), 6(3), 253-258 ; J. Med Chem. (1988), 31(10), 1910-1918 ; Tetrahedron Lett., (1993), 34 (28), 4481-4484 ; ou J. Med. Chem. (1974), 17, 369-371 ; ou encore Bull. Acd. Sci. USSR Div. Chem. Sci. (Engl Transl) (1980) 29, 1830-1833). L'aminé protégée de formule générale **(V.viii)** ainsi obtenue est ensuite déprotégée dans des conditions classiques pour l'homme du métier (par exemple : acide
trifluoroacétique ou HCl dans un solvant organique lorsque Gp est un groupe protecteur de type carbamate).

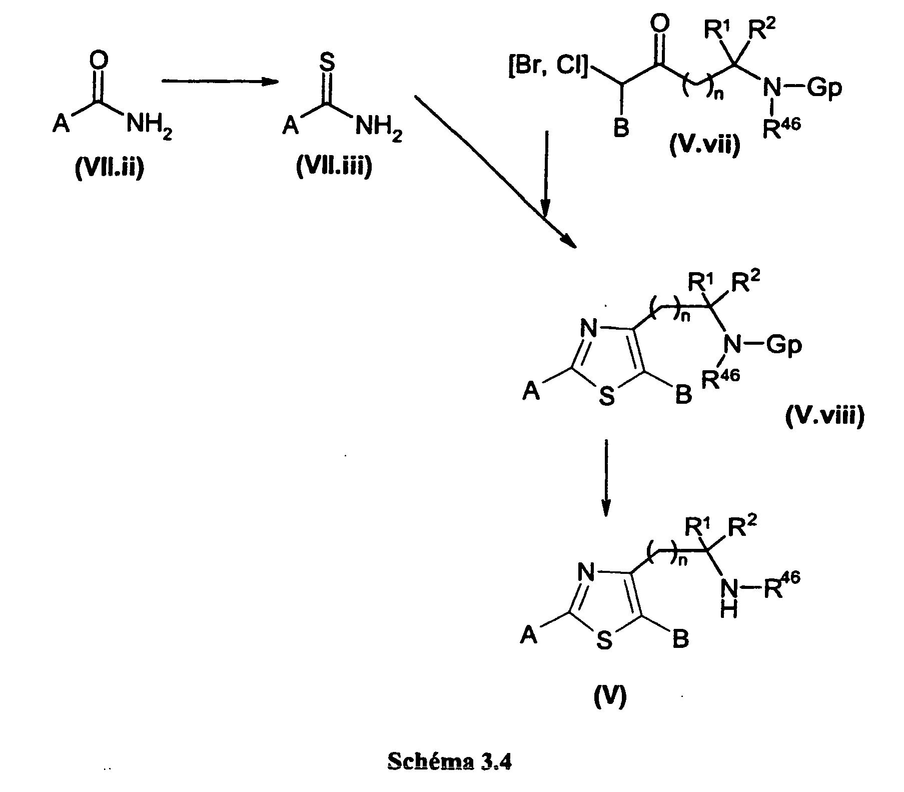

### Obtention des oxazoles de formule générale (V) destinés à la préparation de composés de formule générale (I)₃:

Ces composés sont obtenus selon une méthode résumée dans le schéma 3.5 ci-dessous. Le carboxamide de formule générale **(VII.ii)** est mis à réagir avec le dérivé halogéné de formule générale **(V.vii)**. L'amine protégée de formule générale **(V.ix)** ainsi obtenue est ensuite déprotégée dans des conditions classiques pour l'homme du métier pour donner le composé de formule générale **(V)** (par exemple : acide trifluoroacétique ou HCl dans un solvant organique lorsque Gp est un groupe protecteur de type carbamate).

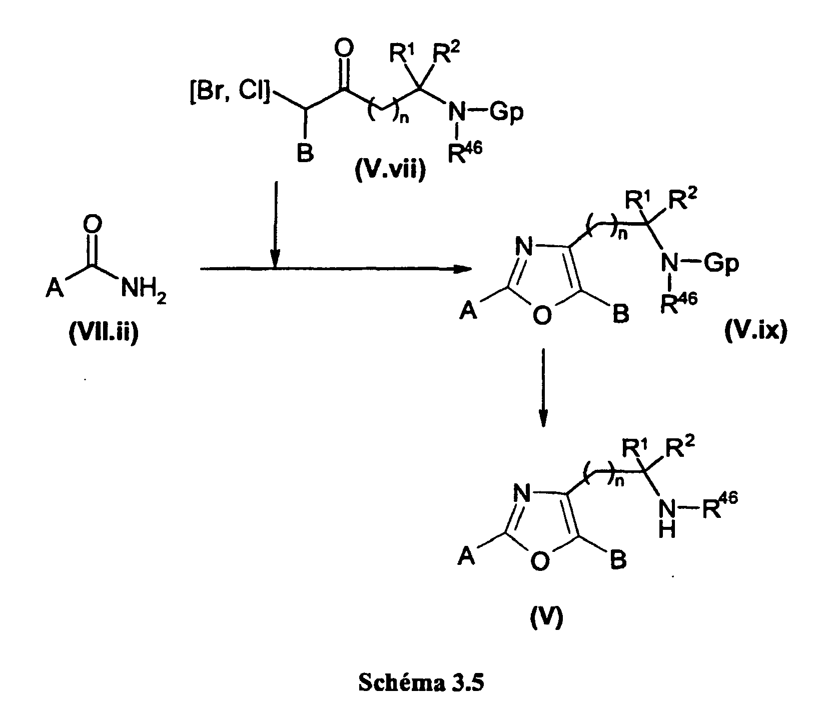

### Préparation des dérivés cétoniques de formule générale (V.i) et de certains dérivés α bromocét%IA5QWZ de formule générale (V.ii), (V.ii), ou (V.vii)

Les dérivés cétoniques de formule générale **(V.i)** non commerciaux ou leurs homologues α-bromocétoniques sont accessibles à partir de méthodes de la littérature ou de méthodes similaires que l'homme du métier aura adaptées. En particulier :
◆ lorsque A représente un radical indolinyle ou tétrahydroquinolyle, les composés de formule générale **(V.i)** sont accessibles à partir de méthodes de la littérature comme, par exemple, J. Med. Chem. (1986), 29, (6), 1009-1015 ou J. Chem. Soc., Perkin Trans. 1 (1992), 24, 3401-3406.
   Alternativement, les composés de formule générale **(V.ii)** dans lesquels A représente un radical indolinyle ou tétrahydroquinolyle dans lequel R³³ représente H peuvent être synthétisés selon un protocole légèrement modifié par rapport à celui décrit dans J. Chem. Soc., Perkin Trans 1 (1992), 24, 3401-3406. Ce protocole est résumé dans le schéma 3.6 ci-après. 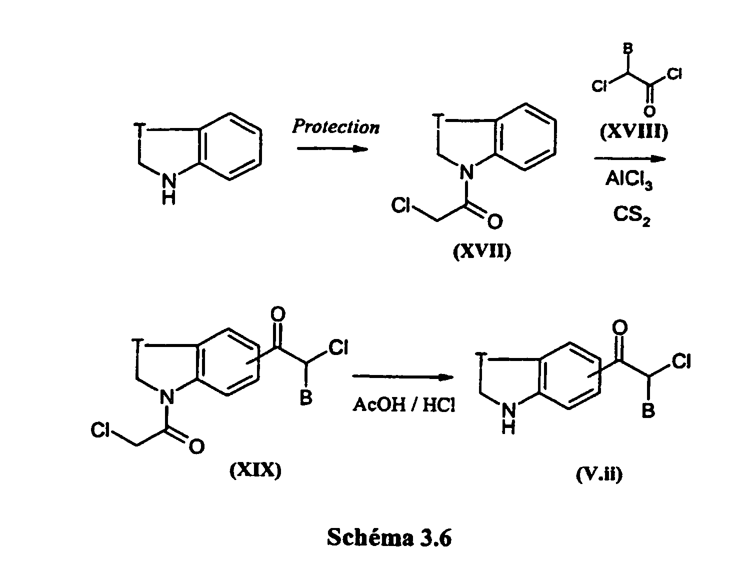 L'indoline ou la tétrahydroquinoléine (T représente -CH₂- ou -(CH₂)₂-) est protégée en utilisant le chlorure de chloroacétyle pour donner le composé de formule générale (**XVII**) qui est soumis à une réaction de Friedel-Crafts (chlorure de chloroacétyle substitué de formule générale (**XVIII**), dans laquelle B a la signification indiquée précédemment, dans un solvant comme le disulfure de carbone et en présence de chlorure d'aluminium) pour conduire au composé de formule générale **(XIX)**. Ensuite le composé de formule générale **(XIX)** est hydrolyse en présence d'acide, par exemple un mélange acide acétique/HCl, pour conduire aux composés de formule générale **(V.ii)** sous forme d'un mélange des isomères méta et para. Ces isomères peuvent être séparés par cristallisation fractionnée dans un solvant comme l'acide acétique glacial.
   L'homme du métier saura adapter les synthèses décrites précédemment aux cas où A représente un radical indolinyle ou tétrahydroquinolyle dans lequel R³³ ne représente pas H. Par exemple, lorsque R³³ représentera un radical alkyle ou aralkyle, les étapes de protection et de déprotection seront inutiles.
lorsque A représente un radical du type 4-(4-hydroxyphényl)-phényle, les composés de formule générale **(V.i)** sont accessibles à partir de méthodes de la littérature comme par exemple J. Org. Chem., (1994), 59(16), 4482-4489.
   Alternativement, les composés de formule générale **(V.i)** et **(V.ii)** dans lesquels A représente un radical du type 4-(4-hydroxyphényl)-phényle sont accessibles par exemple par la méthode illustrée dans le schéma 3.7 ci après. 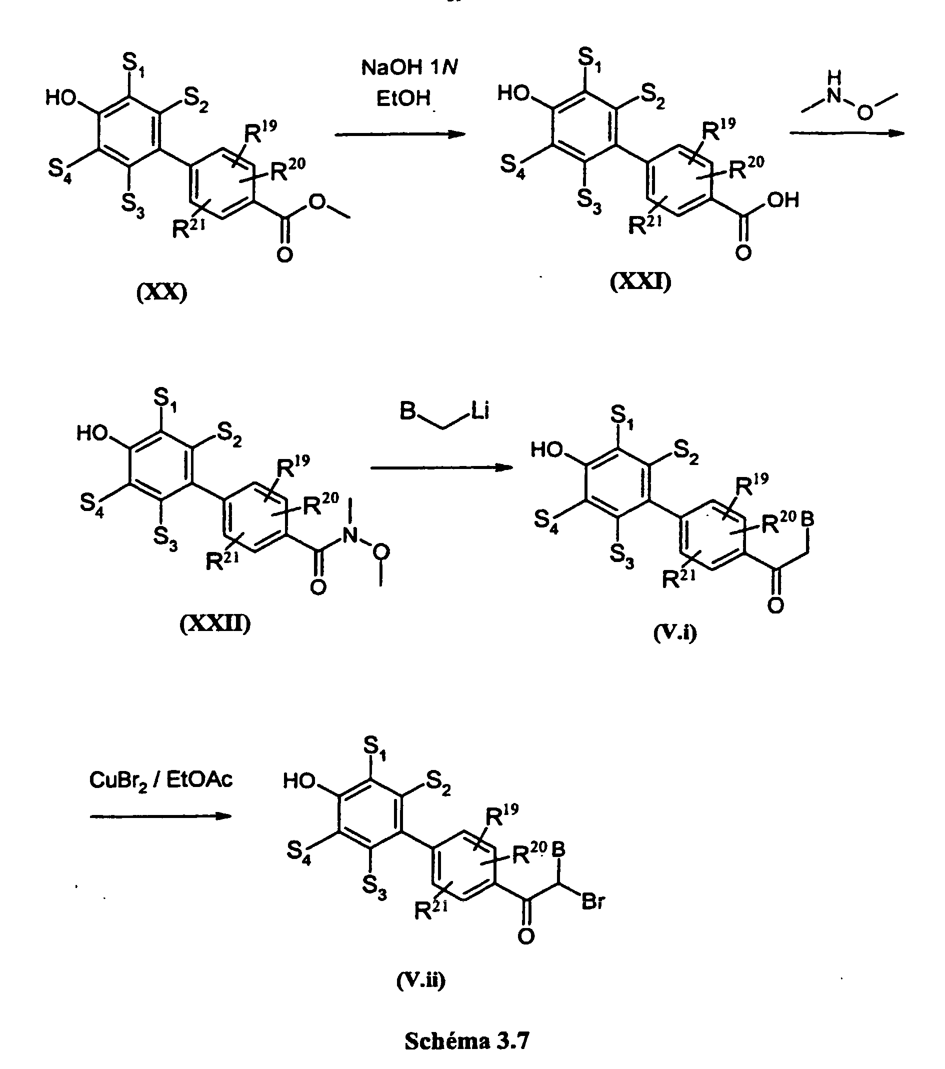 Les composés de formule générale **(V.i)** ou **(V.ii),** dans lesquels S₁, S₂, S₃ et S₄ sont choisis indépendamment parmi un atome d'hydrogène et OH, cyano, nitro, alkyle, alkoxy ou -NR¹⁰R¹¹ tel que défini dans la formule générale **(I),** sont préparés, schéma 3.7, à partir des esters de formule générale **(XX)** (cf. notamment Chem. Lett. (1998), 9, 931-932 et Synthesis (1993), 8, 788-790). Bien entendu, les fonctions phénol ou aniline résultant de la nature des substituants R¹⁹, R²⁰, R²¹, S₁, S₂, S₃ et S₄ peuvent conduire l'homme du métier à ajouter aux étapes représentées dans le schéma 3.7 des étapes de protection (et, ultérieurement dans la synthèse des composés de formule générale **(I),** de déprotection) de ces fonctions afin qu'elles n'interfèrent pas avec le reste de la synthèse chimique. Les esters de formule générale **(XX)** sont hydrolysés pour donner les acides de formule générale **(XXI)**. Ces derniers sont ensuite soumis à un couplage avec la N,O-diméthylhydroxylamine (Syn. Commun. (1995), 25(8), 1255 ; Tetrahedron Lett. (1999), 40(3), 411-414) dans un solvant comme le diméthylformamide ou le dichlorométhane, en présence d'une base telle que la triéthylamine avec du dicyclohexylcarbodiimide ou du chlorhydrate de 1-(3-diméthylaminopropyl)-3-éthylcarbodiimide et de l'hydroxybenzotriazole, pour conduire aux intermédiaires de formule générale **(XXII).** Les composés de formule générale **(V.i)** sont préparés à partir des composés de formule générale **(XXII)** par une réaction de substitution avec MeLi (J. Med. Chem. (1992), 35(13), 2392). Les bromoacétophénones de formule générale **(V.ii)** sont maintenant accessibles à partir de l'acétophénone de formule générale **(V.i)** dans des conditions précédemment décrites.
lorsque A représente un radical carbazolyle, les composés de formule générale **(V.i)** sont accessibles à partir de méthodes de la littérature comme par exemple J. Org. Chem., (1951),16, 1198 ou Tetrahedron (1980), 36, 3017.
   Alternativement, les composés de formule générale **(V.ii)** dans lesquels A représente un radical carbazolyle dans lequel R⁹ représente H peuvent être synthétisés selon un protocole légèrement modifié par rapport à celui décrit pour A = carbazolyle, dans Tetrahedron (1980), 36, 3017. Cette méthode est résumée dans le schéma 3.8 ci-après : 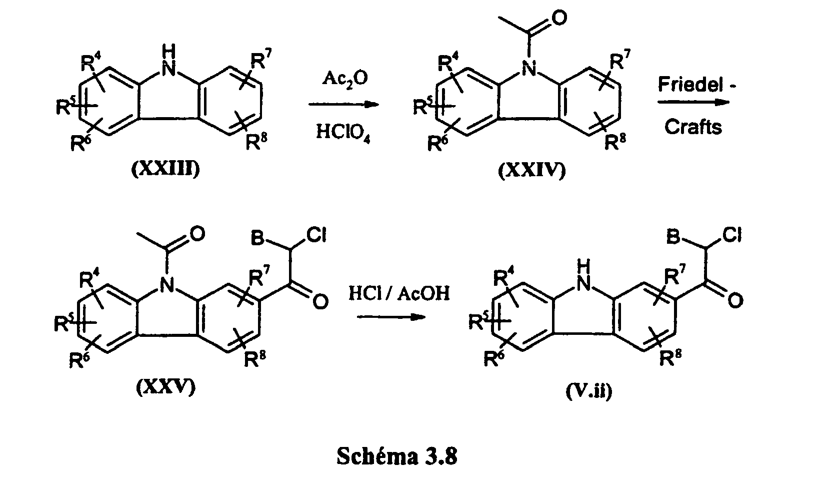 Le carbazole de formule générale **(XXIII)** est protégé en utilisant l'anhydride acétique pour donner le composé de formule générale **(XXIV)**, lequel est soumis à une réaction de Friedel-Crafts (chlorure de chloroacétyle substitué de formule générale **(XVIII)** tel que défini précédemment dans un solvant comme le disulfure de carbone et en présence de chlorure d'aluminium) pour conduire au composé de formule générale **(XXV)**. Ensuite le groupe acyle protégeant la fonction amine est hydrolyse en présence d'acide, par exemple un mélange AcOH/HCl, pour conduire au composé de formule générale **(V.ii).** Lorsque A représente un radical carbazolyle dans lequel R⁹ représente alkyle ou un groupe -COR¹⁵ (cas non représenté dans le schéma 3.8), l'étape d'acylation initiale est inutile et les deux dernières étapes du schéma 3.8 permettent d'obtenir les composés de formule générale **(V.ii)**. Bien entendu, les fonctions phénol ou aniline résultant de la nature des substituants R⁴, R⁵, R⁶, R⁷ et R⁸ peuvent conduire l'homme du métier à ajouter aux étapes représentées dans le schéma 3.8 des étapes de protection (et, ultérieurement dans la synthèse des composés de formule générale **(I)**, de déprotection) de ces fonctions afin qu'elles n'interfèrent pas avec le reste de la synthèse chimique.
lorsque A représente un radical phénothiazinyle, les intermédiaires de formule générale **(V.i)** et **(V.ii)** sont accessibles à partir de méthodes de la littérature : J. Heterocyclic. Chem. (1978), 15, 175-176 et Arzneimittel Forschung (1962), 12, 48.
   Alternativement, les intermédiaires de formule générale **(V.ii)** dans lesquels A représente un radical phénothiazinyle peuvent être préparés selon un protocole légèrement modifié par rapport à celui décrit pour le radical phénothiazinyle dans Arzneimittel Forschung (1962), 12, 48, lequel est résumé dans le schéma 3.9 ci-après (voir aussi les exemples). La phénothiazine de formule générale **(XXVI)** est protégée en utilisant le chlorure de chloroacétyle pour donner le composé de formule générale **(XXVII)**, lequel est ensuite soumis à une réaction de Friedel-Crafts (composé de formule générale **(XVIII)** dans un solvant comme le disulfure de carbone en présence de chlorure d'aluminium) pour conduire au composé de formule générale **(XXVIII).** Lors de la dernière étape du procédé, l'hydrolyse avec HCl/acide acétique est accompagnée d'un échange d'halogène et permet d'obtenir la chlorocétone de formule générale **(V.ii)**. Bien entendu, les fonctions phénol ou aniline résultant de la nature des substituants R⁴, R⁵, R⁶, R⁷ et R⁸ peuvent conduire l'homme du métier à ajouter aux étapes représentées dans le schéma 3.9 des étapes de protection (et, ultérieurement dans la synthèse des composés de formule générale **(I)**, de déprotection) de ces fonctions afin qu'elles n'interfèrent pas avec le reste de la synthèse chimique. 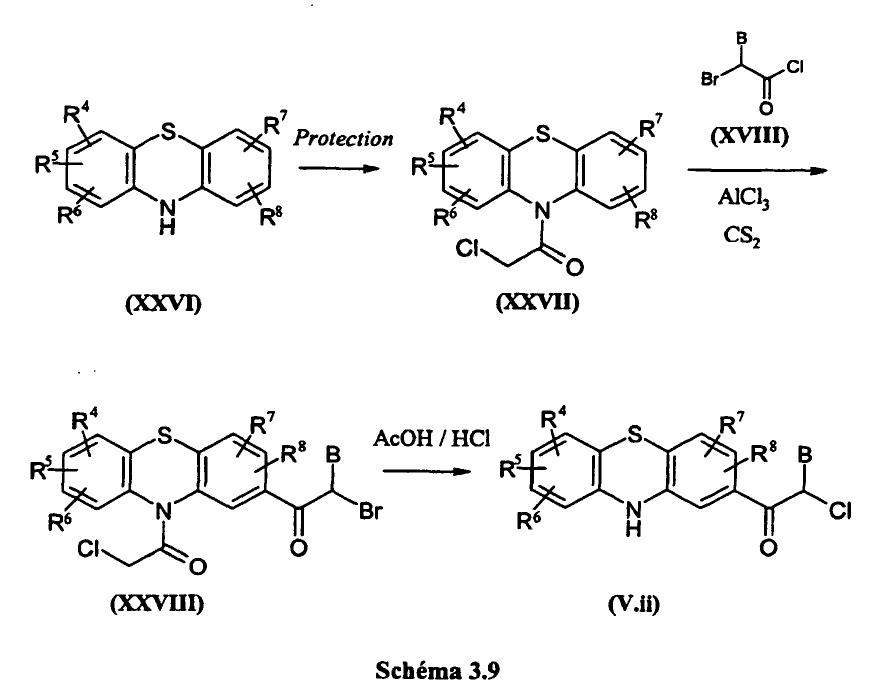
◆ lorsque A représente un radical phénylaminophényle, les composés de formule générale **(V.i)** sont accessibles à partir de méthodes de la littérature comme par exemple Chem. Commun., (1998), 15, (6) 1509-1510 ou Chem Ber., (1986), 119, 3165-3197, ou de méthodes similaires que l'homme du métier aura adaptées.
   Par exemple, les intermédiaires de formule générale **(V.i)*bi*s** et **(V.ii)*bis*** dans lesquels A représente un radical phénylaminophényle (qui correspondent aux composés de formule générale **(V.i)** et **(V.ii)** correspondants dont la fonction aniline a été acétylée), peuvent être préparés selon un protocole légèrement modifié par rapport à celui décrit pour le radical phénylaminophényle dans Chem Ber. (1986), 119, 3165-3197. Ce protocole est résumé dans le schéma 3.10 ci-après. 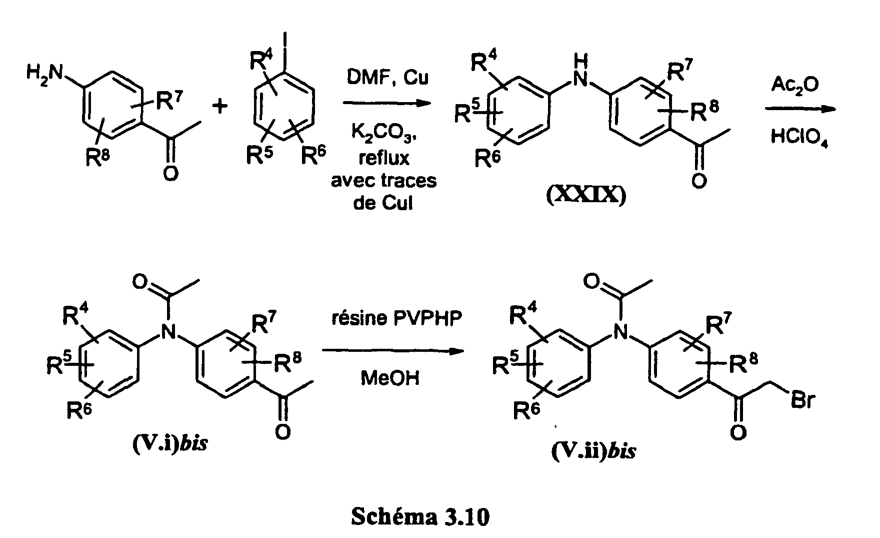 Dans le cas (représenté sur le schéma 3.10) où le radical R⁹ du composé de formule générale **(I)** à synthétiser est un atome d'hydrogène ou un groupe acétyle, la diphénylamine de formule générale **(XXIX)** formée après la réaction de couplage en présence de CuI est protégée par acétylation en utilisant, par exemple, l'anhydride acétique pour donner le composé de formule générale (V.i)*bis*. Dans le cas (non représenté sur le schéma 3.10) où le radical R⁹ du composé de formule générale **(I)** à synthétiser n'est pas un atome d'hydrogène ou un radical acétyle, l'étape d'acétylation est remplacée par une étape de substitution sur l'aniline selon des méthodes classiques connues de l'homme du métier pour donner le composé de formule générale **(V.i)** correspondant. Le composé de formule générale **(V.i)*bis*** (ou **(V.i)**, dans le cas non représenté dans le schéma 3.10) est ensuite soumis à une réaction de bromation à l'aide d'une résine de bromation, la résine PVPHP (*Poly(VinylPyridinium Hydrobromide Perbromide)* ou poly(perbromure d'hydrobromure de vinylpyridinium)), décrite dans J. Macromol. Sci. Chem. (1977), A11, (3), 507-514, pour conduire au composé de formule générale **(*V.ii*)*****bis*** (ou **(V.ii)**, dans le cas non représenté dans le schéma 3.10). Bien entendu, les fonctions phénol ou aniline résultant de la nature des substituants R⁴, R⁵, R⁶, R⁷ et R⁸ peuvent conduire l'homme du métier à ajouter aux étapes représentées dans le schéma 3.10 des étapes de protection (et, ultérieurement dans la synthèse des composés de formule générale **(I)**, de déprotection) de ces fonctions afin qu'elles n'interfèrent pas avec le reste de la synthèse chimique. La déprotection de la fonction aniline acétylée sera effectuée en principe lors de la dernière étape de la synthèse des composés de formule générale **(I).**
◆ lorsque A représente un radical benzopyrane ou benzofuranne tel que défini dans la formule générale **(I)** avec R³² représentant un atome d'hydrogène, les intermédiaires de formule générale **(V.i)** et **(V.ii)** sont accessibles par les méthodes illustrées dans le schéma 3.11 ci après. 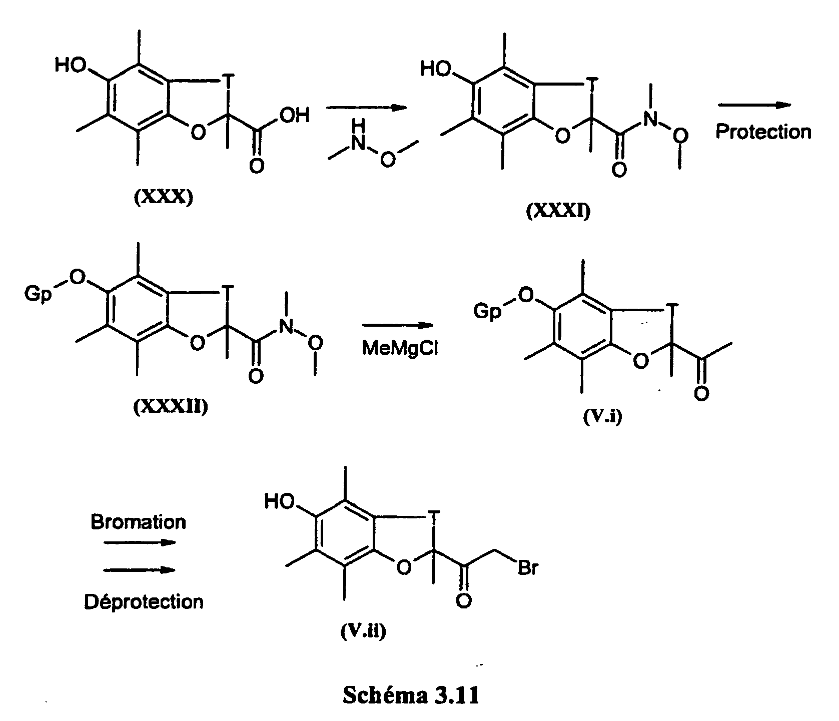 Les composés de formules générales **(V.i)** et **(V.ii)**, schéma 3.11, dans laquelle T est tel que défini ci-dessus et Gp = groupe protecteur, sont préparés à partir des acides de formule générale **(XXX)**. Les acides de formule générale **(XXX)** sont soumis à un couplage avec la N,O-diméthylhydroxylamine (Syn. Commun. (1995), 25, (8), 1255 ; Tetrahedron Lett. (1999), 40, (3), 411-414) dans un solvant comme le diméthylformamide ou le dichlorométhane, en présence d'une base telle que la triéthylamine avec du dicyclohexylcarbodiimide ou du chlorhydrate de 1-(3-diméthylaminopropyl)-3-éthylcarbodiimide et de l'hydroxybenzotriazole, pour conduire aux intermédiaires de formule générale **(XXXI).** La protection de la fonction phénol sous forme de dérivé benzylé ou *tert-*butyldiméthylsilylé ou par d'autres groupes protecteurs (Gp) connus de l'homme du métier est alors effectuée pour conduire aux composés de formule générale **(XXMI)**. Les composés de formule générale **(V.i)** sont préparés à partir des composés de formule générale **(XXXII)** par une réaction de substitution avec un réactif de Grignard, MeMgCl (J. Het. Chem. (1990), 27, 1709-1712) ou avec MeLi (J. Med Chem. (1992), 35, 13). Les bromoacétophénones de formule générale **(V.ii)** sont maintenant accessibles à partir de l'acétophénone de formule générale **(V.i)** dans des conditions précédemment décrites.
   Alternativement, le composé de formule générale **(V.ii)** dans lequel R³² représente un atome d'hydrogène ou un radical alkyle peut être préparé selon un procédé en 3 étapes seulement (cf. schéma 3.12 - voir aussi les exemples). Dans ce procédé, la bromation dans la dernière étape du composé de formule générale **(V.i)** pour donner le composé de formule générale **(V.ii)** sera de préférence effectuée selon J. Am. Chem. Soc. (1999),121, 24. 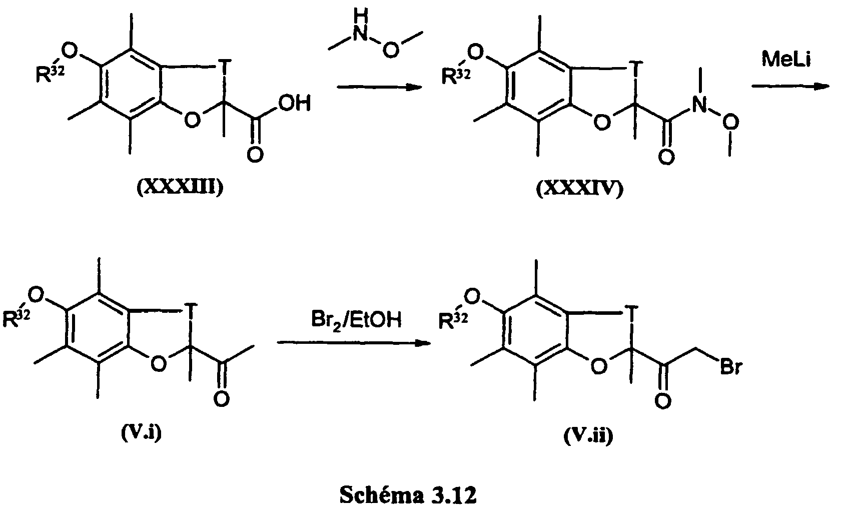 Lorsque A représente un radical phénol substitué, il peut être nécessaire de d'utiliser des intermédiaires de formule générale **(V.ii)** telle que définie précédemment dont la fonction phénol a été acétylée (ci-après désignés comme composés de formule générale **(V.ii)*ter*).** En particulier :
◆ lorsque A représente un radical 4-hydroxy-3,5-diisopropylphényle, les dérivés α-bromocétoniques homologues du composé de formule **(V.ii)** dont la fonction phénol est protégée par un radical acétyle peuvent être préparés comme résumé dans le schéma 3.13 ci-après. 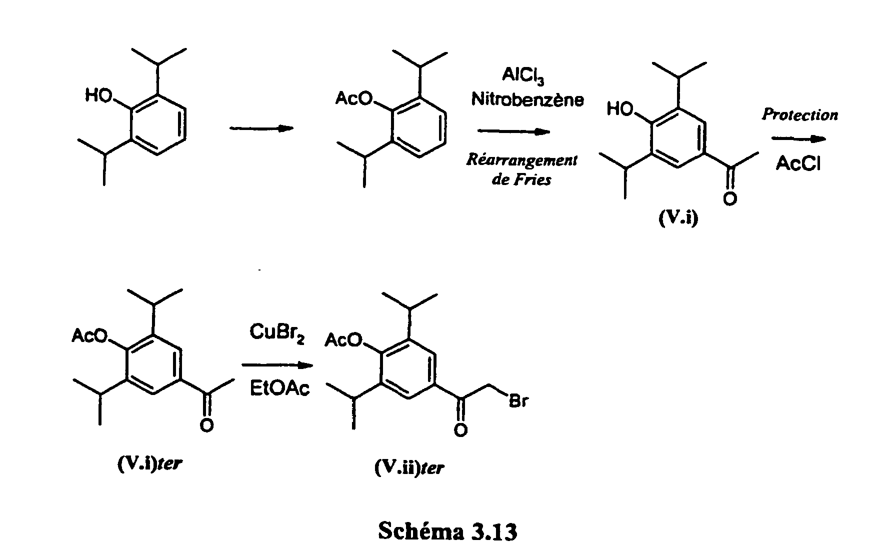 Le 2,6-diisopropylphénol est acétylé selon des méthodes connues de l'homme du métier, par exemple en le faisant réagir avec de l'acide acétique en présence d'anhydride d'acide trifluoroacétique ou avec du chlorure d'acétyle en présence d'une base comme par exemple K₂CO₃. L'homologue acétylé du 2,6-diisopropylphénol est alors soumis à un réarrangement de Fries en présence de chlorure d'aluminium dans un solvant comme le nitrobenzène pour conduire au composé de formule **(V.i).** Ensuite le composé de formule **(V.i)** est acétylé pour conduire au composé de formule **(V.i)*ter*.** Une bromation est alors effectuée avec CuBr₂ comme précédemment décrit pour conduire au composé de formule **(V.ii)*ter*.** L'étape de déprotection pour libérer la fonction phénol interviendra ultérieurement dans la synthèse des composés de formule générale **(I)** (au moment jugé le plus adapté par l'homme du métier).
lorsque A représente un radical de type diméthoxyphénol, les composés de formule générale **(V.ii)*ter*** peuvent être préparés de façon analogue à la synthèse décrite pour le composé de formule **(V.ii)*ter*** dérivé du 2,6-diisopropylphénol, avec éventuellement quelques modifications mineures à la portée de l'homme du métier. Par exemple, lorsque A représente le radical 3,5-diméthoxy-4-hydroxyphényle, le dérivé α-bromocétonique de formule **(V.ii)*ter*** correspondant peut être préparé, par exemple, comme indiqué dans le schéma 3.13 à partir du composé commercial de formule **(XXXV):** 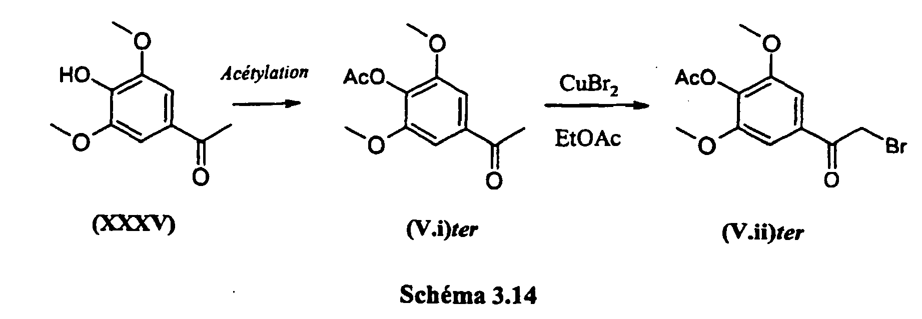 Les composés de formule générale **(V.ii)₂** dans laquelle A et B sont tels que définis précédemment peuvent être préparés selon la méthode résumée dans le schéma 3.15 ci-après. 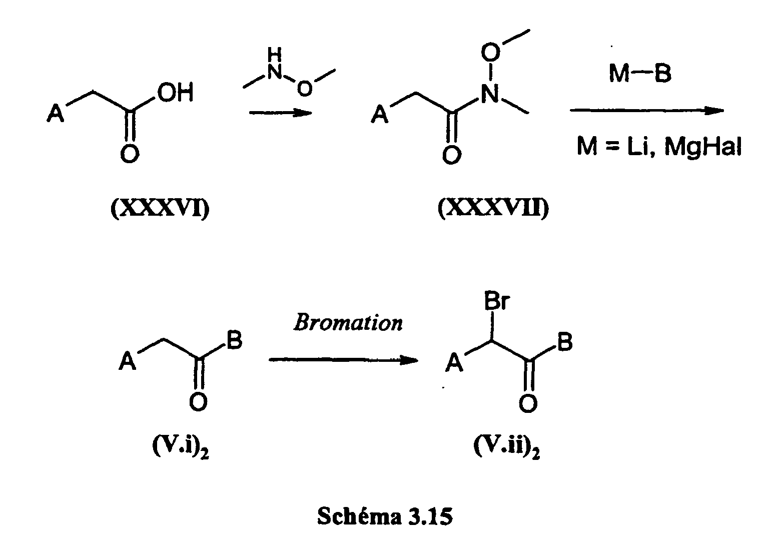 Les acides de formule générale **(XXXVI)** sont soumis à un couplage avec la N,O-diméthylhydroxylamine (Syn. Commun. (1995), 25, (8), 1255 ; Tetrahedron Lett. (1999), 40, (3), 411-414) dans un solvant comme le diméthylformamide ou le dichlorométhane, en présence d'une base telle que la triéthylamine avec du dicyclohexylcarbodiimide ou du chlorhydrate de 1-(3-diméthylaminopropyl)-3-éthylcarbodiimide et de l'hydroxybenzotriazole, pour conduire aux intermédiaires de formule générale **(XXXVII).** Les composés de formule générale **(V.i)₂** sont préparés à partir des composés de formule générale **(XXXVII)** par une réaction de substitution avec des dérivés lithiens ou magnésiens de formule générale B-M dans laquelle M représente Li ou MgHal (Hal = I, Br ou Cl) dans des solvants comme l'éther ou le tétrahydrofuranne anhydre. Les α-bromo- ou α-chlorocétones de formule générale
   **(V.ii)₂** sont maintenant accessibles à partir des cétones de formule générale **(V.i)₂** dans des conditions précédemment décrites.

Par ailleurs, les dérivés α-halogénocétoniques de formule générale **(V.vii)** non commerciaux sont accessibles à partir de méthodes de la littérature. En particulier, ils peuvent être obtenus selon une procédure résumée dans le schéma 3.16.

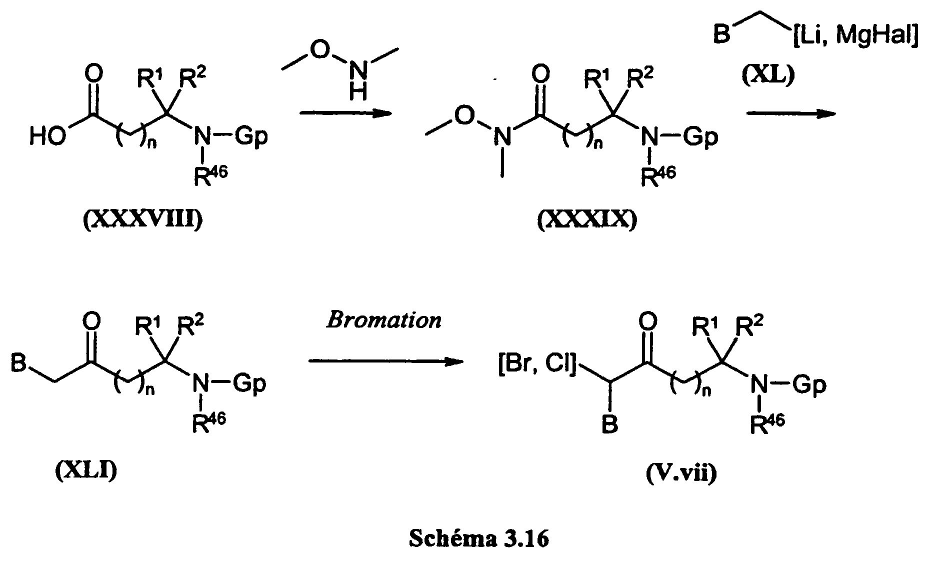

Les aminoacides protégés de formule générale **(XXXVIII)** sont obtenus par la protection des aminoacides correspondants par un groupe de type carbamate selon des méthodes connues de l'homme du métier. Les acides de formule générale **(XXXVIII)** sont ensuite soumis à un couplage avec la N,O-diméthylhydroxylamine (Syn. Commun. (1995), 25, (8), 1255; Tetrahedron Lett. (1999), 40, (3), 411-414) dans un solvant comme le diméthylformamide ou le dichlorométhane, en présence d'une base telle que la triéthylamine avec du dicyclohexylcarbodiimide ou du chlorhydrate de 1-(3-diméthylaminopropyl)-3-éthylcarbodiimide et de l'hydroxybenzotriazole, pour conduire aux intermédiaires de formule générale **(XXXIX).** Les composés de formule générale **(XLI)** sont préparés à partir des composés de formule générale **(XXXIX)** par une réaction de substitution avec des dérivés lithiens ou magnésiens de formule générale **(XL)** (dans laquelle Hal = I, Br ou Cl) dans des solvants comme l'éther ou le tétrahydrofuranne anhydre. Les bromo ou chloroacétophénones de formule générale **(V.vii)** sont maintenant accessibles à partir de l'acétophénone de formule générale **(XLI)** dans des conditions précédemment décrites.

Alternativement, l'homme du métier pourra aussi utiliser ou adapter les synthèses décrites dans Angew. Chem. Int. (1998), 37 (10), 411-414, Liebigs Ann. Chem. (1995), 1217 ou Chem. Pharm. Bull. (1981), 29(11), 3249-3255.

### Préparation des dérivés acides de formule générale (V.iii)

Les dérivés acides de formule générale **(V.iii)** peuvent être obtenus, schéma 3.17, directement par réaction de l'aminoacide commercial de formule générale **(V.vi)** avec des composés de type (ar)alkylchloroformates ou di(ar)alkylcarbonates (Δ représente un radical alkyle ou benzyle) dans des conditions classiques connues de l'homme du métier.

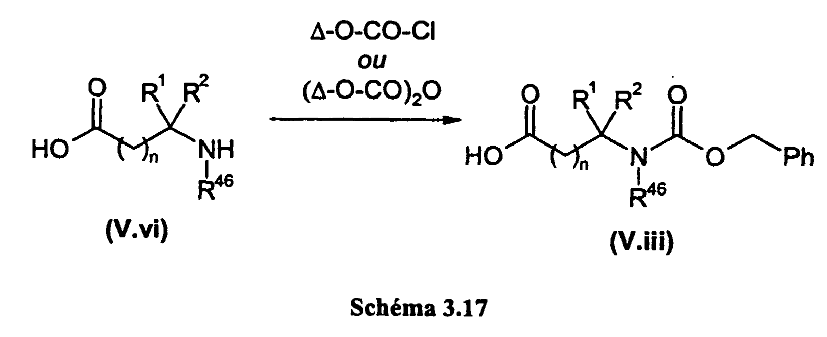

### Préparation des composés de formule générale (V.v)

Les thiocarboxamides de formule générale **(V.v)** peuvent être obtenus en trois étapes à partir des composés de formule générale **(V.vi)** comme indiqué dans le schéma 3.18 ci-dessous. La fonction amine de l'aminoacide de formule générale **(V.vi)** est d'abord protégée dans des conditions classiques avec tBu-O-CO-Cl ou (tBu-O-CO)₂O (ou d'autres groupes protecteurs connus de l'homme du métier), puis l'intermédiaire obtenu est converti en son amide correspondant par des méthodes décrites dans la littérature (cf. par exemple, J. Chem. Soc., Perkin Trans. 1, (1998), 20, 3479-3484 ou la demande de brevet PCT WO 99/09829). Enfin, le carboxamide est converti en thiocarboxamide de formule générale **(V.v)**, par exemple par réaction avec le réactif de Lawesson dans un solvant comme le dioxane ou le tétrahydrofuranne à une température de préférence comprise entre la température ambiante et celle du reflux du mélange, ou encore à l'aide de (P₂S₅)₂ dans des conditions classiques pour l'homme du métier. 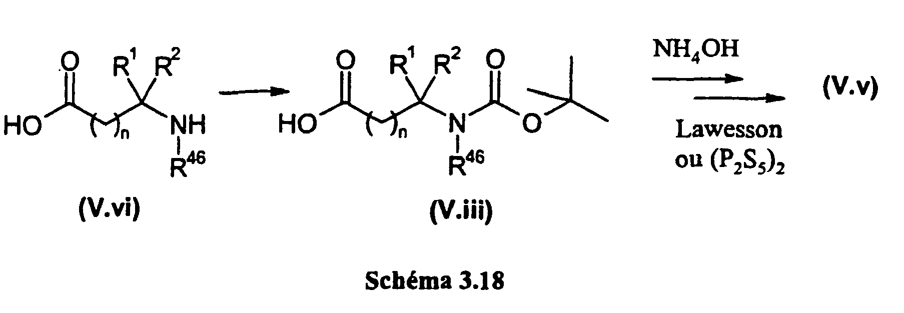 Alternativement, les thiocarboxamides de formule générale **(V.v)** peuvent également être obtenus, schéma 3.19, par addition de H₂S sur les dérivés cyano de formule générale **(V.x)** correspondants dans des conditions classiques connues de l'homme du métier.

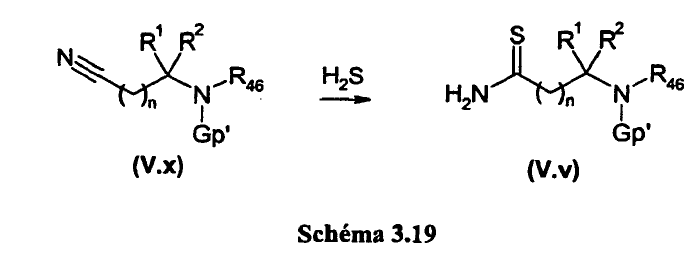

### Préparation des acides de formule générale (VI)

### Préparation des acides dérivés de thiazoles de formule générale (VI)

Les acides de formule générale **(VI)** dérivés de thiazoles peuvent être préparés selon des procédures représentées dans le schéma 4.1 ci-dessous.

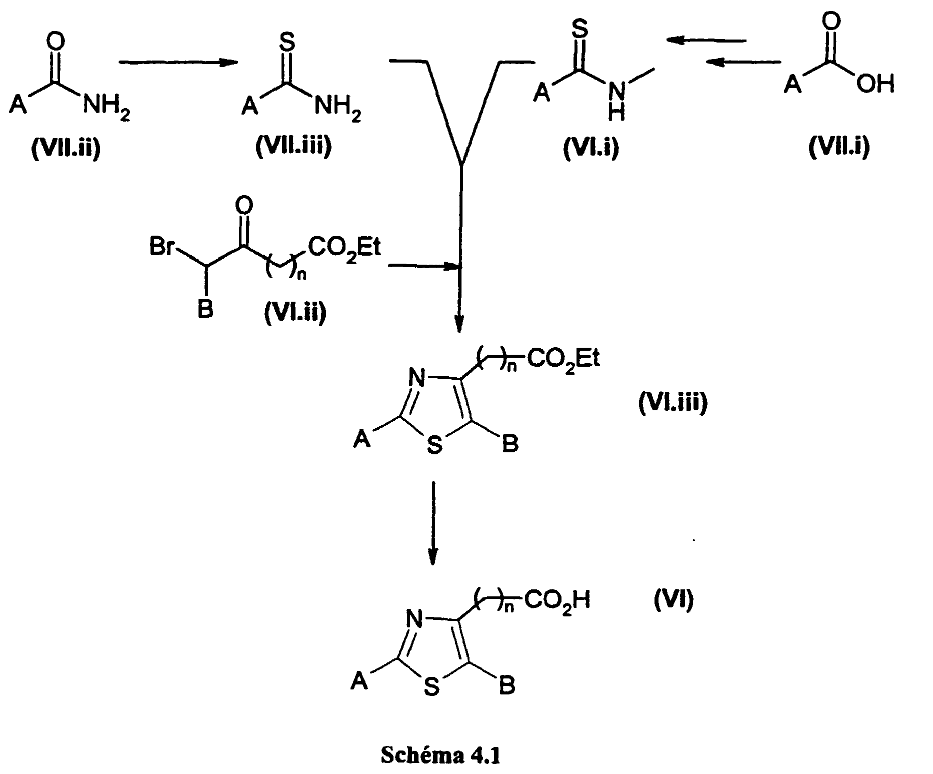

Les carboxamides de formule générale **(VII.ii)** sont traités dans des conditions classiques pour donner le thiocarboxamide de formule générale (**VII.iii**), par exemple par le réactif de Lawesson ou encore à l'aide de (P₂S₅)₂ dans des conditions classiques pour l'homme du métier. Alternativement, l'acide de formule générale **(VII.i)** est activé par action de 1,1'-carbonyldiimidazole puis traité par de la méthylamine dans un solvant polaire aprotique comme par exemple le tétrahydrofuranne. Le carboxamide intermédiaire obtenu est converti en le thiocarboxamide de formule générale **(VI.i)** dans des conditions classiques, par exemple à l'aide du réactif de Lawesson ou encore à l'aide de (P₂S₅)₂ dans des conditions classiques pour l'homme du métier. Le thiocarboxamide de formule générale **(VII.iii)** ou **(VI.i)** est ensuite mis à réagir avec le composé de formule générale **(VI.ii)**, par exemple en chauffant à reflux dans un solvant comme le benzène, le dioxane ou le diméthylformamide. L'ester de formule générale **(VI.iii)** obtenu peut ensuite être saponifié par action d'une base comme par exemple la potasse en milieu alcoolique ou LiOH dans du tétrahydrofuranne pour donner l'acide de formule générale **(VI).**

### Préparation des acides dérivés d'oxazoles de formule générale (VI)

Les acides de formule générale **(VI)** dérivés d'oxazoles peuvent être préparés selon une procédure représentée dans le schéma 4.2 ci-dessous.

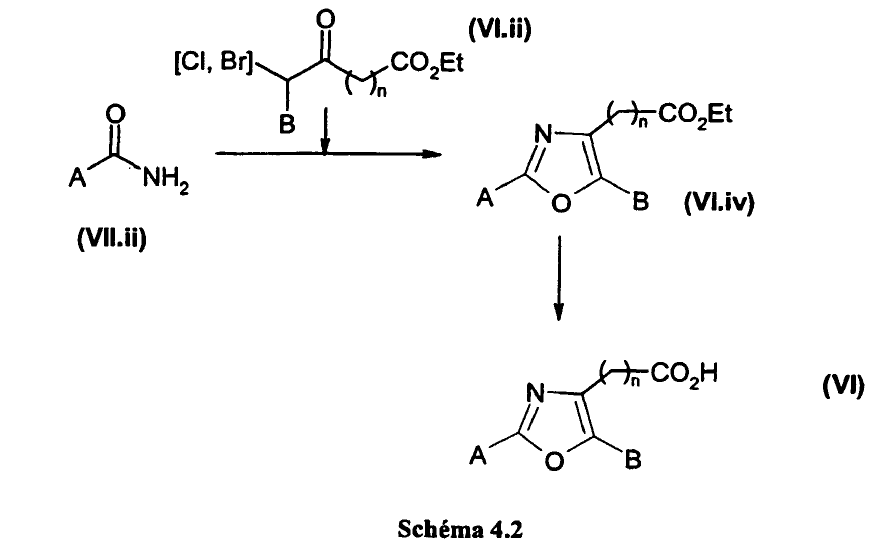

Les carboxamides de formule générale **(VII.ii)** sont mis à réagir avec le composé de formule générale **(VI.ii)** en chauffant, par exemple à reflux, en l'absence ou en la présence d'un solvant comme le diméthylformamide. L'ester de formule générale **(VI.iv)** obtenu peut ensuite être saponifié par action d'une base comme par exemple la potasse en milieu alcoolique ou LiOH dans du tétrahydrofuranne pour donner l'acide de formule générale **(VI).**

### Préparation des acides dérivés d'isoxazolines de formule générale (VI).

Les acides dérivés d'isoxazolines de formule générale **(VI),** utiles à la préparation de composés de formule générale **(I)₄,** peuvent être préparés selon une procédure représentée dans le schéma 4.3 ci-après.

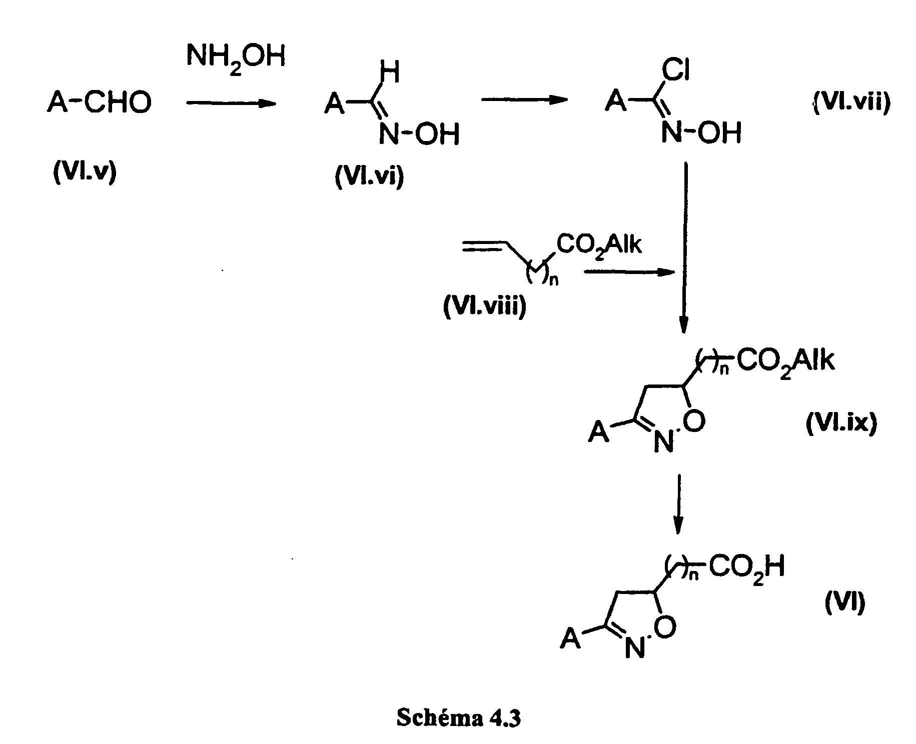

Les acides de formule générale **(VI)** dérivés d'isoxazolines peuvent être préparés comme suit : les aldéhydes commerciaux de formule générale **(VI.v)** sont mis à réagir avec le chlorhydrate d'hydroxylamine. L'oxime de formule générale (VI.vi) ainsi obtenu est activé sous forme de chlorure d'oxime, de formule générale **(VI.vii),** par réaction avec le N-chlorosuccinimide dans le DMF avant de réagir avec les esters de formule générale **(VI.viii)** (dans laquelle Alk représente un radical alkyle) pour conduire aux dérivés isoxazolines selon un protocole expérimental décrit dans la littérature (Tetrahedron Lett., 1996, 37 (26), 4455 ; J. Med. Chem., 1997, 40, 50-60 et 2064-2084). La saponification des isoxazolines de formule générale **(VI.ix)** est ensuite effectuée classiquement (par exemple par action de KOH dans un solvant alcoolique ou LiOH dans un solvant comme le tétrahydrofuranne) pour donner le dérivé acide de formule générale **(VI).**

Les esters insaturés non commerciaux de formule générale **(VI.x)** peuvent être préparés selon des méthodes décrites dans la littérature (J. Med. Chem., 1987, 30, 193 ; J. Org. Chem., 1980, 45, 5017).

### Préparation des thiazoles et oxazoles de formule générale (VII)

### Schéma général

Les acides de formule générale **(VII.i)**, schéma 5.1, sont convertis en les carboxamides correspondants de formule générale **(VII.ii)** par des méthodes décrites dans la littérature (cf. par exemple, J. Chem. Soc., Perkin Trans. 1, (1998), 20, 3479-3484 ou la demande de brevet PCT WO 99/09829). Les composés de formule générale **(VII)** peuvent ensuite obtenus classiquement selon les procédures représentées dans les schémas 5.2 et 5.3 (thiazoles) et le schéma 5.4 (oxazoles) ci-après.

Cette voie de synthèse est utile pour préparer ensuite des composés répondant aux sous-formules générales **(I)₁** et **(I)₃.**

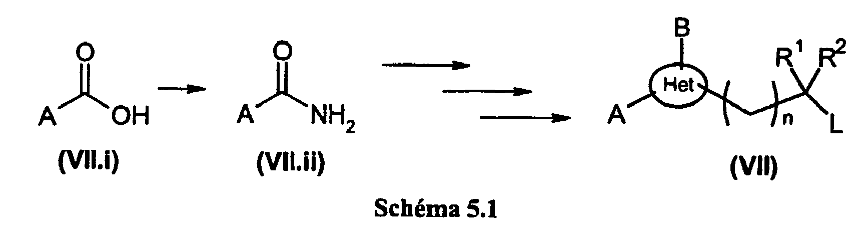

### Obtention des thiazoles de formule générale (VII)

Lorsque R¹ et R² représentent tous deux H, les thiazoles de formule générale **(VII)** destinés à la préparation de composés de formule générale **(I)₃** peuvent être préparés selon la méthode résumée dans le schéma 5.2. Le carboxamide de formule générale **(VII.ii)** est converti en le thiocarboxamide correspondant de formule générale **(VII.iii)** en présence de réactif de Lawesson dans un solvant comme le dioxane ou le benzène à une température de préférence comprise entre la température ambiante et celle du reflux du mélange. Le thiocarboxamide de formule générale **(VII.iii)** est ensuite traité avec l'α-halogénocétoester de formule générale **(VII.iv)** dans laquelle Alk représente un radical alkyle (par exemple méthyle, éthyle ou *tert-*butyle), pour donner l'ester de formule générale **(VII.v),** lequel est réduit en l'alcool correspondant de formule générale **(VII.vi),** par exemple par action d'hydrure de lithium aluminium ou d'hydrure de diisobutylaluminium dans un solvant comme le tétrahydrofuranne. Ce dernier peut alors être converti en un dérivé halogéné de formule générale **(VII)** selon des méthodes connues de l'homme du métier, par exemple, dans le cas d'un dérivé bromé (L = Br), par réaction avec CBr₄ en présence de triphénylphosphine dans du dichlorométhane à température ambiante.

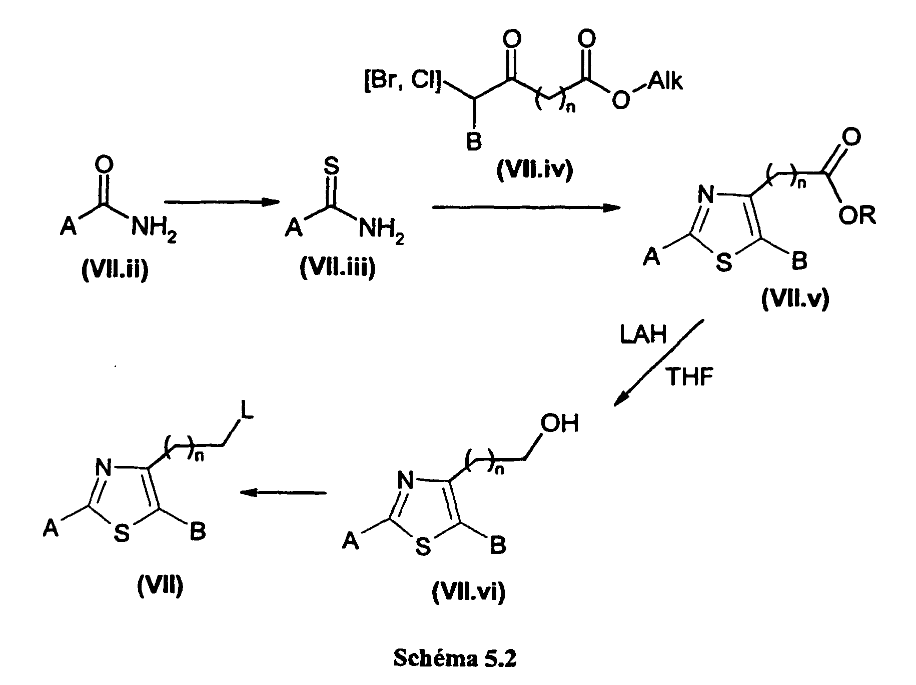

Les thiazoles de formule générale **(VII)** destinés à la préparation de composés de formule générale **(I)₁** peuvent être préparés selon la méthode résumée dans le schéma 5.3. Le dérivé cyano de formule générale **(VII.vii)** dans laquelle Gp' est un groupe protecteur pour une fonction alcool (par exemple un groupe benzyle ou -CO-p dans lequel p représente alkyle, par exemple méthyle ou *tert*-butyle) est converti en le thiocarboxamide correspondant de formule générale **(VII.viii)** par action de H₂S dans un solvant comme l'éthanol en présence de triéthanolamine à une température de préférence comprise entre la température ambiante et celle du reflux du mélange . Le thiocarboxamide de formule générale **(VII.viii)** est ensuite traité avec l'α-halogénocétone de formule générale **(VII.ix)** pour donner le composé de formule générale **(VII.x),** lequel est déprotégé pour donner l'alcool correspondant de formule générale **(VII.xi)** selon des méthodes connues de l'homme du métier (par exemple lorsque Gp' est un groupe protecteur de type acétate, celui-ci est retiré *in situ* par action d'une solution de carbonate de sodium aqueuse). Ce dernier peut alors être converti en un dérivé halogéné de formule générale **(VII)** selon des méthodes connues de l'homme du métier, par exemple, dans le cas d'un dérivé bromé (L = Br), par réaction avec CBr₄ en présence de triphénylphosphine dans du dichlorométhane à température ambiante.

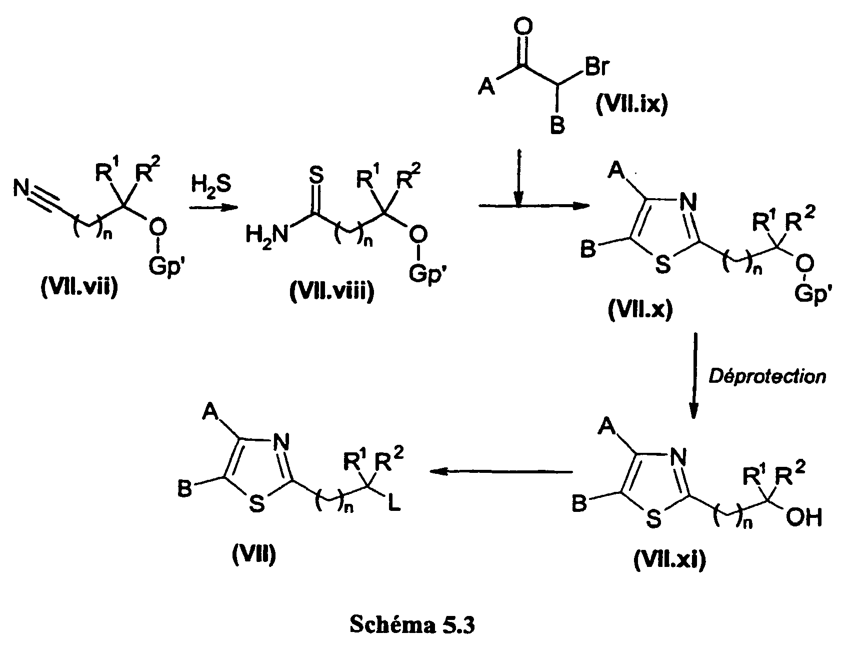

### Obtention des oxazoles de formule générale (VII)

Lorsque R¹ et R² représentent tous deux H, les oxazoles de formule générale **(VII)** destinés à la préparation de composés de formule générale **(I)₃** peuvent être préparés selon la méthode résumée dans le schéma 5.4. Le carboxamide de formule générale **(VII.ii)** est traité avec l'α-halogénocétoester de formule générale **(VII.iv)** dans laquelle Alk représente un radical alkyle (par exemple méthyle, éthyle ou *tert*-butyle), pour donner l'ester /l'acide de formule générale **(VII.xii).** Ce dernier est réduit en l'alcool correspondant de formule générale **(VII.xiii)**, par exemple par action d'hydrure de lithium et d'aluminium ou d'hydrure de diisobutylaluminium dans un solvant comme le tétrahydrofuranne lorsque l'on part de l'ester ou par action de diborane dans le tétrahydrofuranne lorsque l'on part de l'acide. Ce dernier peut alors être converti en un dérivé halogéné de formule générale **(VII)** selon des méthodes connues de l'homme du métier, par exemple, dans le cas d'un dérivé bromé (L = Br), par réaction avec CBr₄ en présence de triphénylphosphine dans du dichlorométhane à température ambiante.

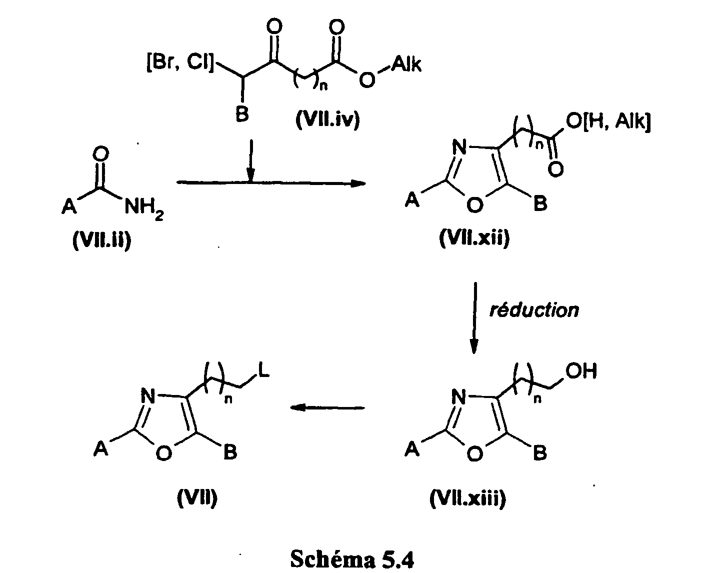

### Préparation des acides de formule générale (VII.i)

Les acides de formule générale **(VII.i)** non commerciaux sont accessibles à partir de méthodes de la littérature. En particulier :
- lorsque A représente un radical phénothiazinyle, les acides de formule générale **(VII.i)** sont accessibles à partir de méthodes de la littérature comme par exemple J. Med. Chem. (1992), 35, 716-724, J. Med. Chem. (1998), 41, 148 -156 ; Synthesis (1988) 215-217 ; ou J. Chem. Soc. Perkin. Trans. 1 (1998), 351-354 ;
- lorsque A représente un radical indolinyle, les acides de formule générale **(VII.i)** sont accessibles à partir de méthodes de la littérature comme par exemple J. Het. Chem. (1993), 30,1133-1136 ou Tetrahedron (1967), 23, 3823 ;
- lorsque A représente un radical phénylaminophényle, les acides de formule générale **(VII.i)** sont accessibles à partir de méthodes de la littérature comme par exemple J. Amer. Chem Soc. (1940), 62, 3208 ; Zh. Obshch. Khim. (1953), 23, 121-122 ou J. Org. Chem. (1974), 1239-1243 ;
- lorsque A représente un radical carbazolyle, les acides de formule générale **(VII.i)** sont accessibles à partir de methodes de la littérature comme par exemple J. Amer. Chem Soc., (1941), 63, 1553-1555 ; J. Chem. Soc. (1934), 1142-1144 ; J. Chem. Soc. (1945), 945-956 ; ou Can. J. Chem. Soc. (1982), 945-956 ; et
- lorsque A représente un radical du type 4-(4-hydroxyphényl)-phényle, on se référera par exemple à la publication suivante : Synthesis, (1993) 788-790.

### Préparation des composés de formule générale (VIII)

Lorsque R¹ et R² représentent tous deux H, les aminoacides protégés de formule générale **(VIII)** sont soit commerciaux, soit obtenus par protection d'aminoacides commerciaux par un groupe de type carbamate selon des méthodes connues de l'homme du métier.

Lorsque au moins l'un de R¹ et R² n'est pas H, et n = 0, les aminoacides protégés de formule générale **(VIII)** sont obtenus en une étape, schéma 6.1, par alkylation, dans un solvant comme le tétrahydrofuranne et à basse température, du composé commercial de formule générale **(VIII.i)** à l'aide de 3 équivalents de butyllithium et d'environ un équivalent de dérivé halogéné de formule générale **(VIII.ii)** dans laquelle R¹ représente un radical de type alkyle, cycloalkyle, cycloalkylalkyle ou arylalkyle et Hal un atome halogène. Selon le cas, une deuxième alkylation (non représentée dans le schéma 6.1) peut être effectuée de façon similaire, permettant ainsi d'obtenir les composés de formule générale **(VIII)** dans lesquels ni R¹ ni R² ne représente H.

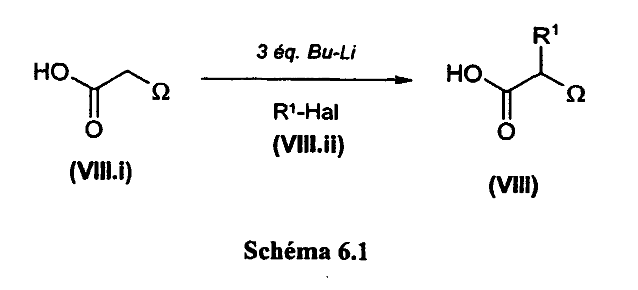

### Préparation des imidazoles, thiazoles et oxazoles de formule générale (IX)

La préparation des intermédiaires de formule générale **(IX)** est décrite dans la demande de brevet WO 98/58934 (cf. en particulier pages 10 à 50 et les exemples de ce document) ou effectuée par analogie à partir de produits de départ commerciaux.

### Préparation des alcools protégés de formule générale (X)

### Préparation des composés de formule générale (X) dérivés d'imidazoles

L'acide de formule générale **(X.i)** est successivement traité, schéma 8.1, par Cs₂CO₃, le composé de formule générale **(V.ii)** et par NH₄OAc, pour donner le composé de formule générale **(X)**. Les conditions réactionnelles sont analogues à celles décrites plus haut pour ce type de synthèse.

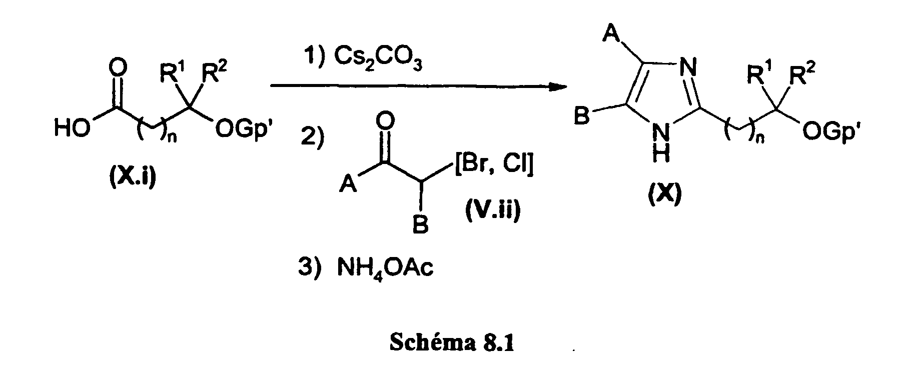

### Préparation des composés de formule générale (X) dérivés de thiazoles

Le dérivé cyano de formule générale **(X.ii)** est traité, schéma 8.2, par H₂S pour donner le thiocarboxamide de formule générale **(X.iii),** lequel, condensé sur le composé de formule générale **(V.ii),** permet d'obtenir le composé de formule générale **(X).** Les conditions réactionnelles sont analogues à celles décrites plus haut (schéma 5.3) pour ce type de synthèse.

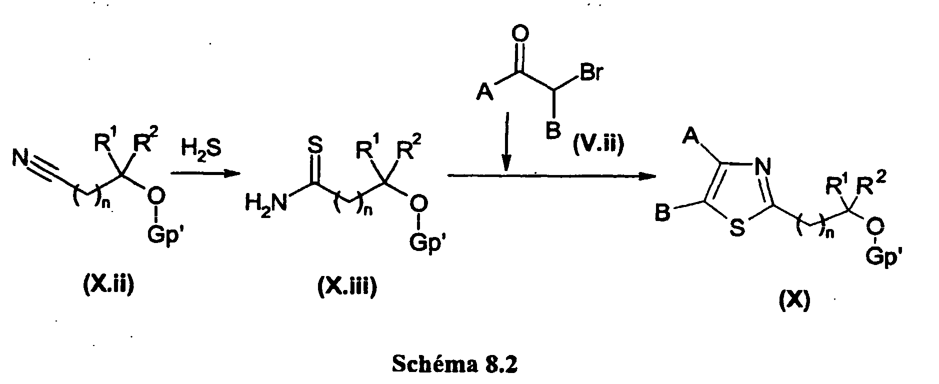

### Préparation des acides de formule générale (XXXVI)

Les acides de formule générale **(XXXVI)** non commerciaux sont accessibles à partir de méthodes de la littérature ou de méthodes similaires que l'homme du métier aura adaptées. En particulier :
◆ lorsque A représente un radical phénothiazinyle, les acides de formule générale **(XXXVI)** sont accessibles à partir de méthodes de la littérature : J. Org. Chem., (1956), 21, 1006 ; Chem. Abstr., 89, 180029 et Arzneimittel Forschung (1969), 19, 1193.
◆ lorsque A représente un radical diphénylamine, les acides de formule générale **(XXXVI)** sont accessibles à partir de méthodes de la littérature : Chem Ber., (1986), 119, 3165-3197*;* J. Heterocyclic. Chem. (1982), 15, 1557-1559 ; Chem. Abstr., (1968), 68, 68730x ; ou par adaptation de ces méthodes par l'homme du métier ;
◆ lorsque A représente un radical du type 4-(4-hydroxyphényl)-phényle, les acides de formule générale **(XXXVI)** sont accessibles à partir de méthodes de la littérature comme par exemple Tetrahedron Lett. (1968), 4739 ou J. Chem. Soc. (1961), 2898.
◆ lorsque A représente un radical carbazolyle, les acides de formule générale **(XXXVI)** sont accessibles à partir de méthodes de la littérature comme par exemple J. Amer. Chem., (1946), 68, 2104 ou J. Het. Chem (1975), 12, 547-549.
◆ lorsque A représente un radical de type benzopyrane ou benzofuranne, les acides de formule générale **(XXXVI)** sont accessibles par des méthodes de la littérature comme par exemple Syn. Commun. (1982), 12(8), 57-66 ; J. Med. Chem. (1995), 38(15), 2880-2886 ; ou Helv. Chim. Acta. (1978), 61, 837-843.
◆ lorsque A représente un radical indolinyle ou tétrahydroquinolyle, les acides de formule générale (XXXVI) sont accessibles à partir de méthodes de la littérature comme, par exemple, J. Med. Chem. (1997), 40, (7), 1049-1062 ; Bioorg. Med. Chem. Lett. (1997), 1519-1524 ; Chem. Abstr. (1968), 69, 43814k ; ou Chem. Abstr. (1966), 66, 17538c.

Bien entendu, les fonctions phénol, amine ou aniline résultant de la nature des substituants sur le radical A des composés de formule générale **(XXXVI)** peuvent conduire l'homme du métier à ajouter aux étapes décrites des étapes de protection / déprotection de ces fonctions afin qu'elles n'interfèrent pas avec le reste de la synthèse chimique.

A moins qu'ils ne soient définis d'une autre manière, tous les termes techniques et scientifiques utilisés ici ont la même signification que celle couramment comprise par un spécialiste ordinaire du domaine auquel appartient cette invention.

Les exemples suivants sont présentés pour illustrer les procédures ci-dessus Les composés exemplifiés qui ne tombent pas sous la formule générale de la revendication 1 ne font pas partie de la présente invention.

### EXEMPLES

### Exemple 1 : 4-[3,5-bis(1,1-diméthyléthyl)-4-hydroxyphényl]-N-méthyl-2-thiazoleméthanamine :

Ce produit est obtenu selon la procédure décrite dans la demande de brevet PCT WO 98/58934. Alternativement, il peut aussi être préparé selon la méthode décrite ci-après.

### 1.1) N-Boc-sarcosinamide :

15,0 g (0,120 mol) de chlorhydrate de sarcosinamide (N-Me-Gly-NH₂.HCl) sont mis en solution dans du dichlorométhane contenant 46,2 ml (0,265 mol) de diisopropyléthylamine. Le mélange est refroidi à 0 °C puis on ajoute par fractions Boc-O-Boc (28,8 g ; 0,132 mol) et on laisse agiter le mélange une nuit à température ambiante. Le milieu réactionnel est alors versé sur de l'eau glacée et extrait au dichlorométhane. La phase organique est lavée successivement avec une solution aqueuse de bicarbonate de sodium à 10% et à l'eau, puis enfin avec une solution de chlorure de sodium saturée. La phase organique est ensuite séchée sur sulfate de magnésium, filtrée et concentrée sous vide. Le produit obtenu est purifié par cristallisation dans de l'éther diisopropylique pour conduire à un solide blanc avec un rendement de 72%. Point de fusion : 103 °C.

### 1.2) 2-{[(1,1-diméthylétoxyy)carbonyl]méthyl}amino-éthanethioamide :

16,0 g (0,085 mol) de l'intermédiaire 1.1 sont dissous dans du diméthoxyéthane (500 ml) et la solution obtenue est refroidie à 5 °C. Du bicarbonate de sodium (28,5 g ; 0,34 mol) puis, par petites portions, (P₂S₅)₂ (38,76 g ; 0,17 mol) sont ajoutés. Le milieu réactionnel est laissé revenir à température ambiante tout en étant agité durant 24 heures. Après évaporation sous vide des solvants, on ajoute au résidu une solution aqueuse de bicarbonate de sodium à 10% et la solution est extraite à l'aide d'acétate d'éthyle. La phase organique est lavée successivement avec une solution aqueuse de bicarbonate de sodium à 10% et à l'eau, puis enfin avec une solution de chlorure de sodium saturée. La phase organique est ensuite séchée sur sulfate de magnésium, filtrée et concentrée sous vide. Le produit obtenu est purifié par cristallisation dans de l'éther pour conduire à un solide de couleur blanche avec un rendement de 65%. Point de fusion : 150-151 °C.

### 1.3) 4-[3,5-bis(1,1-diméthyléthyl)-4-hydroxyphényl]-N-[(1,1-diméthyléthoxy)-carbonyl]-N-méthyl-2-thiazoleméthanamine :

L'intermédiaire 1.2 (4,3 g; 2,11 mmol) et de la bromo-1-(3,5-di*tert-*butyl-4-hydroxyphényl)éthanone (6,9g ; 2,11 mmol) sont dissous dans du benzène (75 ml) sous atmosphère d'argon puis le mélange est agité à température ambiante durant 12 heures. Le milieu réactionnel est porté au reflux pendant 4 heures. Après évaporation des solvants, le résidu est dilué avec du dichlorométhane et lavé avec une solution saturée en NaCl. La phase organique est séparée, séchée sur sulfate de magnésium, filtrée et concentrée sous vide. Le produit attendu est obtenu après chromatographie sur une colonne de silice (éluant : 20% d'acétate d'éthyle dans de l'heptane) sous forme d'une huile qui cristallise très lentement au réfrigérateur avec un rendement de 28%.
Point de fusion : 126,5-127,3 °C.

### 1.4) 4-[3,5-bis(1,1-diméthyléthyl)-4-hydroxyphényl]-N-méthyl-2-thiazoleméthanamine :

A une solution de 2,5 g (5,8 mmol) de l'intermédiaire 1.3 et de 2 ml (1,6 mmol) de triéthylsilane dans 50 ml de dichlorométhane, on ajoute goutte à goutte, à 0 °C, 2,3 ml (29 mmol) d'acide trifluoroacétique. Après une heure d'agitation, le mélange réactionnel est concentré sous vide et le résidu est dilué dans 100 ml d'acétate d'éthyle et 50 ml d'une solution saturée de NaHCO₃. Après agitation et décantation, la phase organique est séchée sur sulfate de magnésium, filtrée et concentrée sous vide. Le résidu est repris dans de l'heptane pour donner, après séchage, un solide blanc avec un rendement de 73%. Point de fusion : 136 °C.

### 1.5 Chlorhydrate de 4-[3,5-bis(1,1-diméthyléthyl)-4-hydroxyphényl]-N-méthyl-2-thiazoleméthanamine:

2,0 g (0,602 mmol) de l'intermédiaire 1.4 sont dissous dans de l'éther anhydre. La solution est refroidie à 0 °C puis on ajoute goutte à goutte 18 ml (1,81 mmol) d'une solution de HCl 1N dans l'éther. On laisse le mélange revenir à température ambiante tout en le maintenant agité. Après filtration et séchage sous vide, un solide blanc est récupéré avec un rendement de 92%. Point de fusion : 185,3-186,0 °C.

### Exemple 2 : 2,6-di(tert-butyl)-4-(2-{[méthyl(2-propynyl)amino]méthyl)-1,3-thiazol-4-yl)phénol :

A une solution de 0,5 g (1,5 mmol) du composé de l'exemple 1 dans 15 ml d'acétonitrile, on ajoute goutte-à-goutte, à 0 °C, 0,52 ml (3,7 mmol) de triéthylamine et un excès de 0,56 g (7,5 mmol) de chloropropargyle. Après une nuit d'agitation, le mélange réactionnel est concentré sous vide et le résidu est dilué avec du dichlorométhane et 50 ml d'une solution saturée de NaCl. Après agitation et décantation, la phase organique est séparée et séchée sur sulfate de magnésium, filtrée et concentrée sous vide. Le produit attendu est obtenu après chromatographie sur une colonne de silice (éluant : 20% acétate d'éthyle dans de l'heptane). Après évaporation, les fractions pures donnent un solide blanc avec un rendement de 20%. Point de fusion : 210-215 °C.
MH+ = 371,20.

### Exemple 3 : 2-[({4-[3,5-di(tert-butyl)-4-hydroxyphényl]-1,3-thiazol-2-yl}méthyl)(méthyl)amino]acétonitrile :

Le protocole expérimental utilisé est identique à celui décrit pour l'exemple 2, le chloroacétonitrile étant utilisé comme produit de départ à la place du chloropropargyle.
On obtient un solide beige avec un rendement de 54%. Point de fusion : 150-156 °C.
MH+ = 372,30

### Exemple 4 : 5-[({4-[3,5-di(tert-butyl)-4-hydroxyphényl]-1,3-thiazol-2-yl}méthyl)(méthyl)amino]pentanenitrile :

Le protocole expérimental utilisé est identique à celui décrit pour l'exemple 2, le bromovaléronitrile étant utilisé comme produit de départ à la place du chloropropargyle.
On obtient une huile jaune avec un rendement de 24%.
MH+ = 414,30

### Exemple 5 : 6-[({4-[3,5-di(tert-butyl)-4-hydroxyphényl]-1,3-thiazol-2-yl}méthyl)(méthyl)amino]hexanenitrile :

Le protocole expérimental utilisé est identique à celui décrit pour l'exemple 2, le bromohexanenitrile étant utilisé comme produit de départ à la place du chloropropargyle. On obtient une huile rouge avec un rendement de 35%.
MH+ = 428,40.

### Exemple 6 : 2,6-di(tert-butyl)-4-(2-{[(2-hydroxyéthyl)(méthyl)amino]méthyl}-1,3-thiazol-4-yl)phénol :

Le protocole expérimental utilisé est identique à celui décrit pour l'exemple 2, le 2-bromoéthanol est utilisé comme produit de départ à la place du chloropropargyle.
On obtient une huile jaune avec un rendement de 57%.
MH+ = 377,30

### Exemple 7 : 4-(2-{[benzyl(méthyl)amino]méthyl}-1,3-thiazol-4-yl)-2,6-di(tert-butyl)phénol :

Le protocole expérimental utilisé est identique à celui décrit pour l'exemple 2, le chlorure de benzyle étant utilisé comme produit de départ à la place du chloropropargyle. On obtient un solide blanc avec un rendement de 52%. Point de fusion: 165-170 °C.
MH+ = 423,30

### Exemple 8 : 2,6-di(tert-butyl)-4-{2-[(méthyl-4-nitroanilino)méthyl]-1,3-thiazol-4-yl}phénol :

Ce produit est obtenu selon la procédure décrite dans la demande de brevet PCT WO 98/58934.

### Exemple 9 : 2,6-di(tert-butyl)-4-(2-{[4-(diméthylamino)(méthyl)anilino]méthyl}-1,3-thiazol-4-yl)phénol :

A une solution de 0,5 g (1,1 mmol) de l'exemple 8 dans 20 ml d'éthanol on ajoute 0,8 ml de paraformaldéhyde et 0,10 g de palladium 10% sur charbon. L'ensemble est placé sous hydrogène pendant 4 heures. Le catalyseur est filtré et le solvant évaporé à sec. Le produit attendu est obtenu après chromatographie sur une colonne de silice (éluant : 3% d'éthanol dans du dichlorométhane). Le composé attendu est obtenu sous forme d'une huile marron avec un rendement de 54%.
MH+ = 452,30

### Exemple 10 : {4-[3,5-di(tert-butyl)-4-hydroxyphenyl]-1,3-thiazol-2-yl}méthylcarbamate de benzyle :

Le composé est fabriqué selon un protocole expérimental décrit dans la demande de brevet WO 98/58934 (voir préparation des intermédiaires 26.1 et 26.2), en utilisant Z-Gly-NH₂ à la place du N-Boc sarcosinamide. Le composé attendu est obtenu sous forme d'une huile jaune pâle avec un rendement de 99%.
MH+ = 453,20

### Exemple 11 : 4-[2-(aminométhyl)-1,3-thiazol-4-yl]-2,6-di(tert-butyl)phénol :

A une solution de 0,106 g (1,1 mmol) du composé de l'exemple 10 dans 10 ml de méthanol on ajoute goutte à goutte 0,1 ml d'une solution d'hydroxyde de potassium à 40%. Après une nuit d'agitation à reflux, le mélange réactionnel est concentré sous vide et le résidu est dilué avec du dichlorométhane et lavé avec une solution HCl 1N puis 50 ml d'une solution saturée de NaCl. La phase organique est séparée et séchée sur sulfate de magnésium, filtrée et concentrée sous vide. Le produit attendu est obtenu après chromatographie sur une colonne de silice (éluant : 5% d'éthanol dans du dichlorométhane) sous forme d'une mousse marron avec un rendement de 76%.
MH+ = 319,29.

### Exemple 12 : 2,6-di(tert-butyl)-4-(2-{[méthyl(4-nitrobenzyl)amino]méthyl}-1,3-thiazol-4-yl)phénol :

Le protocole expérimental utilisé est identique à celui décrit pour l'exemple 2, le bromure de 4-nitro-benzyle étant utilisé comme produit de départ à la place du chloropropargyle. On obtient un solide jaune avec un rendement de 63%. Point de fusion: 114,4-111,7 °C.
MH+ = 468,3

### Exemple 13 : 4-(2-{[(4-aminobenzyl)(méthyl)amino]méthyl}-1,3-thiazol-4-yl)-2,6-di(tert-butyl)phénol :

A une solution de 0,05 g (0,107 mmol) du composé de l'exemple 12 dans un mélange de 0,55 ml d'acide acétique glacial et 0,07 ml d'une solution HCl 12N, on ajoute successivement 0,059 g (0,26 mmol) de SnCl₂,2H₂O et 0,017 g (0,26 mmol) de Zn.
L'ensemble est agité 18 heures à 20 °C. Le mélange réactionnel est ensuite rendu basique par addition d'une solution aqueuse de NaOH à 30%. Le produit est alors extrait à l'aide de 2 fois 50 ml de CH₂Cl₂. La solution organique est lavée avec 50 ml de saumure, séchée sur MgSO₄, filtrée et concentrée sous vide. Le résidu est purifié sur une colonne de silice (éluant : 5% d'éthanol dans du dichlorométhane). On obtient une gomme jaune avec un rendement de 52%.
MH+ = 438,29.

### Exemple 14 : 2,6-di(tert-butyl)-4-(2-{[(4-nitrobenzyl)amino]méthyl}-1,3-thiazol-4-yl)phénol :

A un ballon contenant 30 ml de MeOH anhydre, sous atmosphère inerte, on ajoute successivement 0,5 g (1,57 mmol) du composé de l'exemple 9, 0,237 g (1,57 mmol) de 4-nitrobenzaldéhyde et 1 g de tamis moléculaire 4 Å pulvérulent préalablement activé.
Le mélange réactionnel est agité vigoureusement pendant 18 heures avant l'addition, par portions, de 0.06 g (1.57 mmol) de NaBH₄. L'agitation est maintenue 4 heures supplémentaires avant addition de 5 ml d'eau. Après un quart d'heure, le tamis est filtré et le mélange réactionnel est extrait par 2 fois 100 ml de CH₂Cl₂. La phase organique est lavée successivement avec 50 ml d'eau puis 50 ml de saumure, séchée sur sulfate de sodium, filtrée et concentrée sous vide. Le résidu est purifié sur une colonne de silice (éluant : 50% d'acétate d'éthyle dans de l'heptane). On obtient une huile jaune avec un rendement de 55%.
MH+ = 454,20.

### Exemple 15 : 4-(2-{[(4-aminobenzyl)amino]méthyl}-1,3-thiazol-4-yl)-2,6-di(tert-butyl)phénol :

Le protocole expérimental utilisé est identique à celui décrit pour l'exemple 13, le composé de l'exemple 14 étant utilisé comme produit de départ à la place du composé de l'exemple 12. On obtient une gomme jaune avec un rendement de 83%.
MH+ = 424,20.
*Les composés des exemples 16 à 22 peuvent être obtenus selon des procédures décrites dans la demande de brevet* PCT WO 98/58934*.*

### Exemple 16 : 4-(3,5-bis(1,1-diméthyléthyl)-4-hydroxyphényl)-N-méthyl-N-(4-aminophényl)-2-thiazoleméthanamine :

[il s'agit de l'intermédiaire 26.5 de la demande PCT WO 98/58934]

### Exemple 17 : 4-[3,5-bis(1,1-diméthyléthyl)-4-hydroxyphényl]-N-méthyl-1H-imidazole-2-méthanamine :

L'intermédiaire 26.2 de la demande PCT WO 98/58934 est soumis à une hydrogénation telle que décrite dans l'étape 1.2 du même document en utilisant l'éthanol comme solvant de réaction au lieu du méthanol. On isole le produit attendu sous forme d'une mousse rouge.
MH+ = 316,33.

### Exemple 18 : 4-[3,5-bis(1,1-diméthyléthyl)-4-hydroxyphényl]-N-méthyl-N-(4-nitrophényl)-1H-imidazole-2-méthanamine :

[il s'agit de l'intermédiaire 27.2 de la demande PCT WO 98/58934]

### Exemple 19: 4-[3,5-bis(1,1-diméthyléthyl)-4-hydroxyphényl]-N-méthyl-N-(4-aminophényl)-1H-imidazole-2-méthanamine :

[il s'agit de l'intermédiaire 27.3 de la demande PCT WO 98/58934]

### Exemple 20 : 4-[3,5-bis-(1,1-diméthyléthyl)-4-hydroxyphényl]-N-méthyl-N-(4-nitrobenzoyl)-1H-imidazole-2-méthanamine :

[il s'agit de l'intermédiaire 22.6 de la demande PCT WO 98/58934]

### Exemple 21 : 4-[3,5-bis-(1,1-diméthyléthyl)4-hydroxyphényl]-N-méthyl-N-(4-aminobenzoyl)-1H-imidazole-2-méthanamine :

[il s'agit de l'intermédiaire 22.7 de la demande PCT WO 98/58934]

### Exemple 22: 3-[3,5-bis(1,1-diméthyléthyl)-4-hydroxyphényl]-4,5-dihydro-5-isoxazoleéthanol :

[il s'agit de l'intermédiaire 28.1 de la demande PCT WO 98/58934]

*Le composé de l'exemple 23 peut être obtenu selon des procédures décrites dans la demande de brevet* PCT WO 99/09829*.*

### Exemple 23 : 2-[3,5-bis(1,1-diméthyléthyl)-4-hydroxyphényl]-4-oxazoleéthanol :

[il s'agit de l'intermédiaire 1.C de la demande PCT WO 99/09829 ; alternativement, ce composé peut aussi être obtenu selon la procédure décrite dans J. Med. Chem. (1996), 39, 237-245.]

### Exemple 24 : 4-[{[4-(3,5-ditert-butyl-4-hydroxyphényl)-1,3-thiazol-2-yl]méthyl}(méthyl)amino]butanenitrile :

Le protocole expérimental utilisé est identique à celui décrit pour l'exemple 2, le bromobutyronitrile étant utilisé comme produit de départ à la place du chloropropargyle. On obtient une huile jaune avec un rendement de 18%.
MH⁺ = 400,30.

### Exemple 25 : 2,6-ditert-butyl-4-(2-{[(3-nitrobenzyl)amino]méthyl}-1,3-thiazol-4-yl)phénol :

Le protocole expérimental utilisé est identique à celui décrit pour l'exemple 14, le 3-nitrobenzaldéhyde étant utilisé comme produit de départ à la place du
4-nitrobenzaldéhyde. On obtient une huile jaune avec un rendement de 28%.
MH+ = 454,20.

### Exemple 26 : 2,6-ditert-butyl-4-(4-{2-[méthyl(2-propynyl)amino]éthyl}-1,3-oxazol-2-yl)phénol :

Le composé de l'exemple 23 est transformé en dérivé bromé, intermédiaire 3, selon la procédure indiquée dans le schéma 1(c) de la demande PCT WO 99/09829. Ensuite le dérivé bromé (0,5 g ;1,31 mmol) est ajouté à une solution de N-méthylpropargylamine 0,34 ml (3,94 mmol) et de carbonate de potassium (1,11 g) dans du diméthylformamide (20 ml). Après une nuit d'agitation à 80 °C, le mélange réactionnel est concentré sous vide et le résidu est dilué avec du dichlorométhane et 50 ml d'une solution saturée en NaCl. Après agitation et décantation, la phase organique est séparée et séchée sur sulfate de magnésium, filtrée et concentrée sous vide. Le produit attendu est obtenu après chromatographie sur une colonne de silice (éluant : 50% acétate d'éthyle dans de l'heptane). Après évaporation, les fractions pures donnent une huile jaune avec un rendement de 24%.
MH+ = 369,30.

### Exemple 27 : [{2-[2-(3,5-ditert-butyl-4-hydroxyphényl)-1,3-oxazol-4-yl]éthyll(méthyl)amino]acétonitrile :

Le protocole expérimental utilisé est identique à celui décrit pour le composé de l'exemple 26, le méthylaminoacétonitrile étant utilisé comme produit de départ à la place de la N-méthylpropargylamine. On obtient un solide blanc avec un rendement de 36%. Point de fusion : 165-167,8 °C.

### Exemple 28 : 3-[{2-[2-(3,5-ditert-butyl-4-hydroxyphényl)-1,3-oxazol-4-yl]éthyl}(méthyl)amino]propanenitrile :

Le protocole expérimental utilisé est identique à celui décrit pour l'exemple 26, le N-méthyl-β-alaninenitrile étant utilisé comme produit de départ à la place de la N-méthylpropargylamine. On obtient un solide blanc avec un rendement de 56%. Point de fusion: 104-104,8 °C.

### Exemple 29 : chlorhydrate de 2,6-ditert-butyl-4-{4-[2-(1-pipérazinyl)éthyl]-1,3-oxazol-2-yl}phénol :

### 29.1) tert-butyl 4-{2-[2-(3,5-ditert-butyl-4-hydroxyphényl)-1,3-oxazol-4-yl]éthyl}-1-pipérazinecarboxylate

Le protocole expérimental utilisé est identique à celui décrit pour l'exemple 26, le pipérazinecarboxylate de *tert-*butyle étant utilisé comme produit de départ à la place de la N-méthylpropargylamine. On obtient une huile brune avec un rendement de 72%. MH+ = 486,20.

### 29.2) Chlorhydrate de 2,6-ditert-butyl-4-{4-[2-(1-pipérazinyl)éthyl]-1,3-oxazol-2-yl}phénol

Dans une solution à 0 °C de l'intermédiaire 29.1 (0,450 g ; 9,27 mmol) dans de l'acétate d'éthyle (30 ml), on passe un courant de HCl gaz bulle à bulle. On laisse le mélange revenir à température ambiante durant la nuit. On fait passer un courant d'argon à travers la masse réactionnelle, puis la poudre obtenue est filtrée et lavée avec de l'acétate d'éthyle puis de l'éther pour conduire à un solide blanc avec un rendement de 70%. Point de fusion : > 200 °C.

### Exemple 30 : chlorhydrate de N-méthyl[4-(10B-phénothiazin-2-yl)-1,3-thiazol-2-yl]méthanamine :

Le protocole expérimental utilisé est identique à celui décrit pour l'exemple 1, la 2-bromo-1-(10H-phénothiazin-2-yl)éthanone (J. Heterocyclic. Chem., (1978), 15, 175-176 et Arzneimittel Forschung, (1962), 12, 48), étant utilisée comme produit de départ à la place de la 2-bromo-1-(3,5-ditert-butyl-4-hydroxyphényl)éthanone. Le produit obtenu est purifié par recristallisation dans de l'acide acétique glacial pour conduire à un solide verdâtre. Point de fusion : > 275 °C.

Alternativement, ce composé peut être obtenu selon une méthode analogue, mais en utilisant la 2-chloro-1-(10H-phénothiazin-2-yl)éthanone au lieu de la 2-bromo-1-(10H-phénothiazin-2-yl)éthanone :

### 30.1) 2-chloro-1-(10H-phénothiazin-2-yl)éthanone

De la 2-bromo-1-[10-(chloroacétyl)-10*H*-phénothiazin-2-yl)éthanone (2,2 g ; 5,55 mmol ; préparée d'après un protocole décrit dans J. Heterocyclic. Chem. (1978), 15, 175, suivi par une réaction de Friedel-Crafts) est dissoute à chaud dans un mélange d'acide acétique (20 ml) et de HCl à 20% (5,5 ml) et le mélange obtenu est porté à reflux pendant 30 minutes. Après refroidissement, filtration du précipité, rinçage avec de l'acide acétique (5 ml) et séchage sous vide, le solide obtenu est purifié par cristallisation dans du toluène pour conduire à un produit marron avec un rendement de 82% Point de fusion : 190-191 °C (valeur dans la littérature : 197-198 °C).

### 30.2) chlorhydrate de N-méthyl[4-(10H-phénothiazin-2-yl)-1,3-thiazol-2-yl]méthanamine

L'intermédiaire 30.1 (0,280 g ; 1,0 mmol) et le 2-amino-2-thioxoéthyl(méthyl)carbamate de *tert-*butyle (0,204 g ; 1,0 mmol ; décrit par exemple dans la demande de brevet PCT WO 98/58934) sont dissous dans du toluène et le mélange est porté à reflux pendant 18 heures. Après évaporation du toluène et refroidissement du mélange réactionnel à 0 °C, ce dernier est repris dans une solution 4N de HCl dans du dioxane (10 ml) et le mélange agité une heure à 0 °C avant de laisser la température revenir à température ambiante. Le solide formé est filtré et rincé avec de l'éther. Le produit attendu est obtenu après purification par cristallisation dans de l'acide acétique glacial à chaud pour conduire à un solide verdâtre. Point de fusion : >275 °C.

### Exemple 31 : 2-(4-[1,1'-biphényl]-4-yl-1H-imidazol-2-yl)éthylcarbamate de butyle

### 31.1) N-(butoxycarbonyl)-β-alanine

Une solution contenant de la β-alanine (8,9 g ; 0,1 mol) et 100 ml d'une solution d'hydroxyde de sodium 1N est refroidie à 10 °C. Du n-butyl chloroformate (13,66 g ; 0,1 mol) et 50 ml d'une solution d'hydroxyde de sodium 2 N sont ajoutés simultanément. Après 16 heures d'agitation à 23 °C, on ajoute environ 10 ml d'une solution d'acide chlorhydrique concentré (environ 11 N) pour ajuster le pH à 4-5. L'huile obtenue est extraite à l'acétate d'éthyle (2 x 50 ml), lavée avec de l'eau puis séchée sur sulfate de magnésium. Le produit cristallise dans l'isopentane sous forme d'une poudre blanche (rendement de 68%). Point de fusion : 50,5 °C.

### 31.2) 2-(4-[1,1'-biphényl]-4-yl-1H-imidazol-2-yl)éthylcarbamate de butyle

Un mélange de N-(butoxycarbonyl)-β-alanine (préparée à l'étape 31.1 ; 5,67 g ; 0,03 mol) et de carbonate de césium (4,89 g ; 0,015 mol) dans 100 ml d'éthanol est agité à 23°C pendant 1 heure. L'éthanol est éliminé par évaporation sous pression réduite dans un évaporateur rotatif. Le mélange obtenu est dissous dans 100 ml de diméthylformamide puis de la 4-phényl-bromoacétophénone (8,26 g ; 0,03 mol) est ajoutée. Après 16 heures d'agitation, le solvant est évaporé sous pression réduite. Le mélange obtenu est repris dans de l'acétate d'éthyle puis le bromure de césium est filtré. L'acétate d'éthyle du filtrat est évaporé et huile réactionnelle est reprise dans un mélange de xylène (100 ml) et d'acétate d'ammonium (46,2 g ; 0,6 mol). On chauffe à reflux environ une heure et demie puis, après refroidissement, un mélange d'eau glacée et d'acétate d'éthyle est versé dans le milieu réactionnel. Après décantation, la phase organique est lavée avec une solution saturée en bicarbonate de sodium, séchée sur sulfate de magnésium puis évaporée sous vide. Le solide obtenu est filtré puis lavé à l'éther pour donner une poudre de couleur beige clair (rendement de 50%). Point de fusion : 136,7 °C.
MH+ = 364,3.

### Exemple 32 : N-[2-(4-[1,1'-biphényl]-4-yl-1H-imidazol-2-yl)éthyl]pentanamide

### 32.1) 2-(4-[1,1'-biphényl]-4-yl-1H-imidazol-2-yl)éthylcarbamate de tert-butyle

Ce composé est obtenu selon un mode opératoire analogue à celui de l'étape 31.2 de l'exemple 31, la N-(tert-butoxycarbonyl)-β-alanine remplaçant la β-alanine. On obtient une poudre de couleur jaune avec un rendement de 37%.
MH+ = 364,2.

### 32.2) 2-(4-[1,1'-biphényl]-4-yl-1H-imidazol-2-yl)éthylamine

Du 2-(4-[1,1'-biphényl]-4-yl-1H-imidazol-2-yl)éthylcarbamate de *tert-*butyle (4,8 g ; 0,013 mol) est agité dans 120 ml d'une solution d'acétate d'éthyle saturée en acide chlorhydrique pendant 2 h 30 à une température de 55 °C. Le solide obtenu est filtré et lavé à l'éther. On obtient une poudre de couleur beige clair avec un rendement de 89%.
MH+ = 264,2.

### 32.3) N-[2-(4-[1,1'-biphényl]-4-yl-1H-imidazol-2-yl)éthyl]pentanamide

Un mélange contenant de l'acide valérique (0,24 ml; 0,002 mol), du dicyclohexylcarbodiimide (2,2 ml ; solution 1M dans du chlorure de méthylène) et de l'hydrate de 1-hydroxybenzotriazole (336 mg ; 0,0022 mol) dans 15 ml de diméthylformamide (DMF) est agité à 23°C pendant trente minutes. La 2-(4-[1,1'-biphényl]-4-yl-1H-imidazol-2-yl)éthylamine préparée précédemment est ajoutée puis on agite le mélange pendant 48 heures à 23 °C. La dicyclohexylurée formée est filtrée puis le DMF évaporé sous pression réduite. Le résidu obtenu est repris dans de l'acétate d'éthyle puis la dicyclohexylurée résiduelle est filtrée à nouveau. Le filtrat est lavé à l'eau et extrait à l'aide d'acétate d'éthyle. Le solvant est évaporé puis on procède à une purification sur colonne de silice (éluant : CH₂Cl₂-MeOH / 95-05). On obtient une poudre de couleur blanche avec un rendement de 13%. Point de fusion :
166-167 °C.
MH+ = 348,2.

### Exemple 33 : N-[2-(4-[1,1'-biphényl]-4-yl-1H-imidazo-2-yl)éthyl]-1-butanesulfonamide

Un mélange contenant de la 2-(4-[1,1'-biphényl]-4-yl-1H-imidazol-2-yl)éthylamine (obtenue à l'étape 32.2 de l'exemple 32 ; 660 mg ; 0,0025 mol) et du sulfochlorure de n-butane (390 mg ; 0,0025 mol) dans 20 ml de DMF est agité pendant deux heures à 23 °C. Du carbonate de potassium (345 mg ; 0,0025 mol.) est ensuite ajouté puis l'agitation poursuivie pendant deux heures. Le solvant est évaporé et le mélange réactionnel repris dans de l'eau et du dichlorométhane. La phase organique est lavée avec une solution saturée en chlorure de sodium puis séchée. Le solvant est évaporé et le résidu obtenu purifié sur colonne de silice (éluant : CH₂Cl₂-MeOH / 93-07). On obtient une poudre de couleur beige clair avec un rendement de 19%. Point de fusion : 168,5 °C.
MH+ = 384,2.

### Exemple 34: 4-[2-(2-{[butylamino)carbonyl]amino}éthyl)-1H-imidazol-4-yl]-1,1'-biphényle

Un mélange contenant de la 2-(4-[1,1'-biphényl]-4-yl-1H-imidazol-2-yl)éthylamine (obtenue à l'étape 32.2 de l'exemple 32 ; 660 mg ; 0,0025 mol) et de l'isocyanate de n-butyle (341 mg ; 0,0025 mol) dans 20 ml de 1,2-dichloroéthane est agité pendant quinze minutes à 60 °C. La suspension est agitée durant seize heures à 23 °C et filtrée. Le solide obtenu est lavé avec du 1,2-dichloroéthane et de l'éther. On obtient une poudre de couleur blanche avec un rendement de 66%. Point de fusion : 178°C.
MH+ = 363,3.

### Exemple 35 : N-{(S)-cyclohexyl[4-(4-fluorophényl)-1H-imidazol-2-yl]méthyl}cyclobutanamine

### 35.1) (S)-cyclohexyl[4-(4-fluorophényl)-1H-imidazol-2-yl]méthylcarbamate de tert-butyle

Ce composé est obtenu selon un mode opératoire analogue à la préparation du composé de l'étape 31.2 de l'exemple 31 en utilisant la Boc-aminocyclohexylglycine (9,4 g ; 0,036 mol) à la place de la N-(butoxycarbonyl)-β-alanine et la parafluorobromoacétophénone (7,9 g ; 0,036 mol) à la place de la 4-phényl-bromoacétophénone. On obtient une poudre de couleur blanche avec un rendement de 53%.
MH+ = 374,2.

### 35.2) (S)-cyclohexyl[4-(4-fluorophényl)-1H-imidazol-2-yl]méthanamine

Ce composé est préparé selon un mode opératoire analogue à celui de l'étape 32.2 de l'exemple 32 en utilisant le (S)-cyclohexyl[4-(4-fluorophényl)-1H-imidazol-2-yl]méthylcarbamate de *tert-*butyle (7,5 g ; 0,02 mol) comme composé de départ. On obtient une poudre de couleur blanche avec un rendement de 92%.
MH+ = 274,2.

### 35.3) N-{(S)-cyclohexyl[4-(4-fluorophényl)-1H-imidazol-2-yl]méthyl}cyclobutanamine

Un mélange contenant de la (S)-cyclohexyl[4-(4-fluorophényl)-1H-imidazol-2-yl]méthanamine (préparée à l'étape 5.2 ; 519 mg ; 0,0015 mol), de la triéthylamine (0,4 ml ; 0,003 mol) et de la butanone (140 mg ; 0,002 mol) dans 10 ml de méthanol est agité pendant trente minutes à 23 °C. Du triacétoxyborohydrure de sodium (630 mg ; 0,003 mol) est ensuite ajouté. Le mélange réactionnel est agité pendant seize heures puis versé dans de l'eau. Après extraction à l'acétate d'éthyle, la phase organique est lavée avec une solution saturée en chlorure de sodium puis séchée sur sulfate de magnésium. Le solvant est évaporé et le résidu purifié sur colonne de silice (éluant : mélange CH₂Cl₂-MeOH / 95-05). On obtient une poudre de couleur blanche avec un rendement de 12%. Point de fusion : 170-172 °C.
MH+ = 328,2.

### Exemple 36 : N-[1-(4-cyclohexyl-1H-imidazol-2-yl)heptyl)cyclohexanamine

### 36.1) 2-bromo-1-cyclohexyléthanone

De la cyclohexylacétone (5,4 ml, 0,039 mol) et du brome (2 ml, 0,039 mol) sont agités à 23 °C dans 100 ml de méthanol. Après décoloration, 100 ml d'eau sont doucement ajoutés. Le mélange obtenu est neutralisé avec 5 g de bicarbonate de sodium. On extrait avec de l'éther puis lave la phase organique avec 100 ml d'eau. Après séchage sur sulfate de magnésium, le mélange est concentré à l'évaporateur rotatif. On obtient une huile avec un rendement de 97%.
RMN ¹H (δ ppm, DMSO) : 1,21-1,27 (m, 5H) ; 1,59-1,83 (m, 5H) ; 2,59-2,64 (m, 1H) ; 4,42 (s, 2H).

### 36.2) Acide 2-[(tert-butoxycarbonyl)amino]octanoïque

Un mélange d'acide 2-amino-octanoïque (25,25 g ; 0,156 mol) et de dicarbonate de di-*tert-*butyle (37,8 g ; 0,173 mol) dans 425 ml de dioxane est agité à reflux pendant trois heures. Après retour à 23 °C, le mélange est encore agité pendant vingt quatre heures puis l'insoluble filtré. Le filtrat est évaporé. On obtient une huile avec un rendement de 99%.
RMN H¹ (δ ppm, DMSO) : 0,85 (t, 3H) ; 1,11-1,27 (m, 8H) ; 1,37 (s, 9H) ; 1,51-1,65 (m, 2H) ; 3,81-3,87 (m, 1H) ; 6,96-6,97 (m, 1H) ; 12,3 (s, 1H).
IR (cm⁻¹) : 3500; 2860; 1721 (v_{c=o} (acide)) ; 1680 (v_{c=o} (carbamate)) ; 1513 (v_{c-NH} (carbamate)).

### 36.3) 1-(4-cyclohexyl-1H-imidazol-2-yl)heptylcarbamate de tert-butyle

Ce composé est obtenu selon un mode opératoire analogue à celui de l'étape 31.2 de l'exemple 31, en utilisant l'acide 2-[(tert-butoxycarbonyl)amino]octanoïque (8,1 g ; 0,0314 mol) à la place de la N-(butoxycarbonyl)-β-alanine et la 2-bromo-1-cyclohexyléthanone (6,4 g ; 0,0314 mol) à la place de la 4-phényl-bromoacétophénone. On obtient une huile suffisamment propre pour être utilisée dans la réaction suivante (rendement de 88%).

### 36.4) 1-(4-cyclohexyl-1H-imidazol-2-yl)-1-heptanamine

Ce composé est obtenu selon un mode opératoire analogue à celui de l'étape 32.2 de l'exemple 32 en utilisant comme composé de départ le 1-(4-cyclohexyl-1H-imidazol-2-yl)heptylcarbamate de *tert-*butyle (préparé à l'étape 6.3 ; 10 g ; 0,0275 mol). On obtient un solide jaune sous la forme d'une colle (rendement de 37%).
MH+ = 264,2.

### 36.5) N-[1-(4-cyclohexyl-1H-imidazol-2-yl)heptyl]cyclohexanamine

Ce composé est obtenu selon un mode opératoire analogue à celui de l'étape 35.3 de l'exemple 35 en utilisant comme amine de départ la 1-(4-cyclohexyl-1H-imidazol-2-yl)-1-heptanamine (obtenue à l'étape 6.4 ; 2,5 g ; 0,074 mol) et comme cétone la cyclohexanone (1 ml ; 0,0097 mol). Après purification sur colonne de silice (éluant : acétate d'éthyle - heptane / 7-3 à CH₂Cl₂-MeOH / 95-05), on obtient une poudre de couleur blanche avec un rendement de 12%. Point de fusion : 172-174 °C.
MH+ = 346,3.

### Exemple 37 : N-{1-[4-(3-bromophényl)-1H-imidazol-2-yl]-5-méthylhexyl}-N-cyclohexylamine

### 37.1) Acide 2-[(tert-butoxycarbonyl)amino]-6-méthylheptanoïque

Une solution de diisopropylamine (13,2 ml ; 0,094 mol) dans 130 ml de tétrahydrofuranne (THF) est refroidie à -40 °C. Du n-butyllithium (37 ml d'une solution 2,5 M dans l'hexane ; 0,094 mol) est ajouté goutte à goutte. La température est laissée remonter à 0 °C. A cette température, de la Boc-glycine (5 g ; 0,028 mol) en solution dans 30 ml de THF est introduite dans le mélange. On attend dix minutes à cette température puis on ajoute rapidement du 1-bromo-4-méthylpentane (7,9 ml ; 0,056 mol) en solution dans 20 ml de THF. On laisse alors la température revenir à 23 °C et agite le mélange à cette température pendant une heure. Après hydrolyse avec 100 ml d'eau puis acidification avec 150 ml d'une solution saturée en hydrogénosulfate de potassium, le mélange obtenu est extrait avec 2 fois 50 ml d'acétate d'éthyle. La phase organique est lavée avec 100 ml d'eau puis avec 100 ml d'une solution saturée en chlorure de sodium. Après séchage sur sulfate de magnésium et évaporation du solvant, le résidu obtenu est purifié sur colonne de silice (éluant : acétate d'éthyle - heptane / 6-4) pour donner une poudre de couleur blanche avec un rendement de 50%.
MH+ = 260,3.

### 37.2) 1-[4-(3-bromophényl)-1H-imidazol-2-yl]-5-méthylhexylcarbamate de tert-butyle

Ce composé est obtenu selon un mode opératoire analogue à celui de l'étape 31.2 de l'exemple 31 en utilisant l'acide 2-[(tert-butoxycarbonyl)amino]-6-méthylheptanoïque (3,5 g ; 0,0135 mol) à la place de la N-(butoxycarbonyl)-β-alanine et le bromure de 3-bromophénacyle (3,75 g ; 0,0135 mol) à la place de la 4-phényl-bromoacétophénone. On obtient une poudre blanche avec un rendement de 63%. Point de fusion: 134-136 °C.
MH+ = 436,2.

### 37.3) 1-[4-(3-bromophényl)-1H-imidazol-2-yl]-5-méthyl-1-hexanamine

Ce composé est obtenu selon un mode opératoire analogue à celui de l'étape 32.2 de l'exemple 32 en utilisant comme composé de départ le 1-[4-(3-bromophényl)-1H-imidazol-2-yl]-5-méthylhexylcarbamate de *tert*-butyle (obtenu à l'étape 37.2 ; 3,5 g ; 0,008 mol). On obtient une poudre de couleur blanche avec un rendement de 97%. Point de fusion : 200-202 °C.
MH+ = 336,2.

### 37.4) N-{1-[4-(3-bromophény/)-1H-imidazo/-2-yl]-5-méthylhexy/}- N-cyclohexylamine

Ce composé est obtenu selon un mode opératoire analogue à celui de l'étape 35.3 de l'exemple 35 en utilisant comme amine de départ 1-[4-(3-bromophényl)-1H-imidazol-2-yl]-5-méthyl-1-hexanamine (obtenue à l'étape 7.3 ; 0,8 g ; 0,0019 mol) et comme cétone la cyclohexanone (0,32 ml ; 0,0023 mol). On obtient une poudre de couleur blanche avec un rendement de 38%. Point de fusion : 236-238 °C.
MH+ = 418,2.

### Exemple 38 : N-{1-[4-(4-fluorophényl)-1H-imidazol-2-yl]heptyl}cyclohexanamine

### 38.1) 1-[4-(4-fluorophény/)-1H-imidazol-2-yl]heptylcarbamate de tert-butyle

Ce composé est obtenu selon un mode opératoire analogue à celui de l'étape 31.2 de l'exemple 31 en utilisant l'acide 2-[(tert-butoxycarbonyl)amino]octanoïque (6,2 g ; 0,024 mol) à la place de la N-(butoxycarbonyl)-β-alanine et la 2-bromo-4-fluoroacétophénone (5,2 g ; 0,024 mol) à la place de la 4-phényl-bromoacétophénone. On obtient une poudre blanche (rendement : 58%) qui est suffisamment propre pour être utilisée telle quelle pour la suite.

### 38.2) 1-[4-(4-fluorophényl)-1H-imidazol-2-yl]-1-heptanamine

Ce composé est obtenu selon un mode opératoire analogue à celui de l'étape 32.2 de l'exemple 32 en utilisant comme composé de départ le 1-[4-(4-fluorophényl)-1H-imidazol-2-yl]heptylcarbamate de *tert-*butyle (5,2 g ; 0,014 mol). Après purification sur colonne de silice (éluant : CH₂Cl₂-MeOH-NH₄OH / 89-10-1), on obtient une poudre de couleur grise (rendement de 72%). Point de fusion : 148-150 °C.
MH+ = 276,2.

### 38.3) N-{1-[4-(4-fluorophény/)-1H-imidazol-2-yl]heptyl}cyclohexanamine

Ce composé est obtenu selon un mode opératoire analogue à celui de l'étape 35.3 de l'exemple 35 en utilisant comme amine de départ la 1-[4-(4-fluorophényl)-1H-imidazol-2-yl]-1-heptanamine (0,5 g ; 0,0014 mol) et comme cétone la cyclohexanone (0,17 ml ; 0,0014 mol). On obtient une poudre de couleur blanche avec un rendement de 15%. Point de fusion : 190-192°C.
MH+ = 358,2.

### Exemple 39 : (1R)-N-benzyl-1-(1-benzyl-4-tert-butyl-1H-imidazol-2-yl)-2-(1H-indol-3-yl)éthanamine

De la triéthylamine (0,83 ml ; 0,006 mol) est ajoutée à 23 °C à une solution contenant de la (1*R*)-1-(1-benzyl-4-*tert-*butyl-1*H*-imidazol-2-yl)-2-(1*H*-indol-3-yl)éthanamine (0,7 g ; 0,002 mol ; préparée dans des conditions expérimentales analogues aux précédentes et en utilisant les réactifs de départ et les produits de réaction adéquats) dans 15 ml d'acétonitrile. Le mélange est agité une heure à 23°C puis du chlorure de benzyle (0,23 ml ; 0,002 mol) est ajouté. L'agitation est maintenue durant 16 heures. Le mélange réactionnel est concentré à l'aide d'un évaporateur rotatif et l'huile obtenue est reprise avec de l'acétate d'éthyle et de l'eau. La phase aqueuse est extraite à l'acétate d'éthyle et lavée à l'eau puis avec une solution saturée en chlorure de sodium. Les solvants sont évaporés sous vide. Après purification sur colonne de silice (éluant : AE-heptane / 7-3), on obtient un solide de couleur beige foncé sous la forme d'une colle (rendement de 5%). Base libre. Point de fusion : 60-62 °C.
MH+ = 463,3.

### Exempte 40 : (R,S)-N-benzyl-1-(1-benzyl-4-phényl-1H-imidazol-2-yl)-1-heptanamine

De la (R,S)-1-(4-phényl-1*H*-imidazol-2-yl)heptylamine (1 g ; 0,003 mol ; préparée dans des conditions expérimentales analogues aux précédentes et en utilisant les réactifs de départ et produits de réaction adéquats) est diluée dans 20 ml de diméthylformamide. Du carbonate de potassium (2,2 g ; 0,016 mol) est ajouté à 23 °C puis du bromure de benzyle (1,2 ml ; 0,010 mol) est additionné de façon assez lente. Le mélange est agité 72 heures à 23 °C avant d'être versé dans de l'eau glacée. Le mélange est extrait avec de l'acétate d'éthyle. La phase organique est lavée à l'eau puis avec une solution saturée en chlorure de sodium. Après séchage sur sulfate de magnésium, on concentre les solvants à l'aide d'un évaporateur rotatif. Après purification sur colonne de silice (éluant : acétate d'éthyle-heptane / 10-90), on obtient une poudre de couleur blanche (rendement de 31%). Base libre. Point de fusion : 94-96 °C.
MH+ = 438,3.

### Exemple 41 : N-benzyl-N-[(4-[1,1'-biphényl]-4-yl-1H-imidazol-2-yl)méthyl]-1-hexanamine

De la *N*-benzyl(4-[1,1'-biphényl]-4-yl-1*H*-imidazol-2-yl)méthanamine (1 g; 0,0024 mol ; préparée dans des conditions expérimentales analogues aux précédentes et en utilisant les réactifs de départ et produits de réaction adéquats) est diluée dans 15 ml de diméthylformamide. Du carbonate de potassium (1 g ; 0,0073 mol) est ajouté à 23 °C puis du bromure d'hexane (0,34 ml ; 0,0024 mol) est additionné de façon assez lente. Le mélange réactionnel est amené aux environs de 70°C pendant 3 heures avant d'être versé dans de l'eau glacée. Le mélange est extrait avec de l'acétate d'éthyle et la phase organique lavée à l'eau. Après séchage sur sulfate de magnésium, on concentre les solvants à l'aide d'un évaporateur rotatif. Après purification sur colonne de silice (éluant : acétate d'éthyle-heptane / 7-3), on obtient un solide de couleur jaune clair sous la forme d'une colle (rendement de 13%). Base libre. Point de fusion : 120-122 °C.
MH+ = 424,3.

### Exemple 42 : N-benzyl(4-[1,1-biphényl]-4-yl-1H-imidazol-2-yl)-N-méthylméthanamine

De la (4-[1,1'-biphényl]-4-yl-1*H*-imidazol-2-yl)-*N*-méthylméthanamine (1 g; 0,003 mol ; préparée dans des conditions expérimentales analogues aux précédentes et en utilisant les réactifs de départ et produits de réaction adéquats) est diluée dans 20 ml de diméthylformamide. Du carbonate de potassium (1,23 g ; 0,009 mol) est ajouté à 23 °C puis du bromure de benzyle (0,34 ml ; 0,003 mol) est additionné de façon assez lente. Le mélange réactionnel est agité à cette température pendant 48 heures puis versé dans de l'eau glacée. Le mélange est extrait avec de l'acétate d'éthyle et la phase organique lavée à l'eau. Après séchage sur sulfate de magnésium, on concentre les solvants à l'aide d'un évaporateur rotatif. Après purification sur colonne de silice (éluant : acétate d'éthyle -heptane / 8-2), on obtient un solide de couleur blanche sous forme de colle (rendement de 16%). Base libre. Point de fusion : 106-108 °C.
MH+ = 354,2.

### Exemple 43 : (R,S)-N,N-dihexyl-1-(4-phényl-1H-imidazol-2-yl)-1-heptanamine

De la (R,S)-1-(4-phényl-1*H*-imidazol-2-yl)-1-heptanamine (1 g ; 0,003 mol ; préparée dans des conditions expérimentales analogues aux précédentes et en utilisant les réactifs de départ et produits de réaction adéquats) est diluée dans 10 ml de méthanol. De la triéthylamine (0,9 ml ; 0,006 mol) est ajoutée goutte à goutte puis le mélange agité pendant 30 minutes à 23 °C. De l'hexanal (0,45 ml ; 0,0036 mol) est ensuite ajouté puis le mélange agité pendant une heure à 23°C. Du triacétoxyborohydrure de sodium (1,3 g ; 0,006 mol) est enfin ajouté. Après deux heures d'agitation à 23 °C, de l'eau est rajoutée et le mélange réactionnel extrait avec de l'acétate d'éthyle. La phase organique est lavée à l'eau et séchée sur sulfate de magnésium avant l'évaporation des solvants. Après purification sur colonne de silice (éluant : acétate d'éthyle-heptane / 6-4), on obtient un solide de couleur marron sous la forme d'une colle (rendement de 3%). Base libre. Le point de fusion n'a pu être mesuré (colle).
MH+ = 426,4.

### Exemple 44 : N-[(1R)-2-(1H-indol-3-yl)-1-(4-phényl-1H-imidazol-2-yl)éthyl]-2-pyrimidinamine

De la (1*R*)-2-(1*H*-indol-3-yl)-1-(4-phényl-1*H*-imidazol-2-yl)éthanamine (2 g ; 0,0066 mol ; préparée dans des conditions expérimentales analogues aux précédentes et en utilisant les réactifs de départ et produits de réaction adéquats) est diluée dans 10 ml de n-butanol. De la 2-bromopyrimidine (1 g ; 0,0066 mol) puis de la diisoéthylamine (1,15 ml, 0,0066 mol) sont ajoutées goutte à goutte. Le mélange est ensuite chauffé aux environs de 80 °C pendant 16 heures.Le *n*-butanol est évaporé puis le résidu repris dans de l'eau et de l'acétate d'éthyle. La phase organique est lavée à l'eau puis avec une solution saturée en chlorure de sodium avant d'être séchée sur sulfate de magnésium et concentrée à l'aide d'un évaporateur rotatif. Après purification sur colonne de silice (éluant : acétate d'éthyle-heptane / 7-3 puis CH₂Cl₂-MeOH-NH₄OH/ 95-4,5-0,5 puis acétate d'éthyle), on obtient une poudre de couleur blanche (le rendement est 20%). Base libre. Point de fusion : 138-140 °C.
MH+ = 381,2.

### Exemple 45 : (1-benzyl-4-phényl-1H-imidazol-2-yl)-N,N-diméthylméthanamine

De la (1-benzyl-4-phényl-1*H*-imidazol-2-yl)méthanamine (0,6 g ; 0,0018 mol ; préparée dans des conditions expérimentales analogues aux précédentes et en utilisant les réactifs de départ et produits de réaction adéquats) est diluée dans 15 ml de tétrahydrofuranne. De la triéthylamine (1,12 ml ; 0,008 mol) puis du 4-toluènesulfonate de méthyle (0,75 g ; 0,004 mol) sont ajoutés goutte à goutte. Le mélange est agité 48 heures à 23°C puis versé dans de l'eau glacée. Après extraction avec de l'éther puis décantation, la phase organique est lavée à l'eau puis avec une solution saturée en chlorure de sodium. La phase organique est ensuite séchée sur sulfate de magnésium et concentrée à l'aide d'un évaporateur rotatif. Après purification sur colonne de silice (éluant : acétate d'éthyle-heptane / 7-3 puis CH₂Cl₂-MeOH / 95-5), on obtient une poudre de couleur blanche (rendement de 44%). Base libre. Point de fusion : 78-80 °C.
MH+ = 292,2.

### Exemple 46 : (1R)-N-benzyl-2-(1H-indol-3-yl)-N-métbyl-1-(4-phényl-1H-imidazol-2-yl)éthanamine

De la (1*R*)-N-benzyl-2-(1*H*-indol-3-yl)-1-(4-phényl-1*H*-imidazol-2-yl)éthanamine (0,5 g ; 0,00127 mol ; préparée dans des conditions expérimentales analogues à celle de l'exemple 38 en utilisant les réactifs de départ et produits de réaction adéquats) est diluée dans 25 ml de tétrahydrofuranne. Du tosylate de méthyle (0,24 g ; 0,00127 mol) est ajouté à la solution précédente à 23 °C puis du *tert-*butylate de potassium (0,15 g ; 0,00127 mol) est ajouté de façon assez lente. L'agitation à 23°C est maintenue pendant deux heures puis le mélange chauffé aux environs de 60°C pendant huit heures. Le solvant est évaporé et le résidu obtenu repris avec de l'acétate d'éthyle et une solution de bicarbonate de sodium à 10%. Après décantation, la phase organique est lavée à l'eau et séchée sur sulfate de magnésium. Le solvant est alors évaporé. Après purification sur colonne de silice (éluant : acétate d'éthyle-heptane / 7-3), on obtient un solide de couleur beige clair sous la forme d'une colle (rendement de 4%). Base libre. Point de fusion : 110-112 °C.
**NIH+** = 407,3.
*Les composés des exemples 47 à 318 sont obtenus selon des procédures analogues à celles décrites pour les exemples 31 à 46 ou ci-dessus dans la partie intitulée « Préparation des composés de formule générale (I)* ».

### Exemple 47 : (1R)-2-(1H-indol-3-yl)-N-(2-phényléthyl)-1-(4-phényl-1H-imidazol-2-yl)éthanamine

Base libre. Le point de fusion n'a pu être mesuré (colle).

### Exemple 48 : (1R)-N-benzyl-2-phényl-1-(4-phényl-1H-imidazol-2-yl)éthanamine

Base libre. Point de fusion : 228-230 °C.

### Exemple 49 : N-benzyl(4-phényl-1H-imidazol-2-yl)méthanamine

Base libre. Le point de fusion n'a pu être mesuré (colle).

### Exemple 50 : (1R)-1-(4-tert-butyl-1H-imidazol-2-yl)-2-(1H-indol-3-yl)-éthylcarbamate de tert-butyle

Base libre. Point de fusion : 104-106 °C.

### Exemple 51 : (4-phényl-1H-imidazol-2-yl)méthanamine

Chlorhydrate. Point de fusion : 228-230 °C.

### Exemple 52 : 1-méthyl-1-(4-phényl-1H-imidazol-2-yl)éthylamine

Chlorhydrate. Point de fusion : 200-204 °C.

### Exemple 53 : N-[(1S)-2-(1H-indol-3-yl)-1-(4-phényl-1H-imidazol-2-yl)éthyl]-1-hexanamine

Chlorhydrate. Point de fusion : 132-134 °C.

### Exemple 54 : (R,S)-1-(4-phényl-1H-imidazol-2-yl)heptylcarbamate de tert-butyle

Base libre. Point de fusion : 102-104 °C.

### Exemple 55 : (4-[1,1'-biphényl]-4-yl-1-méthyl-1H-imidazol-2-yl)méthanamine

Chlorhydrate. Point de fusion : 279-280 °C.

### Exemple 56 : (1S)-3-méthyl-1-(4-phényl-1H-imidazol-2-yl)-1-butanamine

Chlorhydrate. Point de fusion : 150-152 °C.

### Exemple 57 : 2-[4-(4-phénoxyphényl)-1H-imidazol-2-yl]éthylcarbamate de butyle

Base libre. Le point de fusion n'a pu être mesuré (colle).

### Exemple 58 : (R,S)-N-[2-(1-méthyl-1H-indol-3-yl)-1-(4-phényl-1H-imidazol-2-yl)éthyl]-1-butanamine

Base libre. Le point de fusion n'a pu être mesuré (colle).

### Exemple 59 : (R,S)-4-(2-{1-[(tert-butoxycarbonyl)amino]pentyl}-1H-imidazol-4-yl)-1,1'-biphényle

Base libre. Point de fusion : 172-176 °C.

### Exemple 60 : (R,S)-N-benzyl-1-(4-[1,1'-biphényl]-4-yl-1H-imidazol-2-yl)-1-pentanamine

Base libre. Point de fusion : 201-203 °C.

### Exemple 61 : N-[2-(4-[1,1'-biphényl]-4-yl-1H-imidazol-2-yl)éthyl]-3,3-diméthyl-butanamide

Base libre. Point de fusion : 186-188 °C.

### Exemple 62 : (1R)-N-benzyl-1-(4,5-diméthyl-1,3-oxazol-2-yl)-2-(1H-indol-3-yl)éthanamine

Base libre. Le point de fusion n'a pu être mesuré (colle).

### Exemple 63 : (R,S)-1-(4-phényl-1H-imidazol-2-yl)hexylcarbamate de tert-butyle

Base libre. Le point de fusion n'a pu être mesuré (colle).

### Exemple 64 : (R,S)-N-hexyl-1-(4-phényl-1H-imidazol-2-yl)-1-heptanamine

Base libre. Point de fusion : 140-142 °C.

### Exemple 65 : (R,S)-1-(4-phényl-1H-imidazol-2-yl)hexylamine

Chlorhydrate. Point de fusion : 146-148 °C.

### Exemple 66 : (R,S)-N-benzy-1-[4-(4-méthoxyphényl)-1H-imidazol-2-yl]-1-heptanamine

Chlorhydrate. Point de fusion : à partir de 115 °C.

### Exemple 67 : (R,S)-N-(2,6-dichlorobenzyl)-1-(4-phényl-1H-imidazol-2-yl)-1-heptanamine

Base libre. Le point de fusion n'a pu être mesuré (colle).

### Exemple 68 : (R,S)-N-(4-chlorobenzyl)-1-(4-phényl-1H-imidazol-2-yl)-1-heptanamine

Base libre. Le point de fusion n'a pu être mesuré (colle).

### Exemple 69 : (R,S)-1-[4-(3-méthoxyphényl)-1H-imidazol-2-yl]heptylamine

Chlorhydrate. Point de fusion : 110-112 °C.

### Exemple 70 : (R,S)-N-(2-chlorobenzyl)-1-(4-phényl-1H-imidazol-2-yl)-1-heptanamine

Base libre. Le point de fusion n'a pu être mesuré (colle).

### Exemple 71 : (R,S)-N-(2-fluorobenzyl)-1-(4-phényl-1H-imidazol-2-yl)-1-heptanamine

Base libre. Le point de fusion n'a pu être mesuré (colle).

### Exemple 72 : (R,S)-N-butyl-1-(4-phényl-1H-imidazol-2-yl)-1-heptanamine

Base libre. Le point de fusion n'a pu être mesuré (colle).

### Exemple 73 : (R,S)-N-isopentyl-N-[1-(4-phényl-1H-imidazol-2-yl)heptyl]amine

Base libre. Le point de fusion n'a pu être mesuré (colle).

### Exemple 74 : (R,S)-1-[4-(3-bromophényl)-1H-imidazol-2-yl]-N-hexyl-1-heptanamine

Base libre. Le point de fusion n'a pu être mesuré (colle).

### Exemple 75 : (R,S)-N-pentyl-1-(4-phényl-1H-imidazol-2-yl)-1-heptanamine

Base libre. Point de fusion : 118-120 °C.

### Exemple 76 : (R,S)-N-[1-(4-phényl-1H-imidazol-2-yl)heptyl]cyclohexanamine

Base libre. Point de fusion : 68-70 °C.

### Exemple 77 : (R,S)-N-benzyl-1-[4-(3,4-dichlorophényl)-1H-imidazol-2-yl]-1-heptanamine

Base libre. Point de fusion : 192-194 °C.

### Exemple 78 : (4-[1,1'-biphényl]-4-yl-1H-imidazol-2-yl)méthylcarbamate de butyle

Base libre. Point de fusion : 130-132 °C.

### Exemple 79 : (R,S)-N-[1-(4-phényl-1H-imidazol-2-yl)heptyl]cyclopentanamine

Base libre. Le point de fusion n'a pu être mesuré (colle).

### Exemple 80 : (S)-cyclohexyl(4-phényl-1H-imidazol-2-yl)méthylamine

Chlorhydrate. Point de fusion : 208-210 °C.

### Exemple 81 : (R,S)-N-{1-[4-(2-chlorophényl)-1H-imidazol-2-yl]heptyl}-cyclohexanamine

Chlorhydrate. Point de fusion : 155-157 °C.

### Exemple 82 : N-[(S)-cyclohexyl(4-cyclohexyl-1H-imidazol-2-yl)méthyl]-cyclohexanamine

Chlorhydrate. Point de fusion : 180-182 °C.

### Exemple 83 : N-[(S)-cyclohexyl(4-phényl-1H-imidazol-2-yl)méthyl]-cyclobutanamine

Chlorhydrate. Point de fusion : 210-212 °C.

### Exemple 84 : (R,S)-N-{1-[4-(4-fluorophényl)-1H-imidazol-2-yl]heptyl}-cyclobutanamine

Chlorhydrate. Point de fusion : 144-146 °C.

### Exemple 85 : N-{(S)-cyclohexyl[4-(3-fluoro-4-méthoxyphényl)-1H-imidazol-2-yl]méthyl}cyclobutanamine

Base libre. Point de fusion : à partir de 95 °C.

### Exemple 86 : N-((S)-cyclohexyl{4-[4-(trifluorométhyl)phényl]-1H-imidazol-2-yl}méthyl)cyclobutanamine

Base libre. Mousse.

### Exemple 87 : N-{(S)-cyclohexyl[4-(3-fluorophényl)-1H-imidazol-2-yl]méthyl}-cyclobutanamine

Base libre. Point de fusion : 172-176 °C.

### Exemple 88 : (1R)-N-benzyl-2-(1H-indol-3-yl)-1-(4-phényl-1H-imidazol-2-yl)éthanamine

Base libre. Point de fusion : 100-102 °C.

### Exemple 89 : (R,S)-2-(1H-indol-3-yl)-1-(5-méthyl-4-phényl-1H-imidazol-2-yl)éthanamine

Chlorhydrate. Point de fusion : 208-210 °C.

### Exemple 90 : (1R)-1-(4,5-diphényl-1H-imidazol-2-yl)-2-(1H-indol-3-yl)éthanamine

Chlorhydrate. Point de fusion : > 260 °C.

### Exemple 91 : (R,S)-2-phényl-1-(4-phényl-1H-imidazol-2-yl)éthanamine

Chlorhydrate. Point de fusion : 180-182 °C.

### Exemple 92 : (R,S)-2-(1-méthyl-1H-indol-3-yl)-1-(4-phényl-1H-imidazol-2-yl)éthylamine

Chlorhydrate. Point de fusion : 110-114 °C.

### Exemple 93 : (1S)-N-benzyl-2-(1H-indol-3-yl)-1-(4-phényl-1H-imidazol-2-yl)éthanamine

Base libre. Point de fusion : 118-120 °C.

### Exemple 94 : (1R)-N-benzyl-1-(4,5-diphényl-1H-imidazol-2-yl)-2-(1H-indol-3-yl)éthanamine

Base libre. Point de fusion : 146-148 °C.

### Exemple 95 : (1R)-N-benzyl-2-(1H-indol-3-yl)-1-(5-méthyl-4-phényl-1H-imidazol-2-yl)éthanamine

Base libre. Point de fusion : 120-122 °C.

### Exemple 96 : (1R)-2-(1H-indol-3-yl)-1-(4-phényl-1H-imidazol-2-yl)-éthylcarbamate de tert-butyle

Base libre. Point de fusion : 208-210 °C.

### Exemple 97 : (1R)-2-(1H-indol-3-yl)-1-(4-phényl-1H-imidazol-2-yl)éthanamine

Chlorhydrate. Le point de fusion n'a pu être mesuré (colle).

### Exemple 98 : N-[(1R)-2-(1H-indol-3-yl)-1-(4-phényl-1H-imidazol-2-yl)éthyl]benzamide

Base libre. Point de fusion : 218-220 °C.

### Exemple 99 : (1R)-2-(1H-indol-3-yl)-1-(4-phényl-1H-imidazol-2-yl)éthylcarbamate de benzyle

Base libre. Point de fusion : 105-108 °C.

### Exemple 100 : (1R)-N-benzyl-2-(1H-indol-3-yl)-1-(4-phényl-1,3-thiazol-2-yl)éthanamine

Base libre. Point de fusion : 134-136 °C.

### Exemple 101 : N-[(1R)-2-(1H-indol-3-yl)-1-(4-phényl-1,3-thiazol-2-yl)-éthyl]benzamide

Base libre. Point de fusion : 108-110 °C.

### Exemple 102 : (1R)-2-(1H-indol-3-yl)-1-[4-(4-nitrophényl)-1H-imidazol-2-yl]-éthylcarbamate de tert-butyle

Base libre. Point de fusion: 220-222 °C.

### Exemple 103 : (4-phényl-1H-imidazol-2-yl)méthylcarbamate de tert-butyle

Base libre. Point de fusion : 170-172 °C.

### Exemple 104 : (1-benzyl-4-phényl-1H-imidazol-2-yl)méthylcarbamate de tert-butyle

Base libre. Point de fusion : 140-142 °C.

### Exemple 105 : (R,S)-N-benzyl-2-(6-fluoro-1H-indol-3-yl)-1-(4-phényl-1H-imidazol-2-yl)éthanamine

Base libre. Point de fusion : 98-100 °C.

### Exemple 106 : (1R)-2-(1H-indol-3-yl)-1-[4-(4-nitrophényl)-1H-imidazol-2-yl]éthanamine

Chlorhydrate. Point de fusion : devient collant vers 220 °C.

### Exemple 107 : (1-benzyl-4-pbényl-1H-imidazol-2-yl)méthanamine

Chlorhydrate. Point de fusion : 248-250 °C.

### Exemple 108 : (1R)-2-(1H-indol-3-yl)-N-(2-phénoxyéthyl)-1-(4-phényl-1H-imidazol-2-yl)éthanamine

Base libre. Point de fusion : 94-96 °C.

### Exemple 109 : (1R)-1-(4-tert-butyl-1H-imidazol-2-yl)-2-(1H-indol-3-yl)éthylamine

Chlorhydrate. Point de fusion : 230-232 °C.

### Exemple 110 : N-benzyl(1-benzyl-4-phényl-1H-imidazol-2-yl)méthanamine

Base libre. Point de fusion : 60-62 °C.

### Exemple 111 : (1R)-2-(1-benzothién-3-yl)-N-benzyl-1-(4-phényl-1H-imidazol-2-yl)éthanamine

Base libre. Point de fusion : 152-154 °C.

### Exemple 112 : (1R)-2-(1H-indol-3-yl)-N-(2-phénoxyéthyl)-1-(4-phényl-1,3-thiazol-2-yl)éthanamine

Base libre. Point de fusion : 124-126 °C.

### Exemple 113 : 1-(4-phényl-1H-imidazol-2-yl)cyclohexylcarbamate de tert-butyle

Base libre. Point de fusion : 170-172 °C.

### Exemple 114 : (R,S)-2-(6-chloro-1H-indol-3-yl)-1-(4-phényl-1H-imidazol-2-yl)éthylcarbamate de test-butyle

Base libre. Point de fusion : 208-210 °C.

### Exemple 115 : 1-(4-phényl-1H-imidazol-2-yl)cyclohexanamine

Chlorhydrate. Point de fusion : 202-204 °C.

### Exemple 116 : N-[(1R)-2-(1H-indol-3-yl)-1-(4-phényl-1H-imidazol-2-yl)éthyl]-N'-phénylurée

Base libre. Composé décrit dans la demande PCT WO 99/64401.

### Exemple 117 : N-[(1R)-2-(1H-indol-3-yl)-1-(4-phényl-1H-imidazol-2-yl)éthyl]benzènecarboximidamide

Base libre. Composé décrit dans la demande PCT WO 99/64401.

### Exemple 118 : (1R)-N-(cyclohexylméthyl)-2-(1H-indol-3-yl)-1-(4-phényl-1H-imidazol-2-yl)éthanamine

Base libre. Composé décrit dans la demande PCT WO 99/64401.

### Exemple 119 : (R,S)-N¹-benzyl-1-(4-phényl-1H-imidazol-2-yl)-1,5-pentanediamine

Base libre. Composé décrit dans la demande PCT WO 99/64401.

### Exemple 120 : (R,S)-5-(benzylamino)-5-(4-phényl-1H-imidazol-2-yl)pentylcarbamate de tert-butyle

Base libre. Composé décrit dans la demande PCT WO 99/64401.

### Exemple 121 : N-[(1R)-2-(1H-indol-3-yl)-1-(4-phényl-1H-imidazol-2-yl)éthyl]-4-méthoxybenzènecarboximidamide

Base libre. Composé décrit dans la demande PCT WO 99/64401.

### Exemple 122 : (R,S)-2-(6-chloro-1H-indol-3-yl)-1-(4-phényl-1H-imidazol-2-yl)éthylamine

Chlorhydrate. Point de fusion : 210-212 °C.

### Exemple 123 : N-benzyl-1-(4-phényl-1H-imidazol-2-yl)cyclohexanamine

Base libre. Point de fusion : 114-116 °C.

### Exemple 124 : (1R)-3-méthyl-1-(4-phényl-1H-indazol-2-yl)butylcarbamate de tert-butyle

Base libre. Point de fusion : 88-90 °C.

### Exemple 125 : (1R)-N-benzyl-3-méthyl-1-(4-phényl-1H-imidazol-2-yl)-1-butanamine

Base libre. Point de fusion : 134-135 °C.

### Exemple 126: (R,S)-phényl(4-phényl-1H-imidazol-2-yl)méthylcarbamate de tert-butyle

Base libre. Point de fusion : 134-136 °C.

### Exemple 127: 1-méthyl-1-(4-pbényl-1H-imidazol-2-yl)éthylcarbamate de tert-butyle

Base libre. Point de fusion : 130-132 °C.

### Exemple 128: (R,S)-phényl(4-phényl-1H-imidazol-2-yl)méthylamine

Chlorhydrate. Le point de fusion n'a pu être mesuré (colle).

### Exemple 129: (1R)-3-phényl-1-(4-phényl-1H-imidazol-2-yl)propylcarbamate de tert-butyle

Base libre. Point de fusion : 72-74 °C.

### Exemple 130: (1R)-2-cyclohexyl-1-(4-phényl-1H imidazol-2-yl)éthylcarbamate de tert-butyle

Base libre. Point de fusion : 184-185 °C.

### Exemple 131: (1R)-3-phényl-1-(4-phényl-1H-imidazol-2-yl)-1-propanamine

Chlorhydrate. Point de fusion : 174-176 °C.

### Exemple 132: (1R)-2-cydohexyl-1-(4-phényl-1H-imidazol-2-yl)éthanamine

Chlorhydrate. Point de fusion : 196-198 °C.

### Exemple 133: (R,S)-N-benzyl(phényl)(4-phényl-1H-imidazol-2-yl)méthanamine

Base libre. Point de fusion : 144-146 °C.

### Exemple 134: (1R)-N-benzyl-2-cyclohexyl-1-(4-phényl-1H-imidazol-2-yl)éthanamine

Base libre. Point de fusion : 52-54 °C.

### Exemple 135: (1R)-N-benzyl-3-phényl-1-(4-phényl-1H-imidazol-2-yl)-1-propanamine

Base libre. Point de fusion : 142-144 °C.

### Exemple 136: (R,S)-N-{5,5,5-trifluoro-1-[4-(4-fluorophényl)-1H-imidazol-2-yl]pentyl}cyclohexanamine

Base libre. Point de fusion : 220 °C.

### Exemple 137: 4-(2-{[(tert-butoxycarbonyl)amino]méthyl}-1H-imidazol-4-yl)-1,1'-biphényle

Base libre. Point de fusion : 100-102 °C.

### Exemple 138: N-{(S)-cyclohexyl[4-(4-méthylsulfonylphényl)-1H-imidazol-2-yl]méthyl}cyclohexanamine

Base libre. Point de fusion : 152-154 °C.

### Exemple 139: N-benzyl-2-(4-phényl-1H-imidazol-2-yl)-2-propanamine

Base libre. Point de fusion : 136-138 °C.

### Exemple 140: 4-(1-benzyl-2-{[(tert-butoxycarbonyl)amino]méthyl}-1H-imidazol-4-yl)-1,1'-biphényle

Base libre. Point de fusion : 167-169 °C.

### Exemple 141: (4-[1,1'-biphényl]-4-yl-1H-imidazol-2-yl)méthanamine

Chlorhydrate. Point de fusion : 240-242 °C.

### Exemple 142: (R,S) 1-(4-phényl-1H-imidazol-2-yl)heptylamine

Chlorhydrate. Point de fusion : 131-134 °C.

### Exemple 143: (1-benzyl-4-[1,1'-biphényl]-4-yl-1H-imidazol-2-yl)méthanamine

Chlorhydrate. Point de fusion : 170-174 °C.

### Exemple 144: N,N dibenzyl(4-[1,1'-biphényl]-4-yl-1H-imidazol-2-yl)méthanamine

Base libre. Point de fusion : 70-74 °C.

### Exempte 145: (R,S)-N-benzyl-1-(4-phényl-1H-imidazol-2-yl)-1-heptanamine

Base libre. Point de fusion : 160-162 °C.

### Exemple 146: 4-(2-{[(tert-butoarycarbonyl)amino]méthyl}-1-méthyl-1H-imidazol-4-yl)-1,1'-biphényle

Base libre. Point de fusion : 208-210 °C.

### Exemple 147: (1S)-1-(4,5-diphényl-1H-imidazol-2-yl)-2-(1H-indol-3-yl)éthylcarbamate de test-butyle

Base libre. Point de fusion : 142-143 °C.

### Exemple 148: (1R)-2-(1H-indol-3-yl)-(1-méthyl-4-phényl-1H-imidazol-2-yl)éthylcarbamate de test-butyle

Base libre. Point de fusion : 96-100 °C.

### Exemple 149: 4-(2-{[(tert-butoxycarbonyl)(méthyl)amino]méthyl}-1H-imidazol-4-yl)-1,1'-biphényle

Base libre. Point de fusion : 72-74 °C.

### Exemple 150: 4-(2-{(1R)-1-[(tert-butoxycarbonyl)amino]-2-cyclohexyléthyl}-1H-imidazol-4-yl)-1,1'-biphényle

Base libre. Point de fusion : 112-114 °C.

### Exemple 151: (1R)-2-(1H-indol-3-yl)-1-(l-méthyl-4-phényl-1H-imidazol-2-yl)éthanamine

Chlorhydrate. Point de fusion : 206-210 °C.

### Exemple 152: 4-(2-{2-[(tert-butoxycarbonyl)amino]éthyl}-1H imidazol-4-yl) 1,1'-biphényle

Base libre. Point de fusion: 140-142 °C.

### Exemple 153: méthyl[(5-méthyl-4-phényl-1H-imidazol-2-yl)méthyl]carbamate de tert-butyle

Base libre. Point de fusion : 70-72 °C.

### Exemple 154: (1R)-1-(4-[1,1'-biphényl]-4-yl-1H-imidazol-2-yl)-2-cyclobexyléthanamine

Chlorhydrate. Point de fusion : 178-180 °C.

### Exemple 155: (4-[1,1'-biphényl]-4-yl-1H-imidazol-2-yl)N-méthylméthanamine

Chlorhydrate. Point de fusion : 218-220 °C.

### Exemple 156: (4,5-diphényl-1H-imidazol-2-yl)méthyl(méthyl)carbamate de tert-butyle

Base libre. Point de fusion : 170-172 °C.

### Exemple 157: (4,5-diphényl-1H-imidazol-2-yl)méthylcarbamate de tert-butyle

Base libre. Point de fusion: 144-146 °C.

### Exemple 158: N-méthyl-(5-méthyl-4-phényl-1H-imidazol-2-yl)méthanamine

Chlorhydrate. Point de fusion : 218-220 °C.

### Exemple 159: (R,S)-N,N-dibenzyl-1-(1-benzyl-4-phényl-1H-imidazol-2-yl)-1-heptanamine

Chlorhydrate. Point de fusion : 130-132 °C.

### Exempte 160: (4,5-diphényl-1H-imidazol-2-yl)méthanamine

Chlorhydrate. Point de fusion : 210-212 °C.

### Exemple 161: 2-(4-[1,1'-biphényl]-4-yl-1H-imidazol-2-yl)éthanamine

Chlorhydrate. Point de fusion : 228-230 °C.

### Exemple 162: (4,5-diphényl-1H-imidazol-2-yl)-N-méthylméthanamine

Chlorhydrate. Point de fusion : 198-200 °C.

### Exemple 163: N-benzyl(4,5-diphényl-1H-imidazol-2-yl)méthanamine

Base libre. Point de fusion : 160-162 °C.

### Exemple 164: N-benzyl-2-(4-[1,1'-biphényl]-4-yl-1H-imidazol-2-yl)éthanamine

Base libre. Point de fusion : 174-176 °C.

### Exemple 165: 4-(2-{[benzyl(tert-butoxycarbonyl)amino]méthyl}-1H-imidazol-4-yl)-1,1'-biphényle

Base libre. Point de fusion : 130-132 °C.

### Exemple 166: (1R)-1-(4-[1,1'-biphényl]-4-yl-1H-imidazol-2-y1)-3-phényl-1-propanamine

Chlorhydrate. Point de fusion : 215-218 °C.

### Exemple 167: 4-(2-{(1R)-1-[(tert-butoxycarbonyl)amino]-3-phénylpropyl}-1H-imidazol-4-yl)-1,1'-biphényle

Base libre. Point de fusion : 154-156 °C.

### Exemple 168: N-benzyl(4-[1,1'-bipbényl]-4-yl-1H-imidazol-2-yl)méthanamine

Chlorhydrate. Point de fusion : > 250 °C.

### Exemple 169: (1R)-N-benzyl-1-(4-[1,1'-biphényl]-4-yl-1H-imidazol-2-yl)-2-cyclohexyléthanamine

Base libre. Point de fusion : 233-238 °C.

### Exemple 170: (1R)-N-benzyl-1-(4-[1,1'-biphényl]-4-yl-1H-imidazol-2-yl)-3-phényl-1-propanamine

Base libre. Point de fusion : 210-213 °C.

### Exemple 171: 4-(2-{3-[(tert-butoxycarbonyl)amino]propyl}-1H-imidazol-4-y)-1,1'-biphényle

Base libre. Point de fusion : 145-146 °C.

### Exemple 172: 4-[2-(2-{[(tert-butylamino)carbothioyl]amino}éthy)-1H-imidazol-4-yl]-1,1'-biphényle

Base libre. Point de fusion : 98-99 °C.

### Exemple 173: 6-(4-phényl-1H-imidazol-2-yl)hexylcarbamate de tert-butyle

Base libre. Le point de fusion n'a pu être mesuré (colle).

### Exemple 174: (R,S)-1-(4-phényl-1H-imidazol-2-yl)pentylcarbamate de tert-butyle

Base libre. Point de fusion : 126 °C.

### Exemple 175: (R,S)-1-(4-[1,1'-biphényl]-4-yl-1H-imidazol-2-yl)-1-pentanamine

Chlorhydrate. Point de fusion : 197-200 °C.

### Exemple 176: N-[2-(4-[1,1'-biphényl]-4-yl-1H-imidazol-2-yl)éthyl]-1-hexanamine

Base libre. Point de fusion : 152-154 °C.

### Exemple 177: 4-[2-(2-{[(tert-butylamino)carbonyl]amino}éthyl)-1H-imidazol-4-yl]-1,1'-biphényle

Base libre. Point de fusion : 195-196 °C.

### Exemple 178: N-benzyl-3-(4-[1,1'-biphényl]-4-yl-1H-imidazol-2-yl)-1-propanamine

Base libre. Point de fusion : 254-256 °C.

### Exemple 179: 3-(4-[1,1'-biphényl]-4-yl-1H-imidazol-2-yl)-1-propanamine

Chlorhydrate. Point de fusion : > 260 °C.

### Exemple 180: 6-(4-phényl-1H-imidazol-2-yl)hexylamine

Chlorhydrate. Point de fusion : 244-246 °C.

### Exemple 181: (R,S)-1-(4-phényl-1H-imidazol-2-yl)pentylamine

Chlorhydrate. Point de fusion : 178-180 °C.

### Exemple 182: (RS)-1-[4-(4-méthylphényl)-1H-imidazol-2-yl]heptylcarbamate de tert-butyle

Base libre. Point de fusion : 77-80 °C.

### Exemple 183: (R,S)-1-[4-(2-méthoxyphényl)-1H-imidazol-2-yl]heptylcarbamate de tert-butyle

Base libre. Point de fusion : 64-65 °C.

### Exemple 184: (R,S)-1-[4-(4-méthylphényl)-1H-imidazol-2-yl]-1-heptanamine

Chlorhydrate. Point de fusion : 157-160 °C.

### Exemple 185: (R,S)-1-[4-(2-méthoxyphényl)-1H-imidazol-2-yl]heptylamine

Chlorhydrate. Point de fusion : 238-240 °C.

### Exemple 186: (R,S)-N-benzyl-1-(4-phényl-1H-imidazol-2-yl)-1-pentanamine

Base libre. Point de fusion : 200-202 °C.

### Exemple 187: (R,S)-1-[4-(4-méthoxyphényl)-1H-imidazol-2-yl]heptylcarbamate de tert-butyle

Base libre. Point de fusion : 125-127 °C.

### Exemple 188: (RS)-1-(4-[1,1'-biphényl]-4-yl-1H-imidazol-2-yl)-1-heptanamine

Chlorhydrate. Point de fusion : 182-184 °C.

### Exemple 189: (RS)-1-[4-(3-bromophényl)-1H-imidazol-2-yl]heptylcarbamate de tert-butyle

Base libre. Point de fusion : 141-143 °C.

### Exemple 190: (R,S)-1-[4-(4-méthoxyphényl)-1H-imidazol-2-yl]heptylamine

Chlorhydrate. Point de fusion : 231-232 °C.

### Exemple 191: (R,S)-1-[4-(3-bromophényl)-1H-imidazol-2-yl]-1-heptanamine

Chlorhydrate. Point de fusion : 230-231 °C.

### Exemple 192: (R,S)-4-(2-{1-[(tert-butoxycarbonyl)amino]heptyl}-1H-imidazol-4-yl)-1,1'-biphényle

Base libre. Point de fusion : 142-144 °C.

### Exemple 193: (R,S)-N-benzyl-1-[4-(3-bromophényl)-1H-imidazol-2-yl]-1-heptanamine

Acétate. Point de fusion : 115-116 °C.

### Exemple 194: 4-(2-{(1S)-1-[(tert-butoxycarbonyl)amino]propyl}-1H-imidazol-4-yl)-1,1'-biphényle

Base libre. Point de fusion : 138-140 °C.

### Exemple 195: (R,S)-N-benzyl-1-(4-[1,1'-biphényl]-4-yl-1H-imidazol-2-yl)-1-heptanamine

Base libre. Point de fusion : 100-102 °C.

### Exemple 196: (1S)-1-(4-[1,1'-biphényl]-4-yl-1H-imidazol-2-yl)-1-propanamine

Chlorhydrate. Point de fusion : > 250 °C.

### Exemple 197: (1S)-1-(4,5-diphényl-1H imidazol-2-yl)propylcarbamate de tert-butyle

Base libre. Point de fusion : 136-138 °C.

### Exemple 198: (1S)-N-benzyl-1-(4-[1,1'-biphényl]-4-yl-1H-imidazol-2-yl)-1-propanamine

Base libre. Point de fusion : 220-222 °C.

### Exemple 199: (1S)-1-(4,5-diphényl-1H-imidazol-2-yl)-1-propanamine

Chlorhydrate. Point de fusion : 224-226 °C.

### Exemple 200: (R,S)-N-benzyl-1-[4-(4-méthylphényl)-1H-imidazol-2-yl]-1-heptanamine

Chlorhydrate. Point de fusion : 185-188 °C.

### Exemple 201: (R,S)-N-benzyl-1-[4-(2-méthoxyphényl)-1H-imidazol-2-y1]-1-heptanamine

Base libre. Point de fusion : 155-157 °C.

### Exemple 202: (R,S)-N-benzyl-1-(4-phényl-1H-imidazol-2-yl)-1-hexanamine

Base libre. Point de fusion : 192-194 °C.

### Exemple 203: 4-[2-(2-{[(néopentyloxy)carbonyl]amino}éthyl)-1H-imidazol-4-yl]-1,1'-biphényle

Base libre. Point de fusion : 162-164 °C.

### Exemple 204: (1S)-N-benzyl-1-(4,5-diphényl-1H-imidazol-2-yl)-1-propanamine

Base libre. Point de fusion : 182-184 °C.

### Exemple 205: (R,S)-4-[2-(1-aminoheptyl)-1H-imidazol-4-yl]benzonitrile

Chlorhydrate. Point de fusion : 218-220 °C.

### Exemple 206: (R,S)-1-[4-(4-bromophényl)-1H-imidazol-2-yl]-1-heptanamine

Base libre. Point de fusion : à partir de 126 °C.

### Exemple 207: (1R)-1-(4-phényl-1H-imidazol-2-yl)butylcarbamate de tert-butyle

Base libre. Point de fusion : 156-158 °C.

### Exemple 208: 4-(2-{(1R)-1-[(tert-butoxycarbonyl)amino]butyl}-1H-imidazol-4-yl)-1,1'-biphényle

Base libre. Point de fusion : 145,6 °C.

### Exemple 209: (1R)-1-(4-[1,1'-biphényl]-4-yl-1H-imidazol-2-yl)-1-butanamine

Chlorhydrate. Point de fusion : 155,4 °C.

### Exemple 210: (R,S)-4-[2-(1-aminoheptyl)-1H-imidazol-4-yl]-2,6-di(tert-butyl)-phénol

Chlorhydrate. Point de fusion : 204-206 °C.

### Exemple 211: (1R)-1-(4-phényl-1H-imidazol-2-yl)-1-butanamine

Chlorhydrate. Point de fusion : 182-184 °C.

### Exemple 212: (R,S)-N-benzyl-1-[4-(4-bromophényl)-1H-imidazol-2-yl]-1-heptanamine

Base libre. Point de fusion : devient collant à partir de 130 °C.

### Exemple 213: (1R)-N-benzyl-1-(4-[1,1'-biphényl]-4-yl-1H-imidazol-2-yl)-1-butanamine

Base libre. Point de fusion : 78,6 °C.

### Exemple 214: (1R)-N-benzyl-1-(4-phényl-1H-imidazol-2-yl)-1-butanamine

Base libre. Point de fusion : 218-220 °C.

### Exemple 215: (R,S)-N-(3-chlorobenzyl)-1-(4-phényl-1H-imidazol-2-yl)-1-heptanamine

Base libre. Le point de fusion n'a pu être mesuré (colle).

### Exemple 216: (R,S)-N-benzyl-1-[4-(3-méthoxyphényl)-1H-imidazol-2-yl]-1-heptanamine

Base libre. Point de fusion : 141-142 °C.

### Exemple 217: (R,S)-4-{2-[1-(benzylamino)heptyl]-1H-imidazol-4-yl}benzonitrile

Base libre. Point de fusion : 188-189 °C.

### Exemple 218: (R,S)-4-[2-(1-aminoheptyl)-1H-imidazol-4-yl]-N,N-diéthylaniline

Chlorhydrate. Point de fusion : 192 °C.

### Exemple 219: (1R)-1-(4-phényl-1H-imidazol-2-yl)éthanamine

Chlorhydrate. Point de fusion : 178-181 °C.

### Exemple 220: (R,S)-1-[4-(4-fluorophényl)-1H-imidazol-2-yl]-1-heptanamine

Chlorhydrate. Point de fusion : 148-150 °C.

### Exemple 221: (R,S)-1-[4-(2-chlorophényl)-1H-imidazol-2-yl]-1-heptanamine

Chlorhydrate. Point de fusion : 138-140 °C.

### Exemple 222: N-[(1S)-1-(4-[1,1'-biphényl]-4-yl-1H-imidazol-2-yl)propyl]-1-butanamine

Base libre. Le point de fusion n'a pu être mesuré (colle).

### Exemple 223: (1R)-N-benzyl-1-(4-phényl-1H-imidazol-2-yl)éthanamine

Base libre. Le point de fusion n'a pu être mesuré (colle).

### Exemple 224: (RS)-N-[1-(4-phényl-1H-imidazol-2-yl)heptyl]-N-propylamine

Base libre. Point de fusion : 94-98 °C.

### Exemple 225: (R,S)-N benzyl-1-[4-(3-méthoxyphényl)-1H-imidazol-2-yl]-1-heptanamine

Chlorhydrate. Point de fusion : à partir de 120 °C.

### Exemple 226: (R,S)-4-{2-[1-(benzylamino)heptyl]-1H-imidazol-4-yl}benzonitrile

Chlorhydrate. Point de fusion : à partir de 185 °C.

### Exemple 227: (R,S)-N-(4-méthoxybenzyl)-1-(4-phény-1-imidazol-2-yl)-1-heptanamine

Base libre. Point de fusion : 126-128 °C.

### Exemple 228: (R,S)-N-benzyl-1-[4-(4-fluorophényl)-1H-imidazol-2-yl]-1-heptanamine

Chlorhydrate. Point de fusion : à partir de 110 °C.

### Exemple 229: (R,S)-N-benzyl-1-[4-(2-chlorophényl)-1H-imidazol-2-yl]-1-heptanamine

Chlorhydrate. Point de fusion : à partir de 90 °C.

### Exemple 230: (R,S)-N-benzyl-N-(1-{4-[4-(diéthylamino)phényl]-1H-imidazol-2-yl}heptyl)amine

Chlorhydrate. Point de fusion : 170 °C.

### Exemple 231: (R,S)-1-[4-(3,4-dichlorophényl)-1H-imidazol-2-yl]-1-heptanamine

Chlorhydrate. Point de fusion : 148-150 °C.

### Exemple 232: (R,S)-1-[4-(3-bromopbényl)-1H-imidazol-2-yl]-5-méthylhexylcarbamate de tert-butyle

Base libre. Point de fusion : 134-136 °C.

### Exemple 233: (R,S)-1-[4-(3-bromophényl)-1H-imidazol-2-yl]-5-méthyl-1-hexanamine

Chlorhydrate. Point de fusion : 200-202 °C.

### Exemple 234: (R,S)-N-isobutyl-1-(4-phényl-1H-imidazol-2-yl)-1-heptanamine

Acétate. Point de fusion : 70-72 °C.

### Exemple 235: (R,S)-N-benzyl-1-[4-(3-bromopbényl)-1H-imidazol-2-yl]-5-méthyl-1-hexanamine

Base libre. Point de fusion : 92-94 °C.

### Exemple 236: (R,S)-N-benzyl-1-[4-(4-méthoxyphényl)-1H-imidazol-2-yl]-1-heptanamine

Base libre. Huile.

### Exemple 237: 4-[2-(2-{[(benzyloxy)carbonyl]amino}éthyl)-1H-imidazol-4-yl]-1,1'-biphényle

Base libre. Point de fusion : 134-136 °C.

### Exemple 238: 4-(2-{1-[(butoxycarbonyl)amino]-1-méthyléthyl}-1H-imidazol-4-yl)1,1'-biphényle

Base libre. Point de fusion : 170-172 °C.

### Exemple 239: 4-(2-{2-[(isobutoxycarbonyl)amino]éthyl}-1H-imidazol-4-yl)-1,1'-biphényle

Base libre. Point de fusion : 134-135 °C.

### Exemple 240: (R,S)-N-[1-(4-phényl-1H-imidazol-2-yl)heptyl]cyclobutanamine

Base libre. Point de fusion : 148-150 °C.

### Exemple 241: 4-(2-{(1S)-1-[(butoxycarbonyl)amino]éthyl}-1H-imidazol-4-yl)-1,1'-biphényle

Base libre. Point de fusion : 118-122 °C.

### Exemple 242: 4-(2-{(1R)-1-[(butoxycarbonyl)amino]éthyl}-1H-imidazol-4-yl)-1,1'-biphényle

Base libre. Point de fusion : 114-116 °C.

### Exemple 243: N-[(S)-cyclohexyl(4-phényl-1H-imidazol-2-yl)méthyl]-cyclohexanamine

Base libre. Point de fusion : 240-242 °C.

### Exemple 244: 4-(2-{2-[(méthoxycarbonyl)amino]éthyl}-1H-imidazol-4-yl)-1,1'-biphényle

Base libre. Point de fusion: 177,2 °C.

### Exemple 245: 4-(2-{2-[(propoxycarbonyl)amino]éthyl}-1H-imidazol-4-yl)-1,1'-biphényle

Base libre. Point de fusion : 141,2 °C.

### Exemple 246: 4-(2-(2-[(éthoxycarbonyl)amino]éthyl)-1H-imidazol-4-yl)-1,1'-biphényle

Base libre. Point de fusion : 132,5 °C.

### Exemple 247: 4-[2-(1-{[(benzyloxy)carbonyl]amino}-1-méthyléthyl)-1H-imidazol-4-yl]-1,1'-biphényle

Base libre. Point de fusion : 148-152 °C.

### Exemple 248: (R,S)-N-isopropyl-N-[1-(4-phényl-1H-imidazol-2-yl)heptyl]amine

Base libre. Point de fusion : 114-116 °C.

### Exempte 249: N-[2-(4-[1,1'-biphényl]-4-yl-1H-imidazol-2-yl)éthyl]-cyclohexanamine

Base libre. Point de fusion : 207-210 °C.

### Exemple 250: (R,S)-N-{1-[4-(3,4-dichlorophényl)-1H-imidazol-2-yl]heptyl}-cyclohexanamine

Chlorhydrate. Point de fusion : 194 °C.

### Exemple 251: 2-[4-(4-fluorophényl)-1H-imidazol-2-yl]éthylcarbamate de butyle

Base libre. Point de fusion : 87°C.

### Exemple 252: (R,S)-N-[1-(4-[1,1'-biphényl]-4-yl-1H-imidazol-2-yl)heptyl]-cyclohexanamine

Chlorhydrate. Point de fusion : 168-170 °C.

### Exemple 253: (R,S)-2-(5-fluoro-1H-indol-3-yl)-1-[4-(4-fluorophényl)-1H-imidazol-2-yl]éthylamine

Chlorhydrate. Point de fusion : 220-222 °C.

### Exemple 254: N-{[4-(3-bromophényl)-1H-imidazol-2-yl]métbyl}cyclohexanamine

Base libre. Point de fusion : 202-204 °C.

### Exemple 255: 2-(4-[1,1'-biphényl]-4-yl-1H-imidazol-2-yl)éthylcarbamate d'hexyle

Base libre. Point de fusion : 116,5-116,8 °C.

### Exemple 256: (R,S)-{2-(5-fluoro-1H-indol-3-yl)-1-[4-(4-fluorophényl)-1H-imidazol-2-yl]éthyl}cyclobutanamine

Chlorhydrate. Point de fusion : 180-190 °C.

### Exemple 257: (R,S)-N-{1-[4-(4-fluorophényl)-1H-imidazol-2-yl]-4-méthylpentyl}-cyclohexanamine

Chlorhydrate. Point de fusion : 230-232 °C.

### Exemple 258: (S)-cyclohexyl[4-(3,4-difluorophényl)-1H-imidazol-2-yl]-méthanamine

Chlorhydrate. Point de fusion : 222-223 °C.

### Exemple 259: (S)-cyclohexyl[4-(3-fluoro-4-méthoxyphényl)-1H-imidazol-2-yl]-méthanamine

Chlorhydrate. Point de fusion : 225-227 °C.

### Exemple 260: (R,S)-cyclopropyl[4-(4-fluorophényl)-1H-imidazol-2-yl]-méthanamine

Chlorhydrate. Point de fusion : 230-232 °C.

### Exemple 261: N-{(S)-cyclohexyl[4-(4-fluorophényl)-1H-imidazol-2-yl]méthyl}-2-propanamine

Base libre. Point de fusion : 210-212 °C.

### Exemple 262: N-{(S)-cyclobexyl[4-(3,4-difluorophényl)-1H-imidazol-2-yl]méthyl}cyclobutanamine

Base libre. Point de fusion : 200-202 °C.

### Exemple 263: (R,S) N-(cyclobexylméthyl)-1-(4-phényl-1H-imidazol-2-yl)-1-heptanamine

Chlorhydrate. Point de fusion : 142-144 °C.

### Exemple 264: N-{(S)-cyclohexyl[4-(4-fluorophényl)-1H-imidazol-2-yl]méthyl}cyclohexanamine

Chlorhydrate. Point de fusion : > 250 °C.

### Exemple 265: (S)-cyclohexyl-N-(cyclohexylméthyl)(4-phényl-1H-imidazol-2-yl)méthanamine

Chlorhydrate. Point de fusion : 180-182 °C.

### Exemple 266: (R,S)-N-{cyclopropyl[4-(4-fluorophényl)-1H-imidazol-2-yl]méthyl}cyclohexanamine

Chlorhydrate. Le point de fusion n'a pu être mesuré (colle).

### Exemple 267: (S)-cyclohexyl-N-(cyclopropylméthyl)(4-phényl-1H-imidazol-2-yl)méthanamine

Chlorhydrate. Point de fusion : 151-152 °C.

### Exemple 268: 2-[4-(4-cyclohexylphényl)-1H-imidazol-2-yl]éthylcarbamate de butyle

Base libre. Point de fusion : 138,4 °C.

### Exemple 269: 4-[2-(2-{[(cyclohexyloxy)carbonyl]amino}éthyl)-1H-imidazol-4-yl]-1,1'-biphényle

Base libre. Point de fusion : 150 °C.

### Exemple 270: N-(S)-cyclobexyl{4-[4-(trifluorométhoxy)phényl]-1H-imidazol-2-yl} méthylpcyclobutanamine

Base libre. Point de fusion : 136-140 °C.

### Exemple 271: 4-[2-(2-{[(cyclopentyloxy)carbonyl]amino}éthyl)-1H-imidazol-4-yl]-1,1'-biphényle

Base libre. Point de fusion : 140,5 °C.

### Exemple 272: (R,S)-N-{1-[4-(3-bromophényl)-21H-imidazol-2-yl]-5-méthylhexyl}-cyclohexanamine

Chlorhydrate. Point de fusion : 216,7 °C.

### Exemple 273: (S)-cyclohexyl-N-(cyclopropylméthyl)[4-(4-fluorophényl)-1H-imidazol-2-yl]-méthanamine

Chlorhydrate. Point de fusion : 221,4 °C.

### Exemple 274: (R,S)-N-{cyclopentyl[4-(4-fluorophényl)-1H-imidazol-2-yl]méthyl}cyclobutanamine

Base libre. Point de fusion : 146-148 °C.

### Exemple 275: N-{(S)-cyclobexyl[4-(4-cyclobexylphényl)-1H-imidazol-2-yl]méthyl}-cyclobutanamine

Chlorhydrate. Point de fusion : 190-192 °C.

### Exemple 276: N-{(1R)-1-[4-(4-fluorophényl)-1H-imidazol-2-yl]-2-méthylpropyl}-cyclohexanamine

Base libre. Point de fusion : 224-226 °C.

### Exemple 277: N-((S)-cyclohexyl{4-[4-(trifluorométhyl)phényl]-1H-imidazol-2-yl}méthyl)cyclobutanamine

Acétate. Point de fusion : à partir de 130 °C.

### Exemple 278: 2-[4-(2,3-dihydro-1,4-benzodioxin-6-yl)-1H-imidazol-2-yl]éthylcarbamate de butyle

Base libre. Gomme.

### Exemple 279: N-{(S)-cyclobexyl[4-(4-fluorophényl)-1-méthyl-1H-imidazol-2-yl]méthyl}cyclohexanamine

Chlorhydrate. Point de fusion : 190-194 °C.

### Exemple 280: 2-(4-[1,1'-biphényl]-4-yl-1H-imidazol-2-yl)éthylcarbamate de cyclohexylméthyle

Base libre. Point de fusion : 132-134 °C.

### Exemple 281: 4-bromo-4'-(2-(2-[(butoxycarbonyl)amino]éthyl)-1H-imidazol-4-yl)-1,1'-biphényle

Base libre. Point de fusion : 166 °C.

### Exemple 282: N-((S)-cyclohexyl{4-méthylthiophényl]-1H-imidazol-2-yl}méthyl)cyclohexanamine

Base libre. Point de fusion : 96-98 °C.

### Exemple 283: N-{(S)-cyclohexyl[4-(4-fluorophényl)-1H-imidazol-2-yl]méthyl}-cyclohexanamine

Base libre. Point de fusion : 260-262 °C.

### Exemple 284: N-[(S)-{4-[3,5-bis(trifluorométhyl)phényl]-1H-imidazol-2-yl}(cyclohexyl)méthyl]cyclohexanamine

Base libre. Point de fusion : 180-182 °C.

### Exemple 285: 2-(4-[1,1'-biphényl]-4-yl-1H-imidazol-2-yl)éthylcarbamate de cyclobutylméthyle

Base libre. Point de fusion : 144-145 °C.

### Exemple 286: 2-[4-(4-fluorophényl)-1H-imidazol-2-yl]éthylcarbamate de cyclobutylméthyle

Base libre. Point de fusion : 149-150 °C.

### Exemple 287: N-{(S)-cyclohexyl[4-(3,4-difluorophényl)-1H-imidazol-2-yl]méthyl}cyclohexanamine

Base libre. Point de fusion : 182,3 °C.

### Exemple 288: 4-[2-(2-([(2-méthoxyéthoxy)carbonyl]amino)éthyl)-1H-imidazol-4-yl]-1,1'-biphényle

Base libre. Point de fusion : 123,3 °C.

### Exemple 289: (S)-1-[4-(3-bromopbényl)-1H-imidazol-2-yl]-1-cyclohexyl-N-(cyclohexylméthyl)méthanamine

Base libre. Point de fusion : 134,3 °C.

### Exemple 290: 4-(2-{(S)-cyclohexyl[(cyclohexylméthyl)amino]méthyl}-1H-imidazol-4-yl)N,N-diéthylaniline

Chlorhydrate. Point de fusion : 204-206 °C.

### Exemple 291: 2,6-ditert-butyl-4-(2-{(S)-cyclohexyl[(cyclobexylméthyl)amino]-méthyl}-1H-imidazol-4-yl)phénol

Chlorhydrate. Point de fusion : 254,6 °C.

### Exemple 292: 4-{2-[(S)-cyclohexyl(cyclohexylamino)méthyl]-1H-imidazol-4-yl}-N,N-diéthylaniline

Chlorhydrate. Point de fusion: 204-210 °C.

### Exemple 293: (S)-1-cyclohexyl-N-(cyclohexylméthyl)-1-[4-(4-fluorophényl)-1H-imidazol-2-yl]méthanamine

Base libre. Point de fusion : 184,8 °C.

### Exemple 294: 2-[4-(4-tert-butylphényl)-1H-imidazol-2-yl]éthylcarbamate de butyle

Base libre. Point de fusion : 106-108 °C.

### Exemple 295: (S)-1-cyclohexyl-N-(cyclohexylméthyl)-1-[4-(4-fluorophényl)-1H-imidazol-2-yl]méthanamine

Chlorhydrate. Point de fusion : 190-192 °C.

### Exemple 296: N-((S)-cyclohexyl{4-[4-(trifluorométhyl)phényl]-1H-imidazol-2-yl}méthyl)cyclohexanamine

Chlorhydrate. Point de fusion : 214,1 °C.

### Exemple 297: N-[(S)-[4-(3-bromophényl)-1H-imidazol-2-yl](cyclohexyl)méthyl]-cyclohexanamine

Chlorhydrate. Point de fusion : 230,4 °C.

### Exemple 298: N-((S)-cyclohexyl{4-[4-(trifluorométhyl)phényl]-1H-imidazol-2-yl}méthyl)cyclohexanamine

Base libre.

### Exemple 299: 2-[4-(4-bromophényl)-1H-imidazol-2-yl]éthylcarbamate de butyle

Base libre. Point de fusion : 99-100 °C.

### Exemple 300: 2-{4-[4-(trifluorométhyl)phényl]-1H-imidazol-2-yl}éthylcarbamate de butyle

Base libre. Point de fusion : 104-105 °C.

### Exemple 301: N-{(S)-cyclohexyl[4-(4-fluorophényl)-1H-imidazol-2-yl]méthyl}cycloheptanamine

Base libre. Point de fusion : 140-142 °C.

### Exemple 302: 2-[4-(4-tert-butylphényl)-1H-imidazol-2-yl]éthylcarbamate de cyclohexylméthyle

Base libre. Point de fusion : 104-106 °C.

### Exemple 303: 2-[4-(4'-bromo-1,1'-biphényl-4-yl)-1H-imidazol-2-yl]éthylcarbamate de cyclohexylméthyle

Base libre. Point de fusion : 130-132 °C.

### Exemple 304: -N-((S)-cyclohexyl{4-[3-(trifluorométhyl)phényl]-1H-imidazol-2-yl}méthyl)cyclohexanamine

Base libre. Point de fusion : 186-188 °C.

### Exemple 305: (S)-1-cyclohexyl-N-(cyclohexylméthyl)-1-{4-[3-(trifluorométhyl)-phényl]-1H-imidazol-2-yl}méthanamine

Base libre. Point de fusion : 143,9 °C.

### Exemple 306: (S)-1-[4-(3-bromophényl)-1H-imidazol-2-yl]-1-cyclohexyl-N-(cyclohexylméthyl)méthanamine

Chlorhydrate. Point de fusion : 206,3 °C.

### Exemple 307: (S)-1-cyclohexyl-N-(cyclohexylméthyl)-1-{4-[3-(trifluorométhyl)-phényl]-1H-imidazol-2-yl}méthanamine

Chlorhydrate. Point de fusion : 198-200 °C.

### Exemple 308: (1R)-2-cyclohexyl-1-[4-(4-fluorophényi)-1H-imidazol-2-yl]éthanamine

Chlorhydrate. Point de fusion : 148-149 °C.

### Exemple 309: N-{(1R)-2-cyclohexyl-1-[4-(4-fluorophényl)-1H-imidazol-2-yl]éthyl}cyclohexanamine

Base libre. Point de fusion : 217-218 °C.

### Exemple 310: 4-{2-[(S)-amino(cyclohexyl)méthyl]-1H-imidazol-4-yl}-N,N-diéthylaniline

Chlorhydrate. Point de fusion : 216-217 °C.

### Exemple 311: (S)-1-cyclohexyl-1-[4-(3-fluorophényl)-1H-imidazol-2-yl]méthanamine

Chlorhydrate. Point de fusion : 238-241 °C.

### Exemple 312: (S)-1-cyclohexyl-N-(cyclohexylméthyl)-1-[4-(3-fluorophényl)-1H-imidazol-2-yl]méthanamine

Chlorhydrate. Point de fusion : 180-186 °C.

### Exemple 313: 2-[4-(4-pyrrolidin-1-ylphényl)-1H-imidazol-2-yl]éthylcarbamate de butyle

Base libre. Point de fusion : 125 °C.

### Exemple 314: N-{(S)-cyclohexyl[4-(3-fluorophényl)-1H-imidazol-2-yl] méthyl}cyclohexanamine

Chlorhydrate. Point de fusion : 213,9 °C.

### Exemple 315: N-{(1R)-2-cyclohexyl-1-[4-(4-fluorophényol)-1H-imidazol-2-yl]éthyl}cyclohexanamine

Chlorhydrate. Point de fusion : se décompose à partir de 250 °C.

### Exemple 316: 4-{2-[(S)-amino(cyclohexyl)méthyl]-1-imidazol-4-yl}-2,6-ditert-butylphénol

Chlorhydrate. Point de fusion : 222-228 °C.

### Exemple 317: 2-[4-(4-pyrrolidin-1-ylphényl)-1H-imidazol-2-yl]éthylcarbamate de butyle

Chlorhydrate. Point de fusion : 165-166 °C.

### Exemple 318: (R)-1-cyclohexyl-N-(cyclohexylméthyl)-1-[4-(4-fluorophényl)-1H-imidazol-2-yl]méthanamine

Chlorhydrate. Point de fusion : 188,2 °C.

### Exemple 319: 2,6-ditert-butyl-4-[4-(hydroxyméthyl)-1,3-thiazol-2-yl]phénol

Le composé de l'exemple 319 peut être obtenu selon un protocole analogue à celui décrit pour le composé de l'exemple 38, étape E de la demande de brevet PCT WO 99/09829, excepté le fait que le bromopyruvate d'éthyle remplace le 3-chloroacétoacétate dans l'étape 38.C et que l'hydrure de disobutylaluminium remplace l'hydrure de lithiumaluminium dans l'étape 38.E.

Alternativement, ce composé peut aussi être obtenu selon la procédure décrite dans J. Med. Chem. (1996), 39, 237-245. Solide blanc. Point de fusion : 123-124 °C.

### Exemple 320: chlorhydrate de méta-[4-(2,3-dihydro-1H-indol-6-yl)-1,3-thiazol-2-yl]-N-méthylméthanamine

### 320.1) Mélange de méta-2-chloro-1-[1-(chloroacétyl)-2,3-dihydro-1H-indol-6-yl]éthanone et de para-2-chloro-1-[1-(chloroacétyl)-2,3-dihydro-1H-indol-6 yl]éthanone

Du 1-(chloroacétyl)-2,3-dihydro-1H-indole (3,9 g ; 20mmol) est dissous dans du disulfure de carbone (40 ml). AlCl₃ (6,15 g ; 46 mmol) est ajouté lentement puis le chlorure de chloroacétyle (1,835 ml ; 22 mmol) est additionné goutte à goutte au mélange qui est ensuite chauffé à reflux durant 18 heures. Après refroidissement du milieu réactionnel, le CS₂ est décanté et de l'eau glacée contenant du HCl concentré ajoutée. Après extraction avec du dichlorométhane, la phase organique est séparée et séchée sur sulfate de magnésium avant d'être filtrée et concentrée sous vide. Le produit attendu (un mélange 50/50 des isomères méta et para) est obtenu par purification par cristallisation dans de l'acide acétique glacial. Solide de couleur blanche (1,6 g ; rendement de 30%).
MH+= 271.

### 320.2) chlorhydrate de méta-2-chloro-1-(2,3-dihydro-1H-indol-6-yl)éthanone

L'intermédiaire 320.1 (mélange d'isomères ; 1,6 g ; 6,0 mmol) est dissous à chaud dans un mélange d'acide acétique (10 ml) et de HCl à 20% (2 ml). Le milieu réactionnel est porté à reflux durant 24 heures. Après évaporation puis purification par cristallisation du chlorhydrate dans l'acide acétique glacial pour séparer le mélange d'isomères, l'isomère méta cristallise sous forme de solide marron (l'isomère para reste dans les eaux mères) avec un rendement de 47%. Point de fusion : décomposition à partir de 158 °C.
MH+ = 196.
La structure méta du composé a été établie par RMN/NOESY.

### 320.3) chlorhydrate de méta-[4-(2,3-dihydro-1H-indol-6-yl)-1,3-thiazol-2-yl]-N-méthylméthanamine

Le protocole expérimental utilisé est identique à celui décrit pour le composé 30.2 de l'exemple 30, l'intermédiaire 320.2 étant utilisé comme produit de départ à la place de l'intermédiaire 30.1, le tétrahydrofuranne remplaçant le toluène en présence d'un équivalent de triéthylamine pour libérer la base du sel. On obtient un solide de couleur marron avec un rendement de 9%. Point de fusion : décomposition à partir de 235 °C.
MH+ = 246.

### Exemple 321: chlorhydrate de 2,5,7,8-tétraméthyl-2-{2-[(méthylamino)méthyl]-1,3-thiazol-4-yl}-6-chromanol

### 321.1) 6-hydroxy-N-méthoxy-N,2,5,7,8-pentaméthyl-2-chromanecarboxamide

A une solution de 5,0 g (20,0 mmol) de l'acide (R,S) 6-hydroxy-2,5,7,8-tétraméthyl-2-chromanecarboxylique (Trolox^{®}) dans 175 ml de DMF, on ajoute successivement 2,2 g (22,0 mmol) de chlorhydrate de O,N-diméthylhydroxylamine, de la triéthylamine (6,2ml), 3,0 g (22,0 mmol) d'hydroxybenzotriazole et 4,2 g (22,0 mmol) de chlorhydrate de 1-(3-diméthylaminopropyl)-3-éthyl-carbodiimide. Après avoir agité le mélange réactionnel une nuit durant à 25 °C, on dilue l'ensemble avec de l'eau glacée et l'agitation est maintenue 30 minutes supplémentaires. Le produit est extrait à l'aide de 3 fois 100 ml d'acétate d'éthyle. La solution organique est lavée successivement avec une solution aqueuse de bicarbonate de sodium à 10%, à l'eau, avec une solution d'acide citrique aqueuse à 10% et finalement avec une solution de chlorure de sodium saturée. La phase organique est ensuite séchée sur sulfate de magnésium, filtrée et concentrée sous vide. Le produit obtenu est purifié par cristallisation dans de l'éther pour conduire à un solide de couleur blanche avec un rendement de 63%. Point de fusion : 139-140 °C.
MH+ = 294.

### 321.2) 1-(6-hydroxy-2,5,7,8-tétraméthyl-3,4-dihydro-2H-chromen-2 yl)éthanone

A une solution de 2,93 g (10,0 mmol) de l'intermédiaire 321.1 dans 100 ml de THF, on ajoute goutte à goutte à la température de -30 °C une solution de méthyllithium (1,6 M ; 31,25 ml ; 50,0 mmol) et on laisse le mélange sous agitation 1 heure à -10°C. Le milieu réactionnel est hydrolysé avec NH₄Cl en solution aqueuse saturée.Le produit est extrait à l'aide de 3 fois 150 ml d'acétate d'éthyle. La phase organique est finalement lavée avec du chlorure de sodium en solution aqueuse saturée avant d'être ensuite séchée sur sulfate de magnésium, filtrée et concentrée sous vide. Le produit obtenu est purifié par cristallisation dans de l'éther diisopropylique pour conduire à un solide blanc avec un rendement de 80,7%. Point de fusion : 97-98 °C.
MH+ = 248.

### 321.3) 2-bromo-1-(6 hydroxy-2,5,7,8-tétraméthyl 3,4-dihydro-2H-chromen-2-yl)éthanone

L'intermédiaire 321.2 (0,777 g ; 3,13 mmol) est dissous dans de l'éthanol (25 ml) sous courant d'argon. La solution est refroidie vers 0°C et du brome (0,18 ml; 4,20 mmol) est ajouté en une seule portion (voir J. Am. Chem. Soc. (1999), 121, 24), puis le mélange est agité une demi-heure en laissant la température revenir à température ambiante. L'excès de brome est éliminé par barbotage d'argon puis on laisse le mélange sous agitation durant 2 heures et demie. L'éthanol est évaporé et le produit obtenu est purifié par cristallisation dans le toluène. Après filtration et lavage avec de l'isopentane, on obtient un solide marron avec un rendement de 36%. Point de fusion : décomposition à partir de 125°C.
MH+ = 326.

### 321.4) chlorhydrate de 2,5,7,8-tétraméthyl2-{2-[(méthylamino)méthyl]-1,3-thiazol-4 yl}-6-chromano/

Le protocole expérimental utilisé est analogue à celui décrit pour le composé 30.2 de l'exemple 30, l'intermédiaire 321.3 étant utilisé comme produit de départ à la place de l'intermédiaire 30.1, et le benzène remplaçant le toluène comme solvant. Le produit obtenu est purifié par cristallisation dans un minimum de dichlorométhane pour conduire à un solide blanc avec un rendement de 48%. Point de fusion : 153-155 °C.

### Exemple 322: chlorhydrate de N-{[4-(9H-carbazol-2-yl)-1,3-thiazol-2-yl]méthyl}-N-méthylamine

### 322.1) 9-acétyl-9H-carbazole

Ce composé est obtenu selon Tetrahedron (1980), 36, 3017-3019. Le carbazole (10 g; 60 mmol) est mis en suspension dans 150 ml d'anhydride acétique. De l'acide perchlorique à 70% (0,5 ml) est ajouté. Après une demi-heure d'agitation à température ambiante, le mélange est versé sur de la glace et le précipité formé est filtré. Après séchage sous vide, redissolution dans du dichlorométhane et traitement au noir animal, la suspension est filtrée sur célite, les solvants sont évaporés et le produit recristallisé dans de l'heptane. On obtient ainsi 12 g de cristaux bruns (rendement de 90%). Point de fusion : 70-71 °C (littérature: 72-74 °C).

### 322.2) 1-(9-acétyl-9H-carbazol-2-yl)-2-chloroéthanone

Ce composé est obtenu selon un protocole analogue à celui de l'étape 320.1 de l'exemple 320, en utilisant 5 g (24 mmol) de l'intermédiaire 322.1. On obtient 5,4 g du composé attendu (rendement de 79%). Solide blanc. Point de fusion : 175-176 °C.

### 322.3) 1-(9H-carbazol-2-yl)-2-chloroéthanone

L'intermédiaire 322.2 (2,85 g;1 mmol) est mis en suspension dans un mélange d'acide acétique (50 ml) et de HCl concentré (5 ml). Le milieu réactionnel est porté 2 heures à reflux avant d'être laissé revenir à température ambiante. Le nouveau précipité formé est filtré. Après séchage sous vide, on obtient 1,9 g d'un solide verdâtre (rendement de 78%). Point de fusion : 203-204 °C.

### 322.4) chlorhydrate de N-{[4-(9H-carbazol-2-yl)-1,3-thiazol-2-yl]méthyl}-N-méthylamine

Ce composé est obtenu selon un protocole analogue à celui de l'étape 30.2 à partir de 487 mg (2 mmol) de l'intermédiaire 322.3 et de 408 mg (2 mmol) de 2-amino-5 2-thioxoéthyl(méthyl)carbamate de *tert*-butyle. On obtient 300 mg du produit attendu (rendement de 43%). Solide blanc. Point de fusion : > 250 °C.

### Exemple 323: chlorhydrate de 3,5-ditert-butyl-4'-{2-[(méthylamino)méthyl]-1,3-thiazol-4-yl}-1,1'-biphényl-4-ol

### 323.1) acide 3',5'-ditert-butyl-4'-hydroxy-1,1'-biphényl-4-carboxylique

5,0 g (1,41 mmol) de 3',5'-ditert-butyl-4'-hydroxy-1,1'-biphényl-4-carboxylate d'éthyle (Chem. Lett. (1998), 9, 931-932) sont dissous dans de l'éthanol (25 ml). La solution est refroidie à 0 °C puis on ajoute goutte à goutte une solution de soude 1*N*. Après agitation une nuit à température ambiante, le milieu réactionnel est porté à reflux pour achever la réaction. Après évaporation des solvants et dilution du résidu avec de l'eau, on acidifie le mélange obtenu avec une solution de HCl 1*N* et on procède à une extraction avec dichlorométhane. La phase organique est lavée avec du chlorure de sodium en solution aqueuse saturée avant d'être ensuite séchée sur sulfate de magnésium, filtrée et concentrée sous vide. Le produit obtenu est purifié par cristallisation dans de l'éther diisopropylique pour conduire à un solide blanc-jaunâtre avec un rendement de 47%. Point de fusion : >240 °C.

### 323.2) 3',5'-ditert-butyl-4'-hydroxy-N-méthoxy-N-méthyl-1,1'-biphényl-4-carboxamide

Le protocole expérimental utilisé est identique à celui décrit pour l'intermédiaire 321.1, l'acide 323.1 remplaçant le Trolox^{®} comme produit de départ. On obtient un solide jaunâtre avec un rendement de 93%. Point de fusion : 175,6-177 °C.

### 323.3) 1-(3;5-ditert-butyl-4'-hydroxy-1,1'-biphényl-4-yl)éthanone

Le protocole expérimental utilisé est identique à celui décrit pour l'intermédiaire 321.2, l'intermédiaire 323.2 remplaçant l'intermédiaire 321.1. On obtient un solide blanc avec un rendement de 74%. Point de fusion : 144-144,7 °C.

### 323.4) 2-bromo-1-(3',5'-ditert-butyl-4'-hydroxy-1,1'-biphényl-4-yl)éthanone

Le protocole expérimental utilisé est identique à celui décrit pour l'intermédiaire 321.3, l'intermédiaire 323.3 remplaçant l'intermédiaire 321.2. On obtient une huile jaune-orange suffisamment pure pour pouvoir être utilisée dans l'étape qui suit (rendement de 100%).

### 323.5) [4-(3;5'-ditert-butyl-4'-hydroxy-1,1'-biphényl-4yl)-1,3-thiazol-2-yl]méthyl(méthyl)carbamate de tert-butyle

Ce composé est préparé selon le protocole expérimental décrit dans l'exemple 1, étape 1.3, en utilisant l'intermédiaire 323.4 à la place de la bromo-1-(3,5-ditert-butyl 4-hydroxyphényl)éthanone. Le composé attendu est obtenu sous forme d'une huile incolore avec un rendement de 46%.
MH+ = 509,43.

### 323.6) Chlorhydrate de 3,5-ditert-butyl-4'-{2-[(méthylamino)méthyl]-1,3-thiazol-4-yl}-1,1'-biphényl-4-ol

0,230 g (0,452 mmol) de l'intermédiaire 323.5 sont dissous dans de l'acétate d'éthyle (20 ml). On fait barboter HCl gaz à travers la solution obtenue préalablement refroidie à 0°C. On laisse ensuite revenir à température ambiante le mélange agité. Le solide formé est filtré et lavé avec de l'acétate d'éthyle puis de l'éther avant d'être séché sous vide. Un solide blanc est obtenu avec un rendement de 85%. Point de fusion : 220-221 °C.

*Les composés des exemples 324 à 330 sont obtenus selon des procédures analogues à*
*celles décrites pour les exemples 31 à 46 ou ci dessus dans la partie intitulée « Préparation des composés de formule générale (I)* ».

### Exemple 324: (1R)-1-[4-(4-fluorophényl)-1H-imidazol-2-yl]-2-phényléthanamine

Chlorhydrate. Point de fusion : 173-180 °C.

### Exemple 325: 2-{4-[4-(diéthylamino)phényl]-1H-imidazol-2-yl}éthylcarbamate de cyclohexylméthyle

Chlorhydrate. Point de fusion: se décompose à partir de 168 °C.

### Exemple 326: 2-[4-(4-pyrrolidin-1-ylphényl)-1H-imidazol-2-yl]éthylcarbanlate de cyclohexylméthyle

Base libre. Point de fusion : 128,5 °C.

### Exemple 327: N-{(1R)-1-[4-(4-fluorophényl)-1H-imidazol-2-yl]-2-phényléthyl}cyclohexanamine

Chlorhydrate. Point de fusion : 210-213 °C.

### Exemple 328: (1R)-N-(cyclohexylméthyl)-1-[4-(4-fluorophényl)-1H-imidazol-2-yl]-2-phényléthanamine

Chlorhydrate. Point de fusion : à partir de 140 °C.

### Exemple 329: 2-[4-(3,5-ditert-butyl-4-hydroxyphényl)-1H-imidazol-2-yl]éthylcarbamate de cyclohexylméthyle

Chlorhydrate. Point de fusion : 111,5 °C.

### Exemple 330: 2-[4-(3,5-ditert-butyl-4-hydroxyphényl)-1H-imidazol-2-yl]éthylcarbamate de butyle

Base libre. Point de fusion : 180,9 °C.

### Exemple 331: chlorhydrate de 2,6-diméthoxy-4-{2-[(méthylamino)méthyl]-1,3-thiazol-4-yl}phénol

### 331.1) acétate de 4-acétyl-2,6-diméthoxyphényle

3,0 g (15,3 mmol) de 3,5-diméthoxy-4-hydroxyacétophénone sont dissous dans du dichlorométhane (30 ml) et 2,53 g (18,3 mmol) de K₂CO₃ sont ajoutés. De la triéthylamine (2,6 ml) est alors ajoutée goutte à goutte. Le milieu réactionnel est refroidi à 0°C et du chlorure d'acétyle (1,31 ml; 18,3 mmol) est ajouté. On laisse le mélange agiter 24 heures à température ambiante avant de le verser sur de l'eau glacée. Après extraction avec du dichlorométhane, la phase organique est lavée avec du chlorure de sodium en solution aqueuse saturée avant d'être séchée sur sulfate de magnésium, filtrée et concentrée sous vide. Le produit obtenu est purifié par cristallisation dans de l'éther pour conduire à un solide blanc avec un rendement de 99%. Point de fusion : 145 °C.

### 331.2) acétate de 4-(bromoacétyl)-2, 6-diméthoxyphényle

L'intermédiaire 331.1 (0,850 g ; 3,57 mmol) est solubilisé dans de l'acétate d'éthyle puis on ajoute 1,35 g (6,07 mmol) de CuBr₂ préalablement séché. Le mélange est porté à reflux pendant 2 heures et demie avant d'être laissé revenir à température ambiante. Du noir végétal est ajouté et le mélange agité pendant 10 minutes. Après filtration et évaporation à sec, le solide obtenu est repris dans de l'éther diisopropylique. Après filtration, on obtient un solide gris avec un rendement de 75%. Point de fusion : 124,2-126,3 °C.

### 331.3) acétate de 4-(2-{[(tert-butoxycarbonyl)(méthyl)amino]méthyl}-1,3-thiazol-4-yl)-2, 6-diméthoxyphényle

L'intermédiaire 331.3 est préparé selon un protocole expérimental décrit dans l'exemple 1, étape 1.3, en utilisant l'intermédiaire 331.2 à la place de la bromo-1-(3,5-ditert-butyl-4-hydroxyphényl)éthanone. Le composé attendu est obtenu sous forme d'un solide blanc avec un rendement de 55%. Point de fusion : 135,2-137,4 °C.

### 331.4) [4-(4-hydroxy-3,5-diméthoxyphényl)-1,3-thiazol-2-yl]méthyl(méthyl)carbamate de tert-butyle

0,530 g (1,25 mmol) de l'intermédiaire 331.3 sont dissous dans du méthanol (20 ml). La solution est refroidie à l'aide d'un bain de glace puis une solution de NaOH 1*N* est ajoutée goutte à goutte. On laisse le mélange revenir à température ambiante tout en l'agitant. Après évaporation à sec et dilution à l'eau du résidu, la solution est neutralisée à l'aide d'acide citrique et extraite avec du dichlorométhane. La phase organique est lavée avec du chlorure de sodium en solution aqueuse saturée avant d'être séchée sur sulfate de magnésium, filtrée et concentrée sous vide. Le produit est obtenu sous forme d'une huile jaune avec un rendement de 96%.
MH+= 381,20.

### 331.5) chlorhydrate de 2,6-diméthoxy-4-{2-[(méthylamino)méthyl]-1,3-thiazol-4-yl}phénol

Le protocole expérimental utilisé est identique à celui décrit pour l'intermédiaire 323.6, l'intermédiaire 331.4 remplaçant l'intermédiaire 323.5. On obtient un solide beige clair avec un rendement de 97%. Point de fusion : 229,8-232,0 °C.

### Exemple 332 : chlorhydrate de 2,6-diisopropyl-4-{2-[(méthylamino)méthyl]-1,3-thiazol-4-yl}phénol

### 332.1) acétate de 2,6-diisopropylphényle

On ajoute 3,45 g (16,4 mmol) d'anhydride trifluoroacétique à 0,83 ml (14,6 mmol) d'acide acétique à 0 °C en laissant le mélange revenir à température ambiante pendant 2 heures. Le mélange est alors refroidi à 0°C et 1,95g (11,0 mmol) de 2,6-diisopropylphénol est ajouté goutte à goutte. Le milieu réactionnel est mainten sous agitation pendant 12 heures avant d'être versé sur de l'eau glacée. Après extraction avec du dichlorométhane, la phase organique est lavée avec du chlorure de sodium en solution aqueuse saturée avant d'être séchée sur sulfate de magnésium, filtrée et concentrée sous vide. On obtient une huile incolore avec un rendement de 86%. Ce produit est suffisamment pur pour pouvoir être utilisé directement dans l'étape suivante

### 332.2) acétate de 1-(4-hydroxy-3,5-diisopropylphényl)éthanone

1,94 g (14,53 mmol) de AlCl₃ sont dissous dans du nitrobenzène (5 ml). Parallèlement 2,0 g (9,08 mmol) de l'intermédiaire 332.1 sont dissous dans du nitrobenzène (1 ml). La solution de l'intermédiaire 332.1 est ajoutée goutte à goutte à la solution de AlCl₃ à température ambiante. Le mélange est porté à 50 °C pendant 48 heures avant d'être laissé revenir à température ambiante. Le milieu réactionnel est alors versé sur de l'eau glacée. Une solution 1*N* de HCl (5 ml) et ensuite une solution concentrée de HCl (2 ml) sont ajoutées. On laisse le mélange agité à température ambiante puis on extrait avec du dichlorométhane. La phase organique est lavée avec du chlorure de sodium en solution aqueuse saturée avant d'être séchée sur sulfate de magnésium, filtrée et concentrée sous vide. Le produit attendu est obtenu après chromatographie sur une colonne de silice (éluant : 13% d'acétate d'éthyle dans de l'heptane). Après évaporation, les fractions pures donnent un solide blanc-gris avec un rendement de 25%. Point de fusion : 88-93 °C.

### 332.3) acétate de 4-acétyl-2,6-diisopropylphényle

Le protocole expérimental utilisé est identique à celui décrit pour l'intermédiaire 331.1-l'intermédiaire 332.2 remplaçant la 3,5-diméthoxy-4-hydroxyacétophénone. On obtient un solide de couleur sable avec un rendement de 95%. Point de fusion : 102-103 °C.

### 332.4) acétate de 4-(bromoacétyl)-2,6-diisopropylphényle

Le protocole expérimental utilisé est identique à celui décrit pour l'intermédiaire 331.2, l'intermédiaire 332.3 remplaçant l'intermédiaire 331.1. On obtient une huile jaune qui cristallise lentement avec un rendement de 88%. Ce produit est suffisamment pur pour pouvoir être utilisé directement dans l'étape suivante.

### 332.5) acétate de 4-(2-{[(tert-butoxycarbonyl)(méthyl)amino]méthyl}-1,3-thiazol-4-yl)-2,6-diisopropylphényle

L'intermédiaire 332.5 est préparé selon un protocole identique à celui décrit pour l'exemple 1, étape 1.3, en utilisant l'intermédiaire 332.4 à la place de la bromo-1-(3,5-ditert-butyl-4-hydroxyphényl)éthanone. Le composé attendu est obtenu sous forme d'un solide jaune pâle avec un rendement de 76%.
MH+ = 447,20.

### 332.6) acétate de tert-butyl [4-(4-hydroxy-3,5-diisopropylphéllyl)-1,3-thiazol-2-yl]méthyl(méthyl)carbamate

Le protocole expérimental utilisé est identique à celui décrit pour l'intermédiaire 331.4, l'intermédiaire 332.5 remplaçant l'intermédiaire 331.3. On obtient une huile ocre avec un rendement de 91%. Ce produit est suffisamment pur pour pouvoir être utilisé directement dans l'étape suivante.
MH+ = 405,20.

### 332.7) chlorhydrate de 2,6-diisopropyl-4-{2-[(méthylamino)méthyl]-1,3-thiazol-4-yl}phénol

Le protocole expérimental utilisé est identique à celui décrit pour l'intermédiaire 323.6, l'intermédiaire 332.6 remplaçant l'intermédiaire 323.5. On obtient un solide beige-rosé avec un rendement de 69%. Point de fusion : se décolore à 162 °C et fond à 173-177 °C.

### Exemple 333: chlorhydrate de 4-{2-[(méthytamino)méthyl]-1,3-thiazol-4-yl}phénol

### 333.1) 2-bromo-1-(4-hydroxyphényl)éthanone

Le protocole expérimental utilisé est identique à celui décrit pour l'intermédiaire 331.2, la 4-hydroxy-acétophénone remplaçant l'intermédiaire 331.1. On obtient un solide marron-rosé avec un rendement de 60%. Point de fusion : 118°C.

### 333.2) [4-(4-hydroxyphényl)-1,3-thiazol-2-yl]méthyl(méthyl)carbamate de tert-butyle

L'intermédiaire 333.2 est préparé selon un protocole identique à celui décrit pour l'exemple 1, étape 1.3, en utilisant l'intermédiaire 333.1 à la place de la bromo-1-(3,5-di*tert*-butyl-4-hydroxyphényl)éthanone et le toluène remplaçant le benzène. Le composé attendu est obtenu sous forme d'une huile jaune-incolore qui cristallise très lentement à froid avec un rendement de 35%.
MH+ = 321,30.

### 333.3) chlorhydrate de 4-{2-[(méthy/amino)méthyl]-1,3-thiazo/-4-yl}phénol

Le protocole expérimental utilisé est identique à celui décrit pour l'intermédiaire 323.6, l'intermédiaire 333.2 remplaçant l'intermédiaire 323.5. On obtient un solide jaune pâle avec un rendement de 100%. Point de fusion : 258-260 °C.

### Exemple 334: 2,6-ditert-butyl-4-[2-(hydroxyméthyl)-1,3-thiazol-4-yl]phénol

[*il s agit de l'intermédiaire 6.d,) de la demande de brevet* EP 432 740]

### 334.1) pivalate de [4-(3,5-ditert-butyl-4-hydroxyphényl)-1,3-thiazol 2 yl]méthyle

L'intermédiaire 334.1 est préparé selon un protocole identique à celui décrit pour l'exemple 1, étape 1.3, en utilisant le 2-(*tert*-butylcarbonyloxy)thioacétamide à la place du 2-{[(1,1-diméthyléthoxy)carbonyl]méthyl}amino-éthanethioamide et le toluène remplaçant le benzène. Le composé attendu est obtenu sous forme d'un solide blanc avec un rendement de 100%. Point de fusion : 114,6-116,0 °C.

### 334.2) 2,6-ditert-butyl-4-[2-(hydroxyméthyl)-1,3-thiazol-4-yl]phénol

Le protocole expérimental utilisé est identique à celui décrit pour l'intermédiaire 331.4, l'intermédiaire 334.1 remplaçant l'intermédiaire 331.3. On obtient un solide blanc avec un rendement de 88%. Point de fusion : 126,4-127,4 °C.

### Exemple 335: chlorhydrate de N-{[4-(4-anilinophényl)-1,3-thiazol-2-yl]méthyl}-N-méthylamine

### 335.1) 1-(4-anilinophényl)éthanone

De la 4-amino-acétophénone (4,87g; 36,0 mmol) est dissoute dans du diméthylformamide (75 ml). On ajoute 15 g (0,108 mol) de carbonate de potassium (préalablement séché à 170 °C sous atmosphère d'argon), 7,236 g (36,0 mmol) d'iodobenzène, 0,4 g de cuivre en poudre et une quantité catalytique d'iodure de cuivre. Le mélange réactionnel est porté à reflux pendant 12 heures. Après avoir laissé le milieu réactionnel revenir à température ambiante, on filtre celui-ci sur célite et le verse sur de l'eau glacée. Après extraction avec de l'acétate d'éthyle, la phase organique est lavée avec de l'eau avant d'être séchée sur sulfate de magnésium, filtrée et concentrée sous vide. Le produit obtenu est purifié par cristallisation dans de l'heptane pour conduire à un solide jaune avec un rendement de 53,4%. Point de fusion : 105 °C.

### 335.2) N-(4-acétylphényl)-N-phénylacétamide

Le protocole expérimental utilisé est identique à celui décrit pour l'intermédiaire 322.1, l'intermédiaire 335.1 remplaçant le 9-acétyl-9H-carbazole et le milieu réactionnel étant toutefois chauffé pendant 15 minutes à 70 °C. Après cristallisation dans de l'heptane, on obtient un solide jaune avec un rendement de 54,2%. Point de fusion : 118-120 °C (valeur dans la littérature : 122-123 °C).

### 335.3) N-[4-(bromoacétyl)phényl]-N-phénylacétamide

L'intermédiaire 335.2 (0,633 g ; 2,5 mmol) est dissous dans du méthanol (20 ml) et on ajoute 1 g (2,0 mmol) de résine de bromation PVPHP (J. Macromol. Sci. Chem. (1977), A11, (3), 507-514). Après agitation sous atmosphère d'argon pendant 4 heures, on filtre et rince les résines au méthanol. Après évaporation des solvants du filtrat et cristallisation dans du méthanol, on obtient un solide blanc avec un rendement de 59%. Point de fusion : 152-153 °C.

### 335.4) (4-{4-[acétyl(phényl)amino]phényl}-1,3-thiazol-2-yl)méthyl(méthyl)carbamate de tert-butyle

L'intermédiaire 335.4 est préparé selon un protocole identique à celui décrit pour l'exemple 1, étape 1.3, en utilisant l'intermédiaire 335.3 à la place de la bromo-1-(3,5-ditert-butyl-4-hydroxyphényl)éthanone et le toluène remplaçant le benzène. Le composé attendu est obtenu sous forme d'une huile avec un rendement de 73%.
MH+ = 438,30.

### 335.5) chlorhydrate de N-(4-{2-[(méthylamino)méthyl]-1,3-thiazol-4-yl}phényl)-N phénylacétamide

Le protocole expérimental utilisé est identique à celui décrit pour l'intermédiaire 322.3, l'intermédiaire 335.4 remplaçant l'intermédiaire 322.2. On obtient un solide blanc-crème avec un rendement de 53%. Point de fusion : > 250 °C.

### 335.6) chlorhydrate de N-{[4-(4-anilinophényl)-1,3-thiazol-2 yl]méthyl}-N-méthylamine

Le protocole expérimental utilisé est identique à celui décrit pour l'intermédiaire 322.3, l'intermédiaire 335.5 remplaçant l'intermédiaire 322.2 et le milieu réactionnel étant chauffé à reflux pendant 12 heures au lieu de 2 heures. On obtient un solide gris avec un rendement de 68%. Point de fusion : > 250 °C.

### Exemple 336 : chlorhydrate de 2,6-ditert butyl-4-{2-[(diméthylamino)méthyl]-1,3-thiazol-4-yl}phénol

### 336.1) 4-[2-(bromométhyl)-1,3-thiazol-4-yl]-2,6-ditert-butylphénol

On dissout 1,5 g (4,70 mmol) de l'intermédiaire 334.2, le (2,6-ditert-butyl-4-[2-(hydroxyméthyl)-1,3-thiazol-4-yl]phénol, dans du dichlorométhane (30 ml). Après ajout de CBr₄ (2,02 g ; 6,10 mmol), on refroidit le milieu réactionnel à 0 °C. PPh₃ (1,48 g ; 5,63 mmol) est ajouté par fractions puis le mélange est laissé revenir à température ambiante. Le milieu réactionnel est alors versé sur de l'eau glacée avant d'être extrait au dichlorométhane. La phase organique est lavée à l'eau salée avant d'être séchée sur sulfate de magnésium, filtrée et concentrée sous vide. Le produit attendu est obtenu après chromatographie sur une colonne de silice (éluant : 30% d'acétate d'éthyle dans de l'heptane), pour conduire à une huile marron avec un rendement de 92%. Ce produit est suffisamment pur pour pouvoir être utilisé directement dans l'étape suivante.
MH+ = 382,20.

### 336.2) chlorhydrate de 2,6-ditert-butyl-4-{2-[(diméthylamino)méthyl]-1,3-thiazol-4-yl}phénol

0,8 ml (1,57 mmol) de diméthylamine et 0,4 ml (2,62 mmol) de triéthylamine sont dissous dans du diméthylformamide (15 ml). 0,400 g (1,05 mmol) de l'intermédiaire 336.1 dissous dans du diméthylformamide (5 ml) sont ajoutés puis on laisse le mélange agité à température ambiante pendant 18 heures. Le milieu réactionnel est ensuite versé sur de l'eau glacée et extrait avec de l'acétate d'éthyle. La phase organique est lavée à l'eau salée avant d'être séchée sur sulfate de magnésium, filtrée et concentrée sous vide. Le produit attendu est obtenu après chromatographie sur une colonne de silice (éluant : 50% d'acétate d'éthyle dans de l'heptane), pour conduire à une huile orange avec un rendement de 92%. Le chlorhydrate est alors obtenu en solubilisant la base dans de l'éther et en ajoutant 1,2 ml d'une solution 1*N* de HCl dans de l'éther. Après filtration et lavage du solide formé avec de l'éther puis de l'isopentane, on obtient un solide beige-rosé avec un rendement de 15,2%. Point de fusion : 166,8-169,0 °C.

*Les composés des exemples 337 à 345 sont obtenus selon des procédures analogues à celles décrites pour les exemples 31 à 46 ou ci-dessus dans la partie intitulée « Préparation des composés de formule générale (I) »*.

### Exemple 337 : 2-[4-(4'-bromo-1,1'-biphényl-4-yl)-1H-imidazol-2-yl]éthylcarbamate de cyclobutylméthyle

Chlorhydrate. Point de fusion : 214-215 °C.

### Exemple 338: 2-[4-(4'-bromo-1,1'-biphényl-4-yl)-1H-imidazol-2-yl]éthylcarbamate d'isobutyle

Base libre. Point de fusion : 158,7 °C.

### Exemple 339 : 2-[4-(4-tert butylphényl)-1H-imidazol-2-yl]éthylcarbamate d'isobutyle

Base libre. Point de fusion : 110,6 °C.

### Exemple 340: 2-[4-(4-tert-butylphényl)-1H-imidazol-2-yl]éthylcarbamate cyclobutylméthyle

Base libre. Point de fusion : 103 °C.

### Exemple 341: 2-[4-(4'-bromo-1,1'-biphényl-4-yl)-1H-imidazol-2-yl]éthylcarbamate de cyclohexyle

Base libre. Point de fusion : 180 °C.

### Exemple 342: 2-[4-(4-tert-butylphényl)-1H-imidazol-2-yl]éthylcarbamate de cyclobexyle

Base libre. Point de fusion : 127-130 °C.

### Exemple 343: 3-[4-(4-fluorophényl)-1H-imidazol-2-yl]propan-1-amine

Chlorhydrate. Point de fusion : 245-246 °C.

### Exemple 344: 2-[4-(4'-bromo-1,1'-biphényl-4-yl)-1H-imidazol-2-yl]éthylcarbamate de 4,4,4-trifluorobutyle

Base libre. Point de fusion : 176,5 °C.

### Exemple 345 : 2-[4-(1,1'-biphényl-4-yl)-1H-imidazol-2-yl]éthylcarbamate de 4,4,4-trifluorobutyle

### Base libre. Point de fusion : 157,3 °C.

### Exemple 346: chlorhydrate de 2,6-ditert-butyl-4-{4-[(méthylamino)méthyl]-1,3-thiazol-2-yl}phénol

### 346.1) 4-[4-(bromométhy/)-1,3-thiazol-2-yl]-2,6-ditert-butylphénol

Le protocole expérimental utilisé est identique à celui décrit pour l'intermédiaire 336.1, le composé de l'exemple 319 remplaçant l'intermédiaire 334.2, le 1,2-dichloroéthane remplaçant le diméthylformamide et le milieu réactionnel étant porté à reflux durant 12 heures. On obtient une huile rougeâtre avec un rendement de 77%. Ce produit est utilisé tel quel directement dans l'étape suivante.

### 346.2) 2,6-ditert-butyl-4{4-[(méthylamino)méthyl]-1,3-thiazol-2-yl}phénol

Le protocole expérimental utilisé est identique à celui décrit pour l'intermédiaire 336.2, l'intermédiaire 346.1 remplaçant l'intermédiaire 336.1, une solution 2*N* de méthylamine dans du tétrahydrofuranne remplaçant la diméthylamine et l'acétonitrile remplaçant le diméthylformamide. Le chlorhydrate est obtenu en solubilisant la base dans de l'éther et en lui ajoutant une solution 1*N* de HCl dans de l'éther. Le solide formé est filtré et purifié par recristallisation dans de l'acétone pour conduire à un solide blanc avec un rendement de 18%. Point de fusion : 184,0-185,0 °C.

### Exemple 347: chlorhydrate de 2,6-ditert-butyl-4-[2-(pipéridin-1-ylméthyl)-1,3-thiazol-4-yl]phénol

Le protocole expérimental utilisé est identique à celui décrit pour l'intermédiaire 336.2, la pipéridine remplaçant la diméthylamine. On obtient un solide blanc avec un rendement de 56%. Point de fusion : > 195 °C.

### Exemple 348 : chlorhydrate de 2,6-ditert-butyl-4-{2-[(4-méthylpipérazin-1-yl)méthyl]-1,3-thiazol-4-yl}phénol

Le protocole expérimental utilisé est identique à celui décrit pour l'intermédiaire 336.2, la N-méthylpiperazine remplaçant la diméthylamine. On obtient un solide marron clair avec un rendement de 62%. Point de fusion : 234,6-235,2 °C.

### Exemple 349 : chlorhydrate de 2,6-ditert-butyl-4-[2-(pipérazin-1-ylméthyl) 1,3-thiazol-4-yl]phénol

### 349.1) 4-{[4-(3,5-ditert-butyl-4-hydroxyphényl)-1,3-thiazol-2-yl]méthyl}pipérazine-1-carboxylate de tert-butyle

Le protocole expérimental utilisé est identique à celui décrit pour l'intermédiaire 336.2, la N-Boc-pipérazine remplaçant la diméthylamine. On obtient un solide orange pâle avec un rendement de 64%. Point de fusion : 108-109 °C.

### 349.2) chlorhydate de 2,6-ditert-butyl-4-[2-(pipérazin-1-ylméthyl)-1,3-thiazol-4-yl]phénol

Le protocole expérimental utilisé est identique à celui décrit pour l'intermédiaire 323.6, l'intermédiaire 349.1 remplaçant l'intermédiaire 323.5. On obtient un solide blanc avec un rendement de 86%. Point de fusion : 255,4-257,7 °C.

### Etude pharmacologique des produits de l'invention

### Etude des effets sur la liaison d'un ligand spécifique de la MAO-B le [³H]Ro 19-6327

L'activité inhibitrice des produits de l'invention est déterminée par la mesure de leurs effets sur la liaison d'un ligand spécifique de la MAO-B, le [³H]Ro 19-6327.

### a) Préparation mitochondriale de cortex de rats

La préparation mitochondriale de cortex de rats est réalisée selon la méthode décrite dans Cesura A M, Galva M D, Imhof R et Da Prada M, J. Neurochem. 48 (1987), 170-176. Les rats sont décapités et leurs cortex prélevés, homogénéisés dans 9 volumes d'un tampon sucrose 0,32 M tamponné à pH 7,4 avec 5 mM d'HEPES, puis centrifugés à 800 g pendant 20 minutes. Les surnageants sont récupérés et les culots lavés 2 fois avec le tampon sucrose 0,32 M comme précédemment. Les surnageant récoltés sont centrifugés à 10000 g pendant 20 minutes. Les culots obtenus sont mis en suspension dans un tampon Tris (50 mM Tris, 130 mM NaCl, 5 mM KCl, 0,5 mM EGTA, 1 mM MgCl₂, pH 7,4) et centrifugés à 10000 g pendant 20 minutes. Cette étape est répétée 2 fois, et le culot final, correspondant à la fraction mitochondriale, est conservé à -80 °C dans le tampon Tris. Le contenu protéique de la préparation est déterminé par la méthode de Lowry.

### b) Liaison du [³H]Ro 19-6327

Dans un tube Eppendorf, 100 µl de la préparation mitochondriale (2 mg protéine/ml) sont incubés pendant 1 heure à 37 °C en présence de 100 µl de [³H] Ro 19-6327 (33 nM, concentration finale) et 100 µl de tampon Tris contenant ou non les inhibiteurs. La réaction est arrêtée par l'addition de 1 ml de tampon Tris froid dans chaque tube, puis les échantillons sont centrifugés 2 minutes à 12000 g. Les surnageants sont aspirés et les culots lavés avec 1 ml de tampon Tris. Les culots sont ensuite solubilisés dans 200 µl de sodium dodécyl sulfate (20% poids/volume) pendant 2 heures à 70 °C. La radioactivité est déterminée par comptage des échantillons en scintillation liquide.

### c) Résultats

Les composés des exemples 1, 3, 6, 22, 24, 26 à 29, 323 et 332 décrits ci-dessus présentent une CI₅₀ inférieure à 10 µM.

### Etude des effets sur la peroxydation lipidique du cortex cérébral de rat

L'activité inhibitrice des produits de l'invention est déterminée par la mesure de leurs effets sur le degré de peroxydation lipidique, déterminée par la concentration en malondialdéhyde (MDA). Le MDA produit par la peroxydation des acides gras insaturés est un bon indice de la peroxydation lipidique (H Esterbauer and KH Cheeseman, Meth. Enzymol. (1990) 186 : 407-421). Des rats mâles Sprague Dawley de 200 à 250 g (Charles River) ont été sacrifiés par décapitation. Le cortex cérébral est prélevé, puis homogénéisé au potter de Thomas dans du tampon Tris-HCl 20 mM, pH = 7,4. L'homogénat est centrifugé deux fois à 50000 g pendant 10 minutes à 4 °C. Le culot est conservé à -80 °C. Le jour de l'expérience, le culot est remis en suspension à la concentration de 1 g / 15 ml et centrifugé à 515 g pendant 10 minutes à 4°C. Le surnageant est utilisé immédiatement pour la détermination de la peroxidation lipidique. L'homogénat de cortex cérébral de rat (500 µl) est incubé à 37 °C pendant 15 minutes en présence des composés à tester ou du solvant (10 µl). La réaction de peroxydation lipidique est initiée par l'ajout de 50 µl de FeCl₂ à 1 mM, d'EDTA à 1 mM et d'acide ascorbique à 4 mM. Après 30 minutes d'incubation à 37 °C la réaction est arrêtée par l'ajout de 50 µl d'une solution de di tertio butyl toluène hydroxylé (BHT, 0,2 %). Le MDA est quantifié à l'aide d'un test colorimétrique, en faisant réagir un réactif chromogène (R) le N-méthyl-2-phénylindole (650 µl) avec 200 µl de l'homogénat pendant 1 heure à 45 °C. La condensation d'une molécule de MDA avec deux molécules de réactif R produit un chromophore stable dont la longueur d'onde d'absorbance maximale est égale à 586 nm. (Caldwell et coll. European J. Pharmacol. (1995) 285, 203-206). Les composés des exemples 1 à 3, 6 à 17, 20 à 30, 320, 321, 323, 331 et 332 décrits ci-dessus présentent une CI₅₀ inférieure à 10 µM.

### Test de liaison sur les canaux sodiques de cortex cérébraux de rat

Le test consiste à mesurer l'interaction des composés vis-à-vis de la liaison de la batrachotoxine tritiée sur les canaux sodiques dépendants du voltage selon le protocole décrit par Brown (J. Neurosci. (1986), 6, 2064-2070).

### Préparation des homogénats de cortex cérébraux de rat

Les cortex cérébraux de rat Sprague-Dawley de 230-250 g (Charles River, France) sont prélevés, pesés et homogénéisés à l'aide d'un broyeur Potter muni d'un piston en téflon (10 allers/retours) dans 10 volumes de tampon d'isolement dont la composition est la suivante (sucrose 0,32 M ; K₂HPO₄ 5 mM ; pH 7,4). L'homogénat subit une première centrifugation à 1000 g pendant 10 minutes. Le surnageant est prélevé et centrifugé à 20000 g pendant 15 minutes. Le culot est repris dans le tampon d'isolement et centrifugé à 20000 g pendant 15 minutes. Le culot obtenu est remis en suspension dans du tampon d'incubation (HEPES 50 mM ; KCl 5,4 mM ; MgSO₄ 0,8 mM ; glucose 5,5 mM ; chlorure de choline 130 mM pH 7,4) puis aliquoté et conservé à -80 °C jusqu'au jour du dosage. La concentration finale en protéines est comprise entre 4 et 8 mg/ml. Le dosage de protéines se fait par un kit commercialisé par BioRad (France).

### Mesure de la liaison de la batrachotoxine tritiée

La réaction de liaison se fait en incubant pendant 1 h 30 à 25 °C 100 µl d'homogénat de cortex de rat contenant 75 µg de protéines avec 100 µl de [³H] batrachotoxine-A 20-alpha benzoate (37,5 Ci/mmol, NEN) à 5 nM (concentration finale), 200 µl de tétrodotoxine à 1 µM (concentration finale) et de venin de scorpion à 40 µg/ml (concentration finale) et 100 µl de tampon d'incubation seul ou en présence des produits à tester aux différentes concentrations. La liaison non spécifique est déterminée en présence de 300 µM de veratridine et la valeur de cette liaison non spécifique est soustraite à toutes les autres valeurs. Les échantillons sont ensuite filtrés à l'aide d'un Brandel (Gaithersburg, Maryland, USA) en utilisant des plaques Unifilter GF/C préincubées avec 0,1 % de polyéthylène imine (20 µl/puits) et rincées 2 fois avec 2 ml de tampon de filtration (HEPES 5 mM ; CaCl₂ 1,8 mM ; MgSO₄ 0,8 mM ; chlorure de choline 130 mM ; BSA 0,01 % ; pH 7,4). Après avoir ajouté 20 µl de Microscint 0^{®}, la radioactivité est comptée à l'aide d'un compteur à scintillation liquide (Topcount, Packard). La mesure est réalisée en duplicat. Les résultats sont exprimés en % de la liaison spécifique de la batrachotoxine tritiée par rapport au témoin.

### Résultats

Les composés des exemples 1, 6, 7, 11, 13, 15, 17, 20, 24, 31 à 38, 42, 43, 46 à 48, 53, 56, 57, 59 à 61, 64 à 80, 82 à 88, 92 à 95, 97, 105, 106, 108, 110, 113, 117, 118, 121 à 123, 125, 128, 130 à 139, 142 à 145, 149, 151, 152, 154, 162 à 166, 168 à 178, 181, 183 à 186, 188, 190 à 196, 198 à 206, 208 à 210, 212 à 218, 220 à 231, 233 à 250, 252 à 259, 261 à 281, 283 à 288, 293 à 313, 324 et 338 à 340 décrits ci-dessus présentent tous une CI₅₀ inférieure ou égale à 1 µM. En outre, les composés des exemples 3, 9, 10, 26, 28 à 30 et 321 décrits ci-dessus présentent une CI₅₀ inférieure ou égale à 3,5 µM.

## Revendications

1. Composés de formule générale **(I)** : 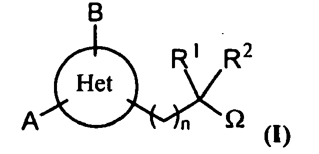 sous forme racémique, d'énantiomère ou toute combinaison de ces formes, dans laquelle Het est un hétérocycle à 5 chaînons comportant 2 hétéroatomes et tel que la formule générale **(I)** corresponde exclusivement à l'une des sous-formules suivantes : 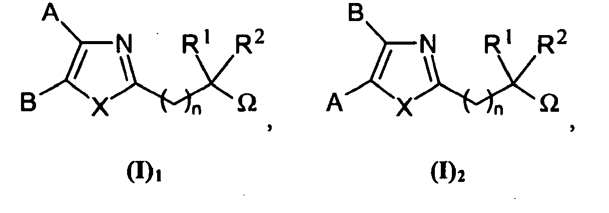 dans lesquelles A représente un radical 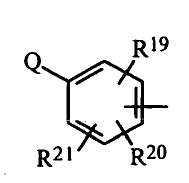 dans lequel Q représente un radical phényle éventuellement substitué par un ou des substituants choisis indépendamment parmi un atome halogène, un radical OH, cyano, nitro, alkyle, alkoxy ou -NR¹⁰R¹¹ et un groupe de deux substituants représentant ensemble un radical méthylène dioxy ou éthylènedioxy,
R¹⁰ et R¹¹ représentant, indépendamment, un atome d'hydrogène ou un radical alkyle, R¹⁹, R²⁰ et R²¹ représentent, indépendamment, un hydrogène, un halogène, le groupe OH ou
SR²⁶, ou un radical alkyle, cycloalkyle, alkényle, alkoxy, cyano, nitro,
R²⁶ représentant un atome d'hydrogène ou un radical alkyle,
X représente S ou NR³⁸,
R³⁸ représentant un atome d'hydrogène,
R¹ représente un atome d'hydrogène, un radical alkyle, aminoalkyle, alkoxyalkyle, cycloalkyle, cycloalkylalkyle, trifluorométhylalkyle, alkényle, allènyle, allènylalkyle, alkynyle, cyanoalkyle, -(CH₂)_{g}-Z¹R³⁹, -(CH₂)g-COR⁴⁰, -(CH₂)_{g}-NHCOR⁷⁰, aryle, aralkyle, arylcarbonyle, hétéroarylalkyle ou aralkylcarbonyle, le groupement aryle des radicaux aryle, aralkyle, arylcarbonyle, hétéroarylalkyle ou aralkylcarbonyle étant lui-même éventuellement substitué par un ou plusieurs substituants choisis parmi le groupe constitué des radicaux alkyle, halogène, alkoxy, nitro, cyano, cyanoalkyle, amino, alkylamino, dialkylamino, - (CH₂)ₖ-Z²R³⁹ ou -(CH₂)ₖ-COR⁴⁰,
Z¹ et Z² représentant une liaison, -O-, -NR⁴¹- ou -S-,
R³⁹ et R⁴¹ représentant, indépendamment à chaque fois qu'ils interviennent, un atome d'hydrogène ou un radical alkyle, alkényle, alkynyle ou cyanoalkyle,
R⁴⁰ représentant, indépendamment à chaque fois qu'il intervient, un atome d'hydrogène ou un radical alkyle, allènyle, allènylalkyle, alkényle, alkynyle, cyanoalkyle, alkoxy ou NR⁴²R⁴³,
R⁴² et R⁴³ représentant, indépendamment à chaque fois qu'ils interviennent, un atome d'hydrogène ou un radical alkyle, allènyle, allènylalkyle, alkényle, alkynyle ou cyanoalkyle,
et R² représente un atome d'hydrogène, un radical alkyle, aminoalkyle, alkoxyalkyle, cycloalkyle, cycloalkylalkyle, trifluorométhylalkyle ou -(CH₂)_{g}-NHCOR⁷¹, ou encore l'un des radicaux aralkyle ou hétéroarylalkyle éventuellement substitués sur le groupe aryle ou hétéroaryle par un ou des groupes choisis indépendamment parmi le groupe composé d'un atome halogène et d'un radical alkyle, alkoxy, hydroxy, cyano, nitro, amino, alkylamino ou dialkylamino,
R⁷⁰ et R⁷¹ représentant indépendamment un radical alkyle ou alkoxy ;
ou bien R¹ et R², pris ensemble avec l'atome de carbone qui les porte, forment un carbocycle de 3 à 7 chaînons ;
B représente un atome d'hydrogène ou un radical alkyle;
Q représente l'un des radicaux NR⁴⁶R⁴⁷ ou OR⁴⁸, dans lesquels :
R⁴⁶ et R⁴⁷ représentent, indépendamment, un atome d'hydrogène ou un radical alkyle, cycloalkyle, cycloalkylalkyle, alkényle, alkynyle, allènyle, allènylalkyle, cyanoalkyle, -(CH₂)_{g}-Z⁴R⁵⁰,-(CH₂)ₖ-COR⁵¹,-(CH₂)ₖ-COOR⁵¹,-(CH₂)ₖ-CONHR⁵¹ ou -SO₂R⁵¹, ou encore un radical choisi parmi les radicaux aryle, aralkyle, aryloxyalkyle, arylcarbonyle, arylimino, aralkylcarbonyle, hétéroaryle et en particulier pyridinyle, pyridinylalkyle ou pyridinylcarbonyle, le groupement aryle ou hétéroaryle desdits radicaux aryle, aralkyle, aryloxyalkyle, arylcarbonyle, arylimino, aralkylcarbonyle, hétéroaryle, pyridinylalkyle ou pyridinylcarbonyle étant éventuellement substitué par un ou des substituants choisis indépendamment parmi halogène, alkyle, alkoxy, hydroxy, nitro, cyano, cyanoalkyle, amino, alkylamino, dialkylamino, -(CH₂)ₖ-Z⁵R⁵⁰, -(CH₂)ₖ-COR⁵¹ et -(CH₂)ₖ-COOR⁵¹,
Z⁴ et Z⁵ représentant une liaison, -O-, -NR⁵²- ou -S-,
R⁵⁰ et R⁵², représentant, indépendamment à chaque fois qu'ils interviennent, un atome d'hydrogène ou un radical alkyle, alkényle, alkynyle, alkoxy, allènyle, allènylalkyle ou cyanoalkyle,
R⁵¹ représentant, indépendamment à chaque fois qu'ils intervient, un atome d'hydrogène, l'un des radicaux cycloalkyle ou cycloalkylalkyle dans lesquels le radical cycloalkyle compte de 3 à 7 atomes de carbone, un radical alkyle linéaire ou ramifié comptant de 1 à 8 atomes de carbone, un radical alkényle, alkynyle, allènyle, allènylalkyle, cyanoalkyle, alkoxyalkyle ou NR⁵⁸R⁵⁹, ou encore un radical aryle ou aralkyle, ledit radical aryle ou aralkyle pouvant être substitué par un ou des substituants choisis indépendamment parmi un atome halogène et un radical alkyle ou alkoxy,
R⁵⁸ et R⁵⁹ représentant, indépendamment, un atome d'hydrogène ou un radical alkyle, alkényle, alkynyle, allènyle, allènylalkyle ou cyanoalkyle,
g et p, chaque fois qu'ils interviennent, étant indépendamment des entiers de 1 à 6, et k et n, chaque fois qu'ils interviennent, étant indépendamment des entiers de 0 à 6 ;
et R⁴⁸ représente un atome d'hydrogène ou un radical alkyle, alkynyle ou cyanoalkyle ;
ou soient des sels de tels composés.

2. Composé selon la revendication 1 ou un de ses sels **caractérisé en qu'**il s'agit d'un composé ou un de ses sels choisi parmi :
2-(4-[1,1-biphényl]-4-yl-1H-imidazol-2-yl)éthylcarbamate de butyle ;
N-[2-(4-[1,1'-biphényl]-4-yl-1H-imidazol-2-yl)éthyl]-1-butanesulfonamide ;
4-[2-(2-{[butylamino)carbonyl]amino}ethyl)-1H-imidazol-4-yl]-1,1'-biphényle ;
*N*-benzyl-*N*-[(4-[1,1'-biphényl]-4-yl-1*H*-imidazol-2-yl)méthyl]-1-hexanamine ;
*N*-benzyl(4-[1,1'-biphényl]-4-yl-1*H*-imidazol-2-yl)-*N*-méthylméthanamine ;
(R,S)-4-(2-{1-[(*tert*-butoxycarbonyl)amino]pentyl}-1*H*-imidazol-4-yl)-1,1'-biphényle;
(R,S)-*N*-benzyl-l-(4-[1,1'-biphényl]-4-yl-1*H*-imidazol-2-yl)-1-pentanamine ;
(4-[1,1'-biphényl]-4-yl-1*H*-imidazol-2-yl)méthylcarbamate de butyle
4-(2-{[(*tert*-butoxycarbonyl)amino]méthyl}-1*H-*imidazol-4-yl)-1,1'-biphényle ;
(4-[1,1'-biphényl]-4-yl-1*H*-imidazol-2-yl)méthanamine ;
*N,N*-dibenzyl(4-[1,1'-biphényl]-4-yl-1*H*-imidazol-2-yl)méthanamine ;
4-(2-{[(*tert*-butoxycarbonyl)(méthyl)amino]méthyl}-1*H*-imidazol-4-yl)-1,1'-biphényle
4-(2- {(1*R*)-1-[(*tert*-butoxycarbonyl)amino]-2-cyclohexyléthyl}-1*H* imidazol-4-yl)-l,l'-biphényle ;
4-(2-{2-[(*tert*-butoxycarbonyl)amino]éthyl}-1*H*-imidazol-4-yl)-1,1'-biphényle ;
(1*R*)-1-(4-[1,1'-biphényl]-4-yl-1*H*-imidazol-2-yl)-2-cyclohexyléthanamine ;
(4-[1,1'-biphényl]-4-yl-1*H*-imidazol-2-yl)-*N-*méthylméthanamine ;
2-(4-[1,1'-biphényl]-4-yl-1*H* imidazol-2-yl)éthanamine ;
*N*-benzyl-2-(4-[1,1'-biphényl]-4-yl-1*H*-imidazol-2-yl)éthanamine ;
4-(2-{[benzyl(*tert*-butoxycarbonyl)amino]méthyl}-1*H-*imidazol-4-yl)-1,1'-biphényle ;
(1*R*)-1-(4-[1,1'-biphényl]-4-yl-1*H*-imidazol-2-yl)-3-phényl-1-propanamine ;
4-(2-{(1*R*)-1-[(*tert*-butoxycarbonyl)amino]-3-phénylpropyl} - 1*H*-imidazol-4-yl)-1,1'-biphényle ;
*N*-benzyl(4-[1,1'-biphényl]-4-yl-1*H*-imidazol-2-yl)méthanamine ;
(1*R*)-*N-*benzyl-1-(4-[1,1'-biphényl]-4-yl-1*H*-imidazol-2-yl)-2-cyclohexyléthanamine ;
(1*R*)-*N*-benzyl-1-(4-[1,1'-biphényl]-4-yl-1*H*-imidazol-2-yl)-3-phényl-1-propanamine ;
4-(2-{3 -[(*tert*-butoxycarbonyl)amino]propyl}-1*H*-imidazol-4-yl)-1,1'-biphényle ;
(R,S)-1-(4-[1,1'-biphényl]-4-yl-1*H*-imidazol-2-yl)-1-pentanamine ;
*N*-[2-(4-[1,1'-biphényl]-4-yl-1*H*-imidazol-2-yl)éthyl]-1-hexanamine ;
4-[2-(2-{[(*tert*-butylamino)carbonyl]amino}éthyl)-1*H*-imidazol-4-yl]-1,1'-biphényle;
*N*-benzyl-3-(4-[1,1'-biphényl]-4-yl-1*H*-imidazol-2-yl)-1-propanamine;
3-(4-[1,1'-biphényl]-4-yl-1*H*-imidazol-2-yl)-1-propanamine ;
(R,S)-1-(4-[1,1'-biphényl]-4-yl-1*H*-imidazol-2-yl)-1-heptanamine ;
(R,S)-4-(2- {1-[(*tert*-butoxycarbonyl)amino]heptyl}-1*H*-imidazol-4-yl)-1,1'-biphényle;
4-(2-{(1*S*)-1-[(*tert*-butoxycarbonyl)amino]propyl}-1*H*-imidazol-4-yl)-1,1'-biphényle;
(R,S)-*N*-benzyl-1-(4-[1,1'-biphényl]-4-yl-1*H*-imidazol-2-yl)-1-heptanamine;
(1*S*)-1-(4-[1,1'-biphényl]-4-yl-1*H*-imidazol-2-yl)-1-propanamine ;
(1*S*)-*N*-benzyl-1-(4-[1,1'-biphényl]-4-yl-1*H*-imidazol-2-yl)-1-propanamine;
4-[2-(2-{[(néopentyloxy)carbonyl]aminol}éthyl)-1*H*-imidazol-4-yl]-1,1'-biphényle;
4-(2-{(1*R*)-1-[(*tert*-butoxycarbonyl)amino]butyl}-1*H*-imidazol-4-yl)-1,1'-biphényle;
(1*R*)-1,-(4-[1,1'-biphényl]-4-yl-1*H*-imidazol-2-yl)-1-butanamine ;
(1*R*)-*N*-benzyl-1-(4-[1,1'-biphényl]-4-yl-1*H*-imidazol-2-yl)-1-butanamine ;
*N*-[(1*S*)-1-(4-[ 1,1'-biphényl]-4-yl-1*H*-imidazol-2-yl)propyl]-1-butanamine ;
4-[2-(2-{[(benzyloxy)carbonyl]amino}éthyl)-1*H*-imidazol-4-yl]-1,1'-biphényle ;
4-(2-{1-[(butoxycarbonyl)amino]-1-méthyléthyl}-1*H*-imidazol-4-yl)-1, l'-biphényle ;
4-(2-{2-[(isobutoxycarbonyl)amino]éthyl}-1*H*-imidazol-4-yl)-1,1'-biphényle ;
4-(2-{(1*S*)-1-[(butoxycarbonyl)amino]éthyl}-1*H*-imidazol-4-yl)-1,1'-biphényle ; 4-(2-{(1*R*)-1-[(butoxycarbonyl)amino]éthyl}-1*H* imidazol-4-yl)-1,1'-biphényle ;
4-(2- {2-[(méthoxycarbonyl)amino] éthyl}-1*H*-imidazol-4-yl)-1,1'-biphényle ;
4-(2-{2-[(propoxycarbonyl)amino]éthyl}-1*H*-imidazol-4-yl)-1,1'-biphényle ;
4-(2-{2-[(éthoxycarbonyl)amino]éthyl}-1*H-*imidazol-4-yl)-1,1'-biphényle ;
4-[2-(1-{[(benzyloxy)carbonyl]amino}-1-méthyléthyl)-1*H-*imidazol-4-yl]-1,1'-biphényle ;
*N*-[2-(4-[1,1'-biphényl]-4-yl-1*H*-imidazol-2-yl)éthyl]-cyclohexanamine ;
(R,S)-*N*-[1-(4-[1,1'-biphényl]-4-yl-1*H*-imidazol-2-yl)heptyl]-cyclohexanamine ;
2-(4-[1,1'-biphényl]-4-yl-1*H*-imidazol-2-yl)éthylcarbamate d'hexyle ;
4-[2-(2-{[(cyclohexyloxy)carbonyl]amino}éthyl)-1*H*-imidazol-4-yl]-1,1'-biphényle ;
4-[2-(2-{[(cyclopentyloxy)carbonyl]amino}éthyl)-1*H*-imidazol-4-yl]-1,1'-biphényle ;
2-(4-[1,1'-biphényl]-4-yl-1*H*-imidazol-2-yl)éthylcarbamate de cyclohexylméthyle ;
4-bromo-4'-(2-{2-[(butoxycarbonyl)amino]éthyl}-1*H*-imidazol-4-yl)-1,1'-biphényle ;
2-(4-[1,1'-biphényl]-4-yl-1*H*-imidazol-2-yl)éthylcarbamate de cyclobutylméthyle ;
4-[2-(2-{[(2-méthoxyéthoxy)carbonyl]amino}éthyl)-1*H*-imidazol-4-yl]-1,1'-biphényle ;
2-[4-(4'-bromo-1,1'-biphényl-4-yl)-1*H*-imidazol-2-yl]éthylcarbamate de cyclohexylméthyle ;
2-[4-(4'-bromo-1,1'-biphényl-4-yl)-1H-imidazol-2-yl]éthylcarbamate de cyclobutylméthyle ;
2-[4-(4'-bromo-1,1'-biphényl-4-yl)-1H-imidazol-2-yl]éthylcarbamate d'isobutyle ;
2-[4-(4'-bromo-1,1'-biphényl-4-yl)-1*H*-imidazol-2-yl]éthylcarbamate de cyclohexyle ;
*N*-[2-(4-[1,1'-biphényl]-4-yl-1*H*-imidazol-2-yl)éthyl]-3,3-diméthyl-butanamide .

3. Composé selon la revendication 1 ou un de ses sels **caractérisé en qu'**il s'agit du composé suivant ou son sel
2-(4-[1,1'-biphényl]-4-yl-1H-imidazol-2-yl)éthylcarbamate de butyle.

4. Utilisation des composés selon les revendications 1 à 3 ou un de leurs sels pharmaceutiquement acceptable, pour préparer un médicament pour le traitement ou la prévention de la douleur, le traitement de l'épilepsie, le traitement de troubles liés à la neurodégénération et en particulier des accidents cérébraux vasculaires, du traumatisme cérébral, ou le traitement des troubles bipolaires ou du syndrome du colon irritable.

5. Utilisation selon la revendication 4 pour préparer un médicament pour le traitement des accidents cérébraux vasculaires ou le traitement du traumatisme cérébral.

6. Un composé selon l'une des revendications 1 à 3 pour son utilisation a titre de médicament

## Claims

1. Compounds of general formula **(I)** 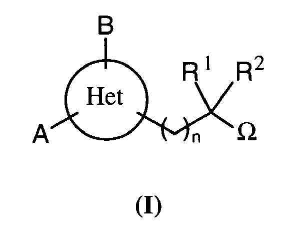 in racemic, enantiomeric form or any combination of these forms, in which Het is a heterocycle with 5 members comprising 2 heteroatoms and such that general formula **(I)** corresponds exclusively to one of the following sub-formulae: 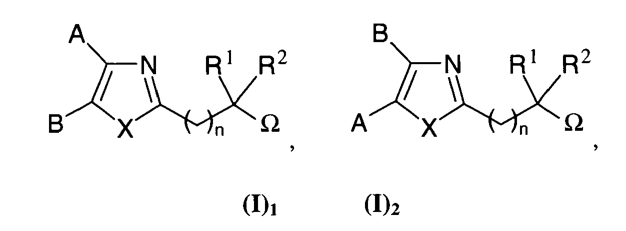 in which
A represents a radical 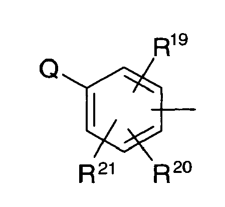 in which Q represents a phenyl radical optionally substituted by one or more substituents chosen independently from a halogen atom, an OH, cyano, nitro, alkyl, alkoxy or -NR¹⁰R¹¹ radical and a group with two substituents representing together a methylenedioxy or ethylenedioxy radical,
R¹⁰ and R¹¹ representing, independently, a hydrogen atom or an alkyl radical, R¹⁹, R²⁰ and R²¹ represent, independently, a hydrogen, a halogen, the OH or SR²⁶ group, or an alkyl, cycloalkyl, alkenyl, alkoxy, cyano, nitro, R²⁶ representing a hydrogen atom or an alkyl radical,
X represents S or NR³⁸,
R³⁸ representing a hydrogen atom,
R¹ represents a hydrogen atom, an alkyl, aminoalkyl, alkoxyalkyl, cycloalkyl, cycloalkylalkyl, trifluoromethylalkyl, alkenyl, allenyl, allenylalkyl, alkynyl, cyanoalkyl, -(CH₂)_{g}-Z¹R³⁹, -(CH₂)_{g}-COR⁴⁰, -(CH₂)_{g}-NHCOR⁷⁰, aryl, aralkyl, arylcarbonyl, heteroarylalkyl or aralkylcarbonyl radical, the aryl group of the aryl, aralkyl, arylcarbonyl, heteroarylalkyl or aralkylcarbonyl radicals itself being optionally substituted by one or more substituents chosen from the group constituted by the alkyl, halogen, alkoxy, nitro, cyano, cyanoalkyl, amino, alkylamino, dialkylamino, -(CH₂)ₖ-Z²R³⁹ or -(CH₂)ₖ-COR⁴⁰ radicals,
Z¹ and Z² representing a bond, -O-, -NR⁴¹- or -S-,
R³⁹ and R⁴¹ representing, independently each time that they occur, a hydrogen atom or an alkyl, alkenyl, alkynyl or cyanoalkyl radical,
R⁴⁰ representing, independently each time that it occurs, a hydrogen atom or an alkyl, allenyl, allenylalkyl, alkenyl, alkynyl, cyanoalkyl, alkoxy or NR⁴²R⁴³ radical,
R⁴² and R⁴³ representing independently, independently each time that they occur, a hydrogen atom or an alkyl, allenyl, allenylalkyl, alkenyl, alkynyl or cyanoalkyl radical, and R² represents a hydrogen atom, an alkyl, aminoalkyl, alkoxyalkyl, cycloalkyl, cycloalkylalkyl, trifluoromethylalkyl or -(CH₂)_{g}-NHCOR⁷¹ radical, or also one of the aralkyl or heteroarylalkyl radicals optionally substituted on the aryl or heteroaryl group by one or more of the groups chosen independently from the group composed of a halogen atom and an alkyl, alkoxy, hydroxy, cyano, nitro, amino, alkylamino or dialkylamino radical,
R⁷⁰ and R⁷¹ representing independently an alkyl or alkoxy radical;
or R¹ and R², taken together with the carbon atom which carries them, form a carbocycle with 3 to 7 members;
B represents a hydrogen atom or an alkyl radical,
Q represents one of the NR⁴⁶R⁴⁷ or OR⁴⁸ radicals, in which:
R⁴⁶ and R⁴⁷ represent, independently, a hydrogen atom or an alkyl, cycloalkyl, cycloalkylalkyl, alkenyl, alkynyl, allenyl, allenylalkyl, cyanoalkyl, -(CH₂)g-Z⁴R⁵⁰, -(CH₂)ₖ-COR⁵¹, -(CH₂)ₖ-COOR⁵¹, -(CH₂)ₖ-CONHR⁵¹ or -SO₂R⁵¹ radical, or also a radical chosen from the aryl, aralkyl, aryloxyalkyl, arylcarbonyl, arylimino, aralkylcarbonyl, heteroaryl and in particular pyridinyl, pyridinylalkyl or pyridinylcarbonyl radicals, the aryl or heteroaryl group of said aryl, aralkyl, aryloxyalkyl, arylcarbonyl, arylimino, aralkylcarbonyl, heteroaryl, pyridinylalkyl or pyridinylcarbonyl radicals being optionally substituted by one or more substituents chosen independently from halogen, alkyl, alkoxy, hydroxy, nitro, cyano, cyanoalkyl, amino, alkylamino, dialkylamino, -(CH₂)ₖ-Z⁵R⁵⁰, -(CH₂)ₖ-COR⁵¹ and -(CH₂)ₖ-COOR⁵¹,
Z⁴ and Z⁵ representing a bond, -O-, -NR⁵²- or -S-,
R⁵⁰ and R⁵², representing, independently each time that they occur, a hydrogen atom or an alkyl, alkenyl, alkynyl, alkoxy, allenyl, allenylalkyl or cyanoalkyl radical,
R⁵¹ representing, independently each time that they occur, a hydrogen atom, one of the cycloalkyl or cycloalkylalkyl radicals in which the cycloalkyl radical has 3 to 7 carbon atoms, a linear or branched alkyl radical containing 1 to 8 carbon atoms, an alkenyl, alkynyl, allenyl, allenylalkyl, cyanoalkyl, alkoxyalkyl or NR⁵⁸R⁵⁹ radical, or also an aryl or aralkyl radical, said aryl or aralkyl radical being able to be substituted by one or more of the substituents chosen independently from a halogen atom and an alkyl or alkoxy radical,
R⁵⁸ and R⁵⁹ representing, independently, a hydrogen atom or an alkyl, alkenyl, alkynyl, allenyl, allenylalkyl or cyanoalkyl radical,
g and p, each time that they occur, being independently integers from 1 to 6, and k and n, each time that they occur, being independently integers from 0 to 6;
and R⁴⁸ represents a hydrogen atom or an alkyl, alkynyl or cyanoalkyl radical ;
or salts thereof.

2. Compound according to claim 1 or a salt thereof, **characterized in that** it is one of the following compounds or a salt thereof :
- butyl 2-(4-[1,1'-biphenyl]-4-yl-1*H*-imidazol-2-yl)ethylcarbamate;
- N-[2-(4-[1,1'-biphenyl]-4-yl-1*H*-imidazol-2-yl)ethyl]-1-butanesulphonamide;
- 4-[2-(2- {[butylamino)carbonyl]amino}ethyl)-1*H*-imidazol-4-yl]-1,1'-biphenyl;
- *N*-benzyl-*N*-[(4-[1,1'-biphenyl]-4-yl-1*H*-imidazol-2-yl)methyl]-1-hexanamine;
- *N*-benzyl(4-[1,1'-biphenyl]-4-yl-1*H*-imidazol-2-yl)-*N*-methylmethanamine;
- (R,S)-4-(2- {1-[(*tert*-butoxycarbonyl)amino]pentyl}-1*H*-imidazol-4-yl)-1,1'-biphenyl;
- (R,S)-*N*-benzyl-1-(4-[1,1'-biphenyl]-4-yl-1*H*-imidazol-2-yl)-1-pentanamine;
- butyl (4-[1,1'-biphenyl]-4-yl-1*H*-imidazol-2-yl)methylcarbamate;
- 4-(2-{ [(*tert*-butoxycarbonyl)amino]methyl}-1*H*-imidazol-4-yl)-1,1'-biphenyl;
- (4-[1,1'-biphenyl]-4-yl-1*H*-imidazol-2-yl)methanamine;
- *N*,*N*-dibenzyl(4-[1,1'-biphenyl]-4-yl-1*H*-imidazol-2-yl)methanamine;
- 4-(2-{[(*tert*-butoxycarbonyl)amino] methyl}-1-methyl-1*H*-imidazol-4-yl)-1,1'-biphenyl;
- 4-(2- {(1*R*)-1-[(*tert*-butoxycarbonyl)amino]-2-cyclohexylethyl }-1*H*-imidazol-4-yl)-1,1'-biphenyl;
- 4-(2-{2-[(*tert*-butoxycarbonyl)amino]ethyl}-1*H*-imidazol-4-yl)-1,1'-biphenyl;
- (1*R*)-1-(4-[1,1'-biphenyl]-4-yl-1*H*-imidazol-2-yl)-2-cyclohexylethanamine;
- (4-[1,1'-biphenyl]-4-yl-1*H*-imidazol-2-yl)-*N*-methylmethanamine;
- 2-(4-[1,1'-biphenyl]-4-yl-1*H*-imidazol-2-yl)ethanamine;
- *N*-benzyl-2-(4-[1,1'-biphenyl]-4-yl-1*H*-imidazol-2-yl)ethanamine;
- 4-(2-{[benzyl(*tert*-butoxycarbonyl)amino]methyl}-1*H*-imidazol-4-yl)-1,1'-biphenyl;
- (1*R*)-1-(4-[1,1'-biphenyl]-4-yl-1*H*-imidazol-2-yl)-3-phenyl-1-propanamine;
- 4-(2-{(1*R*)-1-[(*tert*-butoxycarbonyl)amino]-3-phenylpropyl}-1*H*-imidazol-4-yl)-1,1'-biphenyl;
- *N*-benzyl(4-[1,1'-biphenyl]-4-yl-1*H*-imidazol-2-yl)methanamine;
- (1*R*)-*N*-benzyl-1-(4-[1,1'-biphenyl]-4-yl-1*H*-imidazol-2-yl)-2-cyclohexylethanamine;
- (1*R*)-*N*-benzyl-1-(4-[1,1'-biphenyl]-4-yl-1*H*-imidazol-2-yl)-3-phenyl-1-propanamine;
- 4-(2-{3-[(*tert*-butoxycarbonyl)amino]propyl}-1*H*-imidazol-4-yl)-1,1'-biphenyl;
- (R,S)-1-(4-[1,1'-biphenyl]-4-yl-1*H*-imidazol-2-yl)-1-pentanamine;
- *N*-[2-(4-[1,1'-biphenyl]-4-yl-1*H*-imidazol-2-yl)ethyl]-1-hexanamine;
- 4-[2-(2-{[(*tert*-butylamino)carbonyl]amino}ethyl)-1*H*-imidazol-4-yl]-1,1'-biphenyl;
- *N*-benzyl-3-(4-[1,1'-biphenyl]-4-yl-1*H*-imidazol-2-yl)-1-propanamine;
- 3-(4-[1,1'-biphenyl]-4-yl-1*H*-imidazol-2-yl)-1-propanamine;
- (R,S)-1-(4-[1,1'-biphenyl]-4-yl-1*H*-imidazol-2-yl)-1-heptanamine;
- (R,S)-4-(2-{1-[(*tert*-butoxycarbonyl)amino]heptyl}-1*H*-imidazol-4-yl)-1,1'-biphenyl;
- 4-(2-{(1*S*)-1-[(*tert*-butoxycarbonyl)amino]propyl}-1*H*-imidazol-4-yl)-1,1'-biphenyl;
- (R,S)-*N*-benzyl-1-(4-[1,1'-biphenyl]-4-yl-1*H*-imidazol-2-yl)-1-heptanamine;
- (1*S*)-1-(4-[1,1'-biphenyl]-4-yl-1*H*-imidazol-2-yl)-1-propanamine;
- (1*S*)-*N*-benzyl-1-(4-[1,1'-biphenyl]-4-yl-1*H*-imidazol-2-yl)-1-propanamine;
- 4-[2-(2-{[(neopentyloxy)carbonyl]amino}ethyl)-1*H*-imidazol-4-yl]-1,1'-biphenyl;
- 4-(2- {(1*R*)-1-[(*tert*-butoxycarbonyl)amino]butyl}-1*H*-imidazol-4-yl)-1,1'-biphenyl;
- (1*R*)-1-(4-[1,1'-biphenyl]-4-yl-1*H*-imidazol-2-yl)-1-butanamine;
- (1*R*)-*N*-benzyl-1-(4-[1,1'-biphenyl]-4-yl-1*H*-imidazol-2-yl)-1-butanamine;
- *N*-[(1*S*)-1-(4-[1,1'-biphenyl]-4-yl-1*H*-imidazol-2-yl)propyl]-1-butanamine;
- 4-[2-(2-{[(benzyloxy)carbonyl]amino}ethyl)-1*H*-imidazol-4-yl]-1,1'-biphenyl;
- 4-(2-{1-[(butoxycarbonyl)amino]-1-methylethyl}-1*H*-imidazol-4-yl)-1,1'-biphenyl;
- 4-(2-{2-[(isobutoxycarbonyl)amino]ethyl}-1*H*-imidazol-4-yl)-1,1'-biphenyl;
- 4-(2- {(1*S*)-1-[(butoxycarbonyl)amino]ethyl}-1*H*-imidazol-4-yl)-1,1'-biphenyl;
- 4-(2-{(1R)-1-[(butoxycarbonyl)amino]ethyl}-1*H*-imidazol-4-yl)-1,1'-biphenyl;
- 4-(2-{2-[(methoxycarbonyl)amino]ethyl}-1*H*-imidazol-4-yl)-1,1'-biphenyl;
- 4-(2-{2-[(propoxycarbonyl)amino]ethyl}-1*H*-imidazol-4-yl)-1,1'-biphenyl;
- 4-(2-{2-[(ethoxycarbonyl)amino]ethyl}-1*H*-imidazol-4-yl)-1,1'-biphenyl;
- 4-[2-(1-{[(benzyloxy)carbonyl]amino}-1-methylethyl)-1*H*-imidazol-4-yl]-1,1'-biphenyl;
- *N*-[2-(4-[1,1'-biphenyl]-4-yl-1*H*-imidazol-2-yl)ethyl]-cyclohexanamine;
- (R,S)-*N*-[1-(4-[1,1'-biphenyl]-4-yl-1*H*-imidazol-2-yl)heptyl]-cyclohexanamine;
- hexyl 2-(4-[1,1'-biphenyl]-4-yl-1*H*-imidazol-2-yl)ethylcarbamate;
- 4-[2-(2-{[(cyclohexyloxy)carbonyl]amino}ethyl)-1*H*-imidazol-4-yl]-1,1'-biphenyl;
- 4-[2-(2-{[(cyclopentyloxy)carbonyl]amino}ethyl)-1*H*-imidazol-4-yl]-1,1'-biphenyl;
- cyclohexylmethyl 2-(4-[1,1'-biphenyl]-4-yl-1*H*-imidazol-2-yl)ethylcarbamate;
- 4-bromo-4'-(2-{2-[(butoxycarbonyl)amino]ethyl}-1*H*-imidazol-4-yl)-1,1'-biphenyle;
- cyclobutylmethyl 2-(4-[1,1'-biphenyl]-4-yl-1*H*-imidazol-2-yl)ethylcarbamate;
- 4-[2-(2-{[(2-methoxyethoxy)carbonyl]amino}ethyl)-1*H*-imidazol-4-yl]-1,1'-biphenyle;
- cyclohexylmethyl 2-[4-(4'-bromo-1,1'-biphenyl-4-yl)-1*H*-imidazol-2-yl]ethylcarbamate;
- cyclobutylmethyl 2-[4-(4'-bromo-1,1'-biphenyl-4-yl)-1*H*-imidazol-2-yl]ethylcarbamate;
- isobutyl 2-[4-(4'-bromo-1,1'-biphenyl-4-yl)-1*H*-imidazol-2-yl]ethylcarbamate;
- cyclohexyl 2-[4-(4'-bromo-1,1'-biphenyl-4-yl)-1*H*-imidazol-2-yl]ethylcarbamate;
- *N*-[2-(4-[1,1'-biphenyl]-4-yl-1*H*-imidazol-2-yl)ethyl]-3,3-dimethyl-butanamide;
or a pharmaceutically acceptable salt of one of the latter.

3. Compound according to claim 1 or a salt thereof, **characterized in that** it is the following compound or a salt thereof :
- butyl 2-(4-[1,1'-biphenyl]-4-yl-1*H*-imidazol-2-yl)ethylcarbamate.

4. Use of the compounds according to claims 1 to 3 or a pharmaceutically acceptable salt thereof, for preparing a medicament for the treatment or prevention of pain, the treatment of epilepsy, the treatment of disorders linked to neurodegeneration, and in particular vascular cerebral accidents, cerebral traumatism, or the treatment of bipolar disorders or irritable colon syndrome.

5. Use according to claim 4 for preparing a medicament for the treatment of vascular cerebral accidents or the treatment of cerebral traumatism.

6. Compound according to any one of claims 1 to 3 for its use as a medicament.

## Patentansprüche

1. Verbindungen der allgemeinen Formel (I): 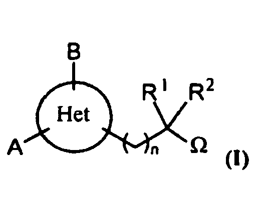 in racemischer Form, in enantiomerer Form oder in allen Kombinationen dieser Formen, wobei Het ein Heterocyclus mit 5 Kettengliedern, die 2 Heteroatome umfassen, ist und so ist, dass die allgemeine Formel (I) ausschließlich einer der folgenden Unterformeln entspricht: 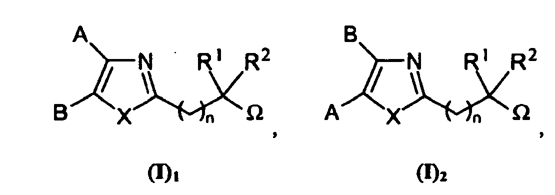 in denen
A einen Rest 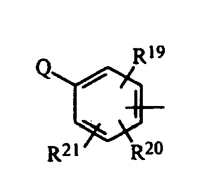 darstellt,
in dem Q einen Phenylrest darstellt, der gegebenenfalls mit einem Substituenten oder Substituenten substituiert ist, unabhängig ausgewählt aus einem Halogenatom, einem OH-, Cyano-, Nitro-, Alkyl-, Alkoxy- oder -NH¹⁰R¹¹-Rest, und wobei eine Gruppe von zwei Substituenten zusammen einen Methylendioxy- oder Ethylendioxy-Rest darstellen,
R¹⁰ und R¹¹ unabhängig ein Wasserstoffatom oder einen Alkylrest darstellen, R¹⁹,R²⁰ und R²¹ unabhängig ein Wasserstoff, ein Halogen, eine OH-Gruppe oder SR²⁶ oder einen Alkyl-, Cycloalkyl-, Alkenyl-, Alkoxy-, Cyano-, Nitro-Rest darstellen,
R²⁶ ein Wasserstoffatom oder einen Alkylrest darstellt,
X S oder NR³⁸ darstellt,
R³⁸ ein Wasserstoffatom darstellt,
R¹ ein Wasserstoffatom, einen Alkyl-, Aminoalkyl, Alkoxyalkyl-, Cycloalkyl-, Cycloalkylalkyl-, Trifluormethylalkyl-, Alkenyl-, Allenyl-, Allenylalkyl-, Alkinyl-, Cyanoalkyl-, -(CH₂)₉-Z¹ R³⁹-, -(CH₂)_{g}-COR⁴⁰-, -(CH₂)_{g}-NHCOR⁷⁰-, Aryl-, Aralkyl-, Arylcarbonyl-, Heteroarylalkyl- oder Aralkylcarbonyl-Rest darstellt, wobei die Arylgruppierung der Aryl-, Aralkyl-, Arylcarbonyl-, Heteroarylalkyl- oder Aralkylcarbonyl-Reste selbst gegebenenfalls mit einem oder mehreren Substituenten, ausgewählt aus der Gruppe, bestehend aus Alkyl-, Halogen-, Alkoxy-, Nitro-, Cyano-, Cyanoalkyl-, Amino-, Alkylamino-, Dialkylamino-, -(CH₂)ₖ-Z²R³⁹- oder -(CH₂)ₖ-COR⁴⁰-Resten, substituiert ist,
Z¹ und Z² eine Bindung, -O-, -NR⁴¹- oder -S- darstellen,
R³⁹ und R⁴¹ unabhängig bei jedem Vorkommen ein Wasserstoffatom oder einen Alkyl-, Alkenyl-, Alkinyl- oder Cyanoalkyl-Rest darstellen,
R⁴⁰ unabhängig bei jedem Vorkommen ein Wasserstoffatom oder einen Alkyl-, Allenyl-, Allenylalkyl-, Alkenyl-, Alkinyl-, Cyanoalkyl-, Alkoxy- oder NR⁴²R⁴³-Rest darstellt,
R⁴² und R⁴³ unabhängig bei jedem Vorkommen ein Wasserstoffatom oder einen Alkyl-, Allenyl-, Allenylalkyl-, Alkenyl-, Alkinyl- oder Cyanoalkyl-Rest darstellen, und
R² ein Wasserstoffatom, einen Alkyl-, Aminoalkyl-, Alkoxyalkyl-, Cycloalkyl-, Cycloalkylalkyl-, Trifluormethylalkyl- oder -(CH₂)_{g}-NHCOR⁷¹-Rest darstellt oder auch einer der Aralkyl- oder Heteroarylalkyl-Reste gegebenenfalls an der Aryl- oder Heteroarylgruppe mit einer Gruppe oder Gruppen, unabhängig ausgewählt aus der Gruppe, bestehend aus einem Halogenatom und einem Alkyl-, Alkoxy-, Hydroxy-, Cyano-, Nitro-, Amino-, Alkylamino- oder Dialkylamino-Rest, substituiert ist,
wobei R⁷⁰ und R⁷¹ unabhängig einen Alkyl- oder Alkoxy-Rest darstellen;
oder R¹ und R² zusammen mit dem Kohlenstoffatom, das sie trägt, einen Carbocyclus mit 3 bis 7 Kettengliedern bilden;
B ein Wasserstoffatom oder einen Alkylrest darstellt;
Ω einen der Reste NR⁴⁶R⁴⁷ oder OR⁴⁸ darstellt, wobei:
R⁴⁶ und R⁴⁷ unabhängig ein Wasserstoffatom oder eine Alkyl-, Cycloalkyl-, Cycloalkylalkyl-, Alkenyl-, Alkinyl-, Allenyl-, Allenylalkyl-, Cyanoalkyl-, -(CH₂)_{g}-Z⁴R⁵⁰-, -(CH₂)ₖ-COR⁵¹-, -(CH₂)ₖ-COOR⁵¹-, -(CH₂)ₖ-CONHR⁵¹- oder-SO₂R⁵¹-Gruppe darstellen, oder auch einen Rest, ausgewählt aus den Aryl-, Aralkyl-, Aryloxyalkyl-, Arylcarbonyl-, Arylimino-, Aralkylcarbonyl-, Heteroaryl- und insbesondere Pyridinyl-, Pyridinylalkyl- oder Pyridinylcarbonyl-Resten, darstellen, wobei die Aryl- oder Heteroaryl-Gruppe der Aryl-, Aralkyl-, Aryloxyalkyl-, Arylcarbonyl-, Arylimino-, Aralkylcarbonyl-, Heteroaryl-, Pyridinylalkyl- oder Pyridinylcarbonyl-Gruppe gegebenenfalls mit einem Substituenten oder Substituenten, unabhängig ausgewählt aus Halogen, Alkyl, Alkoxy, Hydroxy, Nitro, Cyano, Cyanoalkyl, Amino, Alkylamino, Dialkylamino, -(CH₂)ₖ-Z⁵R⁵⁰, -(CH₂)ₖ-COR⁵¹ und -(CH₂)ₖCOOR⁵¹, substituiert ist,
Z⁴ und Z⁵ eine Bindung, -O-, -NR⁵²- oder -S-, darstellen,
R⁵⁰ und R⁵² unabhängig bei jedem Vorkommen ein Wasserstoffatom oder einen Alkyl-, Alkenyl-, Alkinyl-, Alkoxy-, Allenyl-, Allenylalkyl- oder Cyanoalkyl-Rest darstellen,
R⁵¹ unabhängig bei jedem Vorkommen ein Wasserstoffatom, einen der Reste Cycloalkyl oder Cycloalkylalkyl, wobei der Cycloalkyl-Rest 3 bis 7 Kohlenstoffatome umfasst, einen linearen oder verzweigten Alkyl-Rest, der 1 bis 8 Kohlenstoffatome umfasst, einen Alkenyl-, Alkinyl-, Allenyl-, Allenylalkyl-, Cyanoalkyl-, Alkoxyalkyl- oder NR⁵⁸R⁵⁹-Rest darstellt oder einen Aryl- oder Aralkyl-Rest darstellt, wobei der Aryl- oder Aralkyl-Rest durch einen Substituenten oder Substituenten, unabhängig ausgewählt aus einem Halogenatom und einem Alkyl- oder Alkoxy-Rest, substituiert sein kann,
R⁵⁸ und R⁵⁹ unabhängig ein Wasserstoffatom oder einen Alkyl-, Alkenyl-, Alkinyl-, Allenyl-, Allenylalkyl- oder Cyanoalkyl-Rest darstellen,
g und p bei jedem Vorkommen unabhängig ganze Zahlen von 1 bis 6 sind, und k und n bei jedem Vorkommen unabhängig ganze Zahlen von 0 bis 6 sind;
und R⁴⁸ ein Wasserstoffatom oder einen Alkyl-, Alkinyl- oder Cyanoalkyl-Rest darstellt; oder Salze dieser Verbindungen.

2. Verbindung gemäß Anspruch 1 oder eines ihrer Salze, **dadurch gekennzeichnet, dass** es sich um eine Verbindung oder eines ihrer Salze handelt, ausgewählt aus:
Butyl-2-(4-[1,1'-biphenyl]-4-yl-1H-imidazol-2-yl)ethylcarbamat;
N-[2-(4-[1,1'-Biphenyl]-4-yl-1H-imidazol-2-yl)ethyl]-1-butansulfonamid;
4-[2-(2-{[Butylamino)carbonyl]amino}ethyl)-1H-imidazol-4-yl]-1,1'-biphenyl;
*N*-Benzyl-*N-*[(4-[1,1'-biphenyl]-4-yl-1*H*-imidazol-2-yl)-methyl]-1-hexanamin;
*N*-Benzyl(4-[1,1'-biphenyl]-4-yl-1*H*-imidazol-2-yl)-*N*-methylmethanamin;
(R,S)-4-(2-{1-[(tert-Butoxycarbonyl)amino]pentyl}-1*H*-imidazol-4-yl)-1,1'-biphenyl;
(R,S)-N-Benzyl-1-(4-[1,1'-biphenyl]-4-yl-1*H*-imidazol-2-yl)-1-pentanamin;
Butyl-(4-[1,1'-biphenyl]-4-yl-1*H*-imidazol-2-yl)methylcarbamat;
4-(2-{[(tert-Butoxycarbonyl)amino]methyl}-1*H*-imidazol-4-yl)-1,1'-biphenyl;
(4-[1,1'-Biphenyl]-4-yl-1*H*-imidazol-2-yl)methanamin;
*N,N*-Dibenzyl(4-[1,1'-biphenyl]-4-yl-1*H*-imidazol-2-yl)methanamin;
4-(2-{[(tert-Butoxycarbonyl)(methyl)amino]methyl}-1*H*-imidazol-4-yl)-1,1'-biphenyl;
4-(2-{(1*R*)-1-[(tert-Butoxycarbonyl)-amino]-2-cyclohexylethyl}-1*H*-imidazol-4-yl)-1,1'-biphenyl;
4-(2-{2-[(tert-Butoxycarbonyl)amino]ethyl}-1*H*-imidazol-4-yl)-1,1'-biphenyl;
(1*R*)-1-(4-[1,1'-Biphenyl]-4-yl-1*H*-imidazol-2-yl)-2-cyclohexylethanamin;
(4-[1,1'-Biphenyl]-4-yl-1*H*-imidazol-2-yl)-N-methylmethanamin;
2-(4-[1,1'-Biphenyl]-4-yl-1*H*-imidazol-2-yl)ethanamin;
N-Benzyl-2-(4-[1,1'-biphenyl]-4-yl-1*H*-imidazol-2-yl)ethanamin;
4-(2-{[Benzyl(tert-butoxycarbonyl)amino]methyl}-1*H*-imidazol-4-yl)-1,1'-biphenyl;
(1*R*)-1-(4-[1,1'-Biphenyl]-4-yl-1*H*-imidazol-2-yl)-3-pheny)-1-propanamin;
4-(2-{(1*R*)-1-[(tert-Butoxycarbonyl)amino}-3-phenylpropyl}-1*H*-imidazol-4-yl)-1,1'-biphenyl; *N*-Benzyl(4-[1,1'-biphenyl]-4-yl-1*H*-imidazol-2-yl)methanamin;
(1*R*)-N-Benzyl-1-(4-[1,1'-biphenyl]-4-yl-1*H*-imidazol-2-yl)-2-cyclohexylethanamin;
(1*R*)-N-Benzyl-1-(4-[1,1'-biphenyl]-4-yl-1*H*-imidazol-2-yl)-3-phenyl-1-propanamin; 4-(2-{3-[(tert-Butoxycarbonyl)amino]propyl}-1*H*-imidazol-4-yl)-1,1'-biphenyl;
(R,S)-1-(4-[1,1'-Biphenyl]-4-yl-1*H*-imidazol-2-yl)-1-pentanamin; N-{2-(4-[1,1'-Biphenyl]-4-yl-1*H*-imidazol-2-yl)ethyl]-1-hexanamin; 4-[2-(2-{[(tert-Butylamino)carbonyl]amino}ethyl)-1*H*-imidazol-4-yl]-1,1'-biphenyl; N-Benzyl-3-(4-[1,1'-biphenyl]-4-yl-1*H*-imidazol-2-yl)-1-propanamin;
3-(4-[1,1'-Biphenyl]-4-yl-1*H*-imidazol-2-yl)-1-propanamin;
(R,S)-1-(4-[1,1'-Biphenyl]-4-yl-1*H*-imidazol-2-yl)-1-heptanamin; (R,S)-4-(2-{1-[(tert-Butoxycarbonyl)amino]heptyl}-1*H*-imidazol-4-yl)-1,1'-biphenyl;
4-(2-{(1*S*)-1-[(tert-Butoxycarbonyl)amino]propyl}-1*H*-imidazol-4-yl)-1,1'-biphenyl; (R,S)-*N*-Benzyl-1-(4-[1,1'-biphenyl]-4-yl-1*H*-imidazol-2-yl)-1-heptanamin;
(1*S*)-1-(4-[1,1'-Biphenyl]-4-yl-1*H*-imidazol-2-yl)-1-propanamin;
(1*S*)-N-Benzyl-1 -(4-[1,1'-biphenyl]-4-yl-1*H*-imidazol-2-yl)-1-propanamin; 4-[2-(2-{[(Neopentyloxy)carbonyl]amino}ethyl)-1*H*-imidazol-4-yl]-1,1'-biphenyl;
4-(2-{(1R)-1-[(tert-Butoxycarbonyl)amino]butyl}-1*H*-imidazol-4-yl)-1,1'-biphenyl;
(1*R*)-1-(4-[1,1'-Biphenyl]-4-yl-1*H*-imidazol-2-yl)-1-butanamin;
(1*R*)-N-Benzyl-1-(4-[1,1'-biphenyl]-4-yl-1*H*-imidazol-2-yl)-1-butanamin;
*N*-[(1*S*)-1-(4-[1,1'-Biphenyl]-4-yl-1*H*-imidazol-2-yl)propyl]-1-butanamin;
4-[2-(2-{[(Benzyloxy)carbonyl]amino}ethyl)-1*H*-imidazol-4-yl]-1,1'-biphenyl;
4-(2-{1-[(Butoxycarbonyl)amino]-1-methylethyl}-1*H*-imidazol-4-yl)-1,1'-biphenyl;
4-(2-{2-[(Isobutoxycarbonyl)amino]ethyl}-1*H*-imidazol-4-yl)-1,1'-biphenyl;
4-(2-{(1*S*)-1-[(Butoxycarbonyl)amino]ethyl}-1*H*-imidazol-4-yl)-1,1'-biphenyl;
4-(2-{(1*R*)-1-[(Butoxycarbonyl)amino]ethyl}-1*H*-imidazol-4-yl)-1,1'-biphenyl;
4-(2-{2-[(Methoxycarbonyl)amino]ethyl}-1*H*-imidazol-4-yl)-1,1'-biphenyl;
4-(2-{2-[(Propoxycarbonyl)amino]ethyl}-1*H*-imidazol-4-yl)-1,1'-biphenyl;
4-(2-{2-[(Ethoxycarbonyl)amino}ethyl}-1*H*-imidazol-4-yl)-1,1'-biphenyl;
4-[2-(1-{[(Benzyloxy)carbonyl]amino}-1-methylethyl-1*H*-imidazol-4-yl]-1,1'-biphenyl;
*N*-[2-(4-[1,1'-Biphenyl]-4-yl-1*H*-imidazol-2-yl)ethyl]-cyclohexanamin;
(R,S)-*N*-[1-(4-[1,1'-Biphenyl]-4-yl-1*H*-imidazol-2-yl)heptyl]-cyclohexanamin;
Hexyl-2-(4-[1,1'-biphenyl]-4-yl-1*H*-imidazol-2-yl)ethylcarbamat;
4-[2-(2-{[(Cyclohexyloxy)carbonyl]amino}ethyl)-1*H*-imidazol-4-yl]-1,1'-biphenyl;
4-[2-(2-{[(Cyclopentyloxy)carbonyl]amino}ethyl)-1*H*-imidazol-4-yl]-1,1'-biphenyl;
Cyclohexylmethyl-2-(4-[1,1'-biphenyl]-4-yl-1*H*-imidazol-2-yl)ethylcarbamat;
4-Brom-4'-(2-{2-[(butoxycarbonyl)amino]ethyl}-1*H*-imidazol-4-yl)-1,1'-biphenyl;
Cyclobutylmethyl-2-(4-[1,1'-biphenyl]-4-yl-1*H*-imidazol-2-yl)ethylcarbamat;
4-[2-(2-{[(2-Methoxyethoxy)carbonyl]amino}ethyl)-1*H*-imidazol-4-yl]-1,1'-biphenyl;
Cyclohexylmethyl-2-[4-(4'-brom-1,1'-biphenyl-4-yl)-1*H*-imidazol-2-yl]ethylcarbamat;
Cyclobutylmethyl-2-[4-(4'-brom-1,1'-biphenyl-4-yl)-1*H*-imidazol-2-yl]ethylcarbamat;
Isobutyl-2-[4-(4'-brom-1,1'-biphenyl-4-yl)-1*H*-imidazol-2-yl]ethylcarbamat;
Cyclohexyl-2-[4-(4'-brom-1,1'-bipheny)-4-yl)-1*H*-imidazol-2-yl]ethylcarbamat;
*N*-[2-(4-[1,1'-Biphenyl]-4-yl-1*H*-imidazol-2-yl)ethyl]-3,3-dimethylbutanamid.

3. Verbindung gemäß Anspruch 1 oder eines ihrer Salze, **dadurch gekennzeichnet, dass** es sich um die folgende Verbindung oder ihr Salz handelt:
Butyl-2-(4-[1,1'-biphenyl]-4-yl-1*H*-imidazol-2-yl)ethylcarbamat.

4. Verwendung der Verbindungen gemäß den Ansprüchen 1 bis 3 oder eines ihrer pharmazeutisch annehmbaren Salze für die Herstellung eines Medikaments zur Behandlung oder Prävention von Schmerz, Behandlung von Epilepsie, Behandlung von Störungen, die mit Neurodegeneration verbunden sind, und insbesondere von cerebrovaskulären Vorfällen, Hirntrauma, oder Behandlung von bipolaren Störungen oder Reizdarmsyndrom.

5. Verwendung gemäß Anspruch 4 zur Herstellung eines Medikaments zur Behandlung von cerebrovaskulären Vorfällen oder zur Behandlung von Hirntrauma.

6. Verbindung gemäß einem der Ansprüche 1 bis 3 zur Verwendung als Medikament.
